# EUROPEAN PATENT APPLICATION

(11) **EP 4 047 019 A1**
(43) Date of publication of application: **24.08.2022**
(21) Application number: 20898089.6
(22) Date of filing: 08.12.2020
(51) Int. Cl.: C07K 16/24, C12N 15/13, A61K 39/395, A61P 37/02, A61P 29/00, A61P 11/06

(54) **ANTI-TSLP ANTIBODY AND USES THEREOF**

(30) Priority: 13.12.2019 CN 201911278628
(71) Applicant: Sichuan Kelun-Biotech Biopharmaceutical Co., Ltd., Chengdu, Sichuan 611138 (CN); Harbour Biomed (Suzhou) Co., Ltd., Suzhou 215000 (CN)
(72) Inventor: XIAO, Liang, Chengdu Sichuan 611138 (CN); HE, Jinqiu, Suzhou Jiangsu 215000 (CN); XUE, Tongtong, Chengdu Sichuan 611138 (CN); LIU, Hongshui, Suzhou Jiangsu 215000 (CN); WANG, Jingyi, Chengdu Sichuan 611138 (CN); GU, Hongzhuan, Suzhou Jiangsu 215000 (CN); LUO, Shuntao, Chengdu Sichuan 611138 (CN); RONG, Yiping, Suzhou Jiangsu 215000 (CN); LIU, Dengnian, Chengdu Sichuan 611138 (CN); HE, Yun, Suzhou Jiangsu 215000 (CN)
(74) Representative: Turner, Craig Robert
(86) International application number: PCT/CN2020/134438
(87) International publication number: WO 2021/115240

(57) **Abstract**

Related are an anti-TSLP antibody or an antigen-binding fragment thereof, nucleic acid molecules coding the same, and a method for preparing the same. The anti-TSLP antibody or the antigen-binding fragment thereof have high affinity to TSLP, capable of effectively binding with TSLP and blocking the proliferative effect of TSLP with respect to Ba/F3-hTSLPR-hIL7Rα cells, and blocking the capability of TSLP in activating and secreting cytokines with respect to PBMC. At the same time, further related are a medicinal composition comprising the antibody or the antigen-binding fragment thereof, and uses of the composition in preparing a medicament for preventing and/or treating asthma, allergic inflammation, an allergic reaction or autoimmune disease.

SEQUENCE LISTING

<110>  Sichuan Kelun-Biotech Biopharmaceutical Co., Ltd.
       Harbour Biomed (Suzhou) Co., Ltd.

<120>  Anti-TSLP antibody and uses thereof

<130>  P2020TC1327

<150>  CN201911278628.7
<151>  2019-12-13

<160>  75

<170>  PatentIn version 3.5

<210>  1
<211>  121
<212>  PRT
<213>  Artificial Sequence

<220>
<223>  Heavy chain variable region of antibody 25A5C5

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Arg Phe Thr Phe Ser Arg Tyr
            20                  25                  30

Trp Met Asn Trp Val Arg Gln Ala Pro Gly Lys Gly Leu Glu Trp Val
        35                  40                  45

Ala Asn Ile Lys Gln Asp Gly Asn Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Val Val Gly Ala Pro Asp Thr Phe Asp Ile Trp Gly
            100                 105                 110

Gln Gly Thr Met Val Thr Val Ser Ser
        115                 120


<210>  2
<211>  107
<212>  PRT
<213>  Artificial Sequence

<220>
<223>  Light chain variable region of antibody 25A5C5

<400>  2

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asp Tyr
            20                  25                  30

Leu Asn Trp Tyr Gln Gln Lys Pro Gly Thr Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Asp Asn Leu Pro Leu
                85                  90                  95

Thr Phe Gly Gly Gly Thr Lys Val Glu Ile Lys
            100                 105


<210>  3
<211>  8
<212>  PRT
<213>  Artificial Sequence

<220>
<223>  IMGT 25A5C5 CDR-H1

<400>  3

Arg Phe Thr Phe Ser Arg Tyr Trp
1               5


<210>  4
<211>  8
<212>  PRT
<213>  Artificial Sequence

<220>
<223>  IMGT 25A5C5 CDR-H2

<400>  4

Ile Lys Gln Asp Gly Asn Glu Lys
1               5


<210>  5
<211>  14

<212>  PRT
<213>  Artificial Sequence

<220>
<223>  IMGT 25A5C5 CDR-H3

<400>  5

Ala Arg Asp Arg Val Val Gly Ala Pro Asp Thr Phe Asp Ile
1               5                   10


<210>  6
<211>  6
<212>  PRT
<213>  Artificial Sequence

<220>
<223>  IMGT 25A5C5 CDR-L1

<400>  6

Gln Asp Ile Ser Asp Tyr
1               5


<210>  7
<211>  3
<212>  PRT
<213>  Artificial Sequence

<220>
<223>  IMGT 25A5C5 CDR-L2

<400>  7

Asp Ala Ser
1


<210>  8
<211>  9
<212>  PRT
<213>  Artificial Sequence

<220>
<223>  IMGT/ AbM  25A5C5 CDR-L3

<400>  8

Gln Gln Tyr Asp Asn Leu Pro Leu Thr
1               5


<210>  9
<211>  10
<212>  PRT
<213>  Artificial Sequence

<220>
<223>  AbM 25A5C5 CDR-H1

<400>  9

Arg Phe Thr Phe Ser Arg Tyr Trp Met Asn
1               5                   10

<210>  10
<211>  10
<212>  PRT
<213>  Artificial Sequence

<220>
<223>  AbM  25A5C5 CDR-H2

<400>  10

Asn Ile Lys Gln Asp Gly Asn Glu Lys Tyr
1               5                   10

<210>  11
<211>  12
<212>  PRT
<213>  Artificial Sequence

<220>
<223>  AbM 25A5C5 CDR-H3

<400>  11

Asp Arg Val Val Gly Ala Pro Asp Thr Phe Asp Ile
1               5                   10

<210>  12
<211>  11
<212>  PRT
<213>  Artificial Sequence

<220>
<223>  AbM 25A5C5 CDR-L1

<400>  12

Gln Ala Ser Gln Asp Ile Ser Asp Tyr Leu Asn
1               5                   10

<210>  13
<211>  7
<212>  PRT
<213>  Artificial Sequence

<220>
<223>  AbM 25A5C5 CDR-L2

<400>  13

Asp Ala Ser Asn Leu Glu Thr
1               5

<210>  14
<211>  330

<212>  PRT
<213>  Artificial Sequence

<220>
<223>  modified human IgG1 heavy constant region

<400>  14

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Ala Ala Gly Ala Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  15
<211>  330
<212>  PRT
<213>  Artificial Sequence

<220>
<223>  modified human IgG1 heavy constant region

<400>  15

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
        100                 105                 110

Pro Ala Pro Glu Ala Ala Gly Ala Pro Ser Val Phe Leu Phe Pro Pro
    115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
        180                 185                 190

His Gln Asp Trp Leu Asn Gly Lys Glu Tyr Lys Cys Lys Val Ser Asn
    195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
        260                 265                 270

Asn Tyr Lys Thr Thr Pro Pro Val Leu Asp Ser Asp Gly Ser Phe Phe
    275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Ala His Tyr Thr
305                 310                 315                 320

Gln Lys Ser Leu Ser Leu Ser Pro Gly Lys
                325                 330

<210>  16
<211>  107
<212>  PRT

<213>  Artificial Sequence

<220>
<223>  Human ¦Ê light chain constant region

<400>  16

<210>  17
<211>  123
<212>  PRT
<213>  Artificial Sequence

<220>
<223>  Heavy chain variable region of antibody 27C2B6

<400>  17

Gln Val Gln Leu Gln Glu Ser Gly Pro Gly Leu Val Lys Pro Ser Gly
1               5                   10                  15

Thr Leu Ser Leu Thr Cys Ala Val Ser Gly Asp Ser Ile Ser Asn Tyr
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Arg Thr Asn Tyr Asn Pro Ser Leu
    50                  55                  60

Lys Ser Arg Val Thr Leu Ser Val Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Asp Ala Pro Ser Asp Tyr Gly Asp Tyr Gly Phe Phe Asp His
            100                 105                 110

Trp Gly Gln Gly Thr Leu Val Ile Val Ser Ser
        115                 120

<210>  18
<211>  112
<212>  PRT
<213>  Artificial Sequence

<220>
<223>  Light chain variable region of antibody 27C2B6

<400>  18

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Cys Leu Gly Ser Asp Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro Tyr Thr Phe Gly Gln Gly Thr Lys Leu Glu Ile Lys
            100                 105                 110

<210>  19
<211>  9
<212>  PRT
<213>  Artificial Sequence

<220>
<223>  IMGT 27C2B6 CDR-H1

<400>  19

Gly Asp Ser Ile Ser Asn Tyr Asn Trp

1               5


<210>  20
<211>  7
<212>  PRT
<213>  Artificial Sequence

<220>
<223>  IMGT 27C2B6 CDR-H2

<400>  20

Ile Tyr His Ser Gly Arg Thr
1               5


<210>  21
<211>  16
<212>  PRT
<213>  Artificial Sequence

<220>
<223>  IMGT 27C2B6 CDR-H3

<400>  21

Ala Arg Asp Ala Pro Ser Asp Tyr Gly Asp Tyr Gly Phe Phe Asp His
1               5                   10                  15


<210>  22
<211>  11
<212>  PRT
<213>  Artificial Sequence

<220>
<223>  IMGT 27C2B6 CDR-L1

<400>  22

Gln Ser Leu Leu His Ser Asn Gly Tyr Asn Tyr
1               5                   10


<210>  23
<211>  3
<212>  PRT
<213>  Artificial Sequence

<220>
<223>  IMGT  27C2B6 CDR-L2/37C2D10 CDR-L2

<400>  23

Leu Gly Ser
1


<210>  24
<211>  9
<212>  PRT
<213>  Artificial Sequence

<220>
<223>  IMGT/AbM 27C2B6 CDR-L3 /  IMGT/AbM 37C2D10 CDR-L3

<400>  24

Met Gln Ala Leu Gln Thr Pro Tyr Thr
1               5

<210>  25
<211>  11
<212>  PRT
<213>  Artificial Sequence

<220>
<223>  AbM 27C2B6 CDR-H1

<400>  25

Gly Asp Ser Ile Ser Asn Tyr Asn Trp Trp Ser
1               5                   10

<210>  26
<211>  9
<212>  PRT
<213>  Artificial Sequence

<220>
<223>  AbM 27C2B6 CDR-H2

<400>  26

Glu Ile Tyr His Ser Gly Arg Thr Asn
1               5

<210>  27
<211>  14
<212>  PRT
<213>  Artificial Sequence

<220>
<223>  AbM 27C2B6 CDR-H3

<400>  27

Asp Ala Pro Ser Asp Tyr Gly Asp Tyr Gly Phe Phe Asp His
1               5                   10

<210>  28
<211>  16
<212>  PRT
<213>  Artificial Sequence

<220>
<223>  AbM 27C2B6 CDR-L1

<400>  28

Arg Ser Ser Gln Ser Leu Leu His Ser Asn Gly Tyr Asn Tyr Leu Asp

1               5                   10                  15


<210>  29
<211>  7
<212>  PRT
<213>  Artificial Sequence

<220>
<223>  AbM 27C2B6 CDR-L2/37C2D10 CDR-L2

<400>  29

Leu Gly Ser Asp Arg Ala Ser
1               5


<210>  30
<211>  123
<212>  PRT
<213>  Artificial Sequence

<220>
<223>  Heavy chain variable region of antibody 37C2D10

<400>  30

Gln Val Gln Leu Gln Glu Ser Gly Pro Gly Leu Val Lys Pro Ser Gly
1               5                   10                  15


Thr Leu Ser Leu Thr Cys Ala Val Ser Gly Asp Ser Ile Ser Ser Asp
            20                  25                  30


Asn Trp Trp Asn Trp Val Arg Gln Pro Pro Gly Lys Gly Leu Gln Trp
        35                  40                  45


Ile Gly Glu Ile Phe His Ser Gly Arg Thr Asn Tyr Asn Pro Ser Leu
    50                  55                  60


Lys Ser Arg Val Thr Ile Ser Val Asp Met Ser Lys Asn Gln Phe Ser
65                  70                  75                  80


Leu Lys Leu Ser Ser Val Thr Ala Ala Asp Thr Ala Val Tyr Phe Cys
                85                  90                  95


Ala Arg Asp Ala Pro Asn Asp Tyr Gly Asp Tyr Gly Phe Phe Asp Tyr
            100                 105                 110


Trp Gly Gln Gly Ile Leu Val Thr Val Ser Ser
        115                 120


<210>  31
<211>  112
<212>  PRT
<213>  Artificial Sequence

<220>
<223>  Light chain variable region of antibody 37C2D10

<400>  31

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Glu Tyr Asn Tyr Leu Asp Trp Tyr Leu Gln Lys Pro Gly Gln Ser
        35                  40                  45

Pro Gln Leu Leu Ile Tyr Leu Gly Ser Asp Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Ile Tyr Tyr Cys Met Gln Ala
                85                  90                  95

Leu Gln Thr Pro Tyr Thr Phe Gly Gln Gly Thr Lys Leu Glu Ile Lys
            100                 105                 110


<210>  32
<211>  9
<212>  PRT
<213>  Artificial Sequence

<220>
<223>  IMGT 37C2D10 CDR-H1

<400>  32

Gly Asp Ser Ile Ser Ser Asp Asn Trp
1               5


<210>  33
<211>  7
<212>  PRT
<213>  Artificial Sequence

<220>
<223>  IMGT 37C2D10 CDR-H2

<400>  33

Ile Phe His Ser Gly Arg Thr
1               5


<210>  34

<211>  16
<212>  PRT
<213>  Artificial Sequence

<220>
<223>  IMGT 37C2D10 CDR-H3

<400>  34

Ala Arg Asp Ala Pro Asn Asp Tyr Gly Asp Tyr Gly Phe Phe Asp Tyr
1               5                   10                  15


<210>  35
<211>  11
<212>  PRT
<213>  Artificial Sequence

<220>
<223>  IMGT 37C2D10 CDR-L1

<400>  35

Gln Ser Leu Leu His Ser Asn Glu Tyr Asn Tyr
1               5                   10


<210>  36
<211>  11
<212>  PRT
<213>  Artificial Sequence

<220>
<223>  AbM 37C2D10 CDR-H1

<400>  36

Gly Asp Ser Ile Ser Ser Asp Asn Trp Trp Asn
1               5                   10


<210>  37
<211>  9
<212>  PRT
<213>  Artificial Sequence

<220>
<223>  AbM 37C2D10 CDR-H2

<400>  37

Glu Ile Phe His Ser Gly Arg Thr Asn
1               5


<210>  38
<211>  14
<212>  PRT
<213>  Artificial Sequence

<220>
<223>  AbM 37C2D10 CDR-H3

<400>  38

Asp Ala Pro Asn Asp Tyr Gly Asp Tyr Gly Phe Phe Asp Tyr
1               5                   10

<210>  39
<211>  16
<212>  PRT
<213>  Artificial Sequence

<220>
<223>  AbM 37C2D10 CDR-L1

<400>  39

Arg Ser Ser Gln Ser Leu Leu His Ser Asn Glu Tyr Asn Tyr Leu Asp
1               5                   10                  15

<210>  40
<211>  123
<212>  PRT
<213>  Artificial Sequence

<220>
<223>  Heavy chain variable region of antibody 43B1A8/43B1A8-hIgG/43B1-H6L1

<400>  40

Gln Met Gln Leu Val Glu Ser Gly Gly Gly Ala Val Gln Pro Gly Arg
1               5                   10                  15

Ser Leu Arg Leu Ser Cys Ala Ala Ser Gly Phe Thr Phe Arg Ser Tyr
            20                  25                  30

Asp Met His Trp Val Arg Gln Ala Pro Gly Lys Gly Leu Glu Trp Val
        35                  40                  45

Ala Val Ile Trp Tyr Asp Gly Ser Asn Glu Asn Tyr Ala Asp Ser Val
    50                  55                  60

Lys Gly Arg Phe Thr Thr Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Arg Ser Pro Leu Trp Tyr Gly Glu Pro Asp Asp Ala Phe Asp Ile
            100                 105                 110

Trp Gly Gln Gly Thr Leu Val Thr Val Ser Ser
        115                 120

<210>  41

<211>  107
<212>  PRT
<213>  Artificial Sequence

<220>
<223>  Light chain variable region of antibody 43B1A8/43B1A8-hIgG/43B1-H6L1

<400>  41

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Asp Phe Thr Leu Thr Ile Ser Ser Leu Arg Ser
65                  70                  75                  80

Glu Asp Phe Ala Val Tyr Tyr Cys Gln His Tyr Asn Asn Trp Pro Leu
                85                  90                  95

Thr Phe Gly Gly Gly Thr Lys Val Glu Ile Lys
            100                 105


<210>  42
<211>  8
<212>  PRT
<213>  Artificial Sequence

<220>
<223>  IMGT 43B1A8/43B1A8-hIgG/43B1-H6L1/43B1-H2L2 CDR-H1

<400>  42

Gly Phe Thr Phe Arg Ser Tyr Asp
1               5


<210>  43
<211>  8
<212>  PRT
<213>  Artificial Sequence

<220>
<223>  IMGT 43B1A8/43B1A8-hIgG/43B1-H6L1/43B1-H2L2 CDR-H2

<400>  43

Ile Trp Tyr Asp Gly Ser Asn Glu
1               5

<210>  44
<211>  16
<212>  PRT
<213>  Artificial Sequence

<220>
<223>  IMGT 43B1A8/43B1A8-hIgG/43B1-H6L1/43B1-H2L2 CDR-H3

<400>  44

Ala Arg Ser Pro Leu Trp Tyr Gly Glu Pro Asp Asp Ala Phe Asp Ile
1               5                   10                  15


<210>  45
<211>  6
<212>  PRT
<213>  Artificial Sequence

<220>
<223>  IMGT 43B1A8/43B1A8-hIgG/43B1-H6L1/43B1-H2L2 CDR-L1

<400>  45

Gln Ser Val Ser Ser Asn
1               5


<210>  46
<211>  3
<212>  PRT
<213>  Artificial Sequence

<220>
<223>  IMGT 43B1A8/43B1A8-hIgG/43B1-H6L1/43B1-H2L2 CDR-L2

<400>  46

Gly Ala Ser
1


<210>  47
<211>  9
<212>  PRT
<213>  Artificial Sequence

<220>
<223>  IMGT/ AbM 43B1A8/43B1A8-hIgG/43B1-H6L1/43B1-H2L2 CDR-L3

<400>  47

Gln His Tyr Asn Asn Trp Pro Leu Thr
1               5


<210>  48
<211>  10
<212>  PRT
<213>  Artificial Sequence

<220>
<223>  AbM 43B1A8/43B1A8-hIgG/43B1-H6L1/43B1-H2L2 CDR-H1

<400>  48

Gly Phe Thr Phe Arg Ser Tyr Asp Met His
1               5                   10


<210>  49
<211>  10
<212>  PRT
<213>  Artificial Sequence

<220>
<223>  AbM 43B1A8/43B1A8-hIgG/43B1-H6L1/43B1-H2L2 CDR-H2

<400>  49

Val Ile Trp Tyr Asp Gly Ser Asn Glu Asn
1               5                   10


<210>  50
<211>  14
<212>  PRT
<213>  Artificial Sequence

<220>
<223>  AbM 43B1A8/43B1A8-hIgG/43B1-H6L1/43B1-H2L2 CDR-H3

<400>  50

Ser Pro Leu Trp Tyr Gly Glu Pro Asp Asp Ala Phe Asp Ile
1               5                   10


<210>  51
<211>  11
<212>  PRT
<213>  Artificial Sequence

<220>
<223>  AbM 43B1A8/43B1A8-hIgG/43B1-H6L1/43B1-H2L2 CDR-L1

<400>  51

Arg Ala Ser Gln Ser Val Ser Ser Asn Leu Ala
1               5                   10


<210>  52
<211>  7
<212>  PRT
<213>  Artificial Sequence

<220>
<223>  AbM 43B1A8/43B1A8-hIgG/43B1-H6L1/43B1-H2L2 CDR-L2

<400>  52

Gly Ala Ser Thr Arg Ala Thr
1               5

<210>  53
<211>  120
<212>  PRT
<213>  Artificial Sequence

<220>
<223>  Heavy chain variable region of antibody 90H3H11

<400>  53

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Ile Ser Asn His
            20                  25                  30

Asp Ile His Trp Val Arg Gln Ala Pro Gly Lys Gly Leu Glu Trp Val
        35                  40                  45

Thr Leu Ile Trp Tyr Asp Gly Ser Asn Gln Tyr Tyr Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Asp Tyr Ala Asp Tyr Val Tyr Phe His His Trp Gly Gln
            100                 105                 110

Gly Thr Leu Val Thr Val Ser Ser
        115                 120


<210>  54
<211>  107
<212>  PRT
<213>  Artificial Sequence

<220>
<223>  Light chain variable region of antibody 90H3H11

<400>  54

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Ile Asn Ser Tyr
            20                  25                  30

Leu Asn Trp Tyr Arg Gln Lys Pro Gly Lys Ala Pro Glu Leu Leu Ile

35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Tyr
                85                  90                  95

Thr Phe Gly Gln Gly Thr Glu Leu Glu Ile Lys
            100                 105

<210>  55
<211>  8
<212>  PRT
<213>  Artificial Sequence

<220>
<223>  IMGT 90H3H11 CDR-H1

<400>  55

Gly Phe Thr Ile Ser Asn His Asp
1               5

<210>  56
<211>  8
<212>  PRT
<213>  Artificial Sequence

<220>
<223>  IMGT 90H3H11 CDR-H2

<400>  56

Ile Trp Tyr Asp Gly Ser Asn Gln
1               5

<210>  57
<211>  13
<212>  PRT
<213>  Artificial Sequence

<220>
<223>  IMGT 90H3H11 CDR-H3

<400>  57

Ala Arg Asp Asp Tyr Ala Asp Tyr Val Tyr Phe His His
1               5                   10

<210>  58
<211>  6

<212>  PRT
<213>  Artificial Sequence

<220>
<223>  IMGT 90H3H11 CDR-L1

<400>  58

Gln Asn Ile Asn Ser Tyr
1               5


<210>  59
<211>  3
<212>  PRT
<213>  Artificial Sequence

<220>
<223>  IMGT 90H3H11 CDR-L2

<400>  59

Ala Ala Ser
1


<210>  60
<211>  9
<212>  PRT
<213>  Artificial Sequence

<220>
<223>  IMGT/ AbM 90H3H11 CDR-L3

<400>  60

Gln Gln Ser Tyr Ser Thr Pro Tyr Thr
1               5


<210>  61
<211>  10
<212>  PRT
<213>  Artificial Sequence

<220>
<223>  AbM 90H3H11 CDR-H1

<400>  61

Gly Phe Thr Ile Ser Asn His Asp Ile His
1               5                   10


<210>  62
<211>  10
<212>  PRT
<213>  Artificial Sequence

<220>
<223>  AbM 90H3H11 CDR-H2

<400>  62

Leu Ile Trp Tyr Asp Gly Ser Asn Gln Tyr
1               5                   10

<210>  63
<211>  11
<212>  PRT
<213>  Artificial Sequence

<220>
<223>  AbM 90H3H11 CDR-H3

<400>  63

Asp Asp Tyr Ala Asp Tyr Val Tyr Phe His His
1               5                   10

<210>  64
<211>  11
<212>  PRT
<213>  Artificial Sequence

<220>
<223>  AbM 90H3H11 CDR-L1

<400>  64

Arg Ala Ser Gln Asn Ile Asn Ser Tyr Leu Asn
1               5                   10

<210>  65
<211>  7
<212>  PRT
<213>  Artificial Sequence

<220>
<223>  AbM 90H3H11 CDR-L2

<400>  65

Ala Ala Ser Ser Leu Gln Ser
1               5

<210>  66
<211>  453
<212>  PRT
<213>  Artificial Sequence

<220>
<223>  heavy chain of 43B1A8-hIgG

<400>  66

Gln Met Gln Leu Val Glu Ser Gly Gly Gly Ala Val Gln Pro Gly Arg
1               5                   10                  15

Ser Leu Arg Leu Ser Cys Ala Ala Ser Gly Phe Thr Phe Arg Ser Tyr
            20                  25                  30

Asp Met His Trp Val Arg Gln Ala Pro Gly Lys Gly Leu Glu Trp Val
        35                  40                  45

Ala Val Ile Trp Tyr Asp Gly Ser Asn Glu Asn Tyr Ala Asp Ser Val
    50                  55                  60

Lys Gly Arg Phe Thr Thr Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Arg Ser Pro Leu Trp Tyr Gly Glu Pro Asp Asp Ala Phe Asp Ile
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Ala
225                 230                 235                 240

Ala Gly Ala Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val

275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  67
<211>  214
<212>  PRT
<213>  Artificial Sequence

<220>
<223>  light chain of 43B1A8-hIgG/43B1-H6L1

<400>  67

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Asp Phe Thr Leu Thr Ile Ser Ser Leu Arg Ser
65                  70                  75                  80

Glu Asp Phe Ala Val Tyr Tyr Cys Gln His Tyr Asn Asn Trp Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 68
<211> 123
<212> PRT
<213> Artificial Sequence

<220>
<223> VH of 43B1-H2L2

<400> 68

Gln Met Gln Leu Val Glu Ser Gly Gly Gly Ala Val Gln Pro Gly Gly
1               5                   10                  15

Ser Leu Arg Leu Ser Cys Ala Ala Ser Gly Phe Thr Phe Arg Ser Tyr
            20                  25                  30

Asp Met His Trp Val Arg Gln Ala Pro Gly Lys Gly Leu Glu Trp Val
        35                  40                  45

Ala Val Ile Trp Tyr Asp Gly Ser Asn Glu Asn Tyr Ala Asp Ser Val
    50                  55                  60

Lys Gly Arg Phe Thr Thr Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Arg Ser Pro Leu Trp Tyr Gly Glu Pro Asp Asp Ala Phe Asp Ile
            100                 105                 110

Trp Gly Gln Gly Thr Leu Val Thr Val Ser Ser
        115                 120

<210>  69
<211>  107
<212>  PRT
<213>  Artificial Sequence

<220>
<223>  VL of 43B1-H2L2

<400>  69

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Asp Phe Thr Leu Thr Ile Ser Ser Leu Gln Ser
65                  70                  75                  80

Glu Asp Phe Ala Val Tyr Tyr Cys Gln His Tyr Asn Asn Trp Pro Leu
                85                  90                  95

Thr Phe Gly Gly Gly Thr Lys Val Glu Ile Lys
            100                 105

<210>  70
<211>  327
<212>  PRT
<213>  Artificial Sequence

<220>
<223>  Modified human IgG4 heavy chain constant region

<400>  70

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu

195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Ala His Tyr Thr Gln Lys Ser
305                 310                 315                 320

Leu Ser Leu Ser Leu Gly Lys
                325

<210>  71
<211>  453
<212>  PRT
<213>  Artificial Sequence

<220>
<223>  heavy chain of 43B1-H2L2

<400>  71

Gln Met Gln Leu Val Glu Ser Gly Gly Gly Ala Val Gln Pro Gly Gly
1               5                   10                  15

Ser Leu Arg Leu Ser Cys Ala Ala Ser Gly Phe Thr Phe Arg Ser Tyr
            20                  25                  30

Asp Met His Trp Val Arg Gln Ala Pro Gly Lys Gly Leu Glu Trp Val
        35                  40                  45

Ala Val Ile Trp Tyr Asp Gly Ser Asn Glu Asn Tyr Ala Asp Ser Val
    50                  55                  60

Lys Gly Arg Phe Thr Thr Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Arg Ser Pro Leu Trp Tyr Gly Glu Pro Asp Asp Ala Phe Asp Ile
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Ala
225                 230                 235                 240

Ala Gly Ala Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala

325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Ala His Tyr Thr Gln Lys Ser Leu Ser
        435                 440                 445

Leu Ser Pro Gly Lys
    450

<210>  72
<211>  214
<212>  PRT
<213>  Artificial Sequence

<220>
<223>  light chain of 43B1-H2L2

<400>  72

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Asp Phe Thr Leu Thr Ile Ser Ser Leu Gln Ser
65                  70                  75                  80

Glu Asp Phe Ala Val Tyr Tyr Cys Gln His Tyr Asn Asn Trp Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  73
<211>  450
<212>  PRT
<213>  Artificial Sequence

<220>
<223>  heavy chain of 43B1-H6L1

<400>  73

Gln Met Gln Leu Val Glu Ser Gly Gly Gly Ala Val Gln Pro Gly Arg
1               5                   10                  15

Ser Leu Arg Leu Ser Cys Ala Ala Ser Gly Phe Thr Phe Arg Ser Tyr
            20                  25                  30

Asp Met His Trp Val Arg Gln Ala Pro Gly Lys Gly Leu Glu Trp Val
        35                  40                  45

Ala Val Ile Trp Tyr Asp Gly Ser Asn Glu Asn Tyr Ala Asp Ser Val
    50                  55                  60

Lys Gly Arg Phe Thr Thr Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Arg Ser Pro Leu Trp Tyr Gly Glu Pro Asp Asp Ala Phe Asp Ile
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Lys Thr Tyr Thr Cys Asn Val
        195                 200                 205

Asp His Lys Pro Ser Asn Thr Lys Val Asp Lys Arg Val Glu Ser Lys
    210                 215                 220

Tyr Gly Pro Pro Cys Pro Pro Cys Pro Ala Pro Glu Phe Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser Gln Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Phe Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gly Leu Pro Ser Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Gln Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Arg Leu Thr Val Asp
                405                 410                 415

Lys Ser Arg Trp Gln Glu Gly Asn Val Phe Ser Cys Ser Val Met His
            420                 425                 430

Glu Ala Leu His Ala His Tyr Thr Gln Lys Ser Leu Ser Leu Ser Leu
        435                 440                 445

Gly Lys
    450

<210>  74
<211>  330
<212>  PRT
<213>  Artificial Sequence

<220>
<223>  Human IgG1 constant region

<400>  74

<210>  75
<211>  327
<212>  PRT
<213>  Artificial Sequence

<220>
<223>  Human IgG4 heavy chain constant region

<400>  75

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

## Description

### Technical Field

The invention belongs to the field of therapeutic monoclonal antibodies, in particular to an antibody against the TSLP, and to the use of the antibody in treating diseases.

### Background Art

Thymic stromal lymphopoietin (TSLP) is an IL7-like inflammatory cytokine, which is mainly secreted by epithelial cells, such as skin, lung, thymus and gastrointestinal tract in response to microorganisms, physical damages or inflammatory cytokines (e.g., IL-1β and TNF), and can also be secreted by stromal cells, keratinocytes, dendritic cells (DC) and mast cells, etc. under pathological conditions such as inflammation. TSLP plays an important role in initiating allergic and adaptive airway inflammation. TSLP is highly expressed in the airway of a patient with asthma as compared with the healthy control group, and the level thereof correlates with the expression of TH2 cytokines and chemokines as well as the severity of diseases. TSLP can induce the maturation of dendritic cells (DC), upregulate OX40L expression, the interaction of OX40-OX40L is involved in T cell-induced initial polarization of TH2 cells, which release cytokines such as IL-4, IL-5, IL-13 after differentiation, contributing to mast cell and eosinophil infiltration and a series of allergic inflammatory responses, and pathological changes in the airway, thereby causing asthma attack. TSLP is effective in activating mast cells and natural killer T (NKT) cells, producing TH2 cytokines such as IL-13, thereby exacerbating the development and progression of airway inflammation.

The receptor for TSLP is a heterodimeric receptor complex consisting of IL-7Rα and the unique TSLPR chain (CRFL2). The binding of TSLP heterodimeric receptor causes the activation of STAT5 and cell proliferation. TSLPR and IL-7Rα are highly expressed in DC cells.

Therefore, it is urgent and necessary to develop anti-TSLP antibodies with high specificity and affinity, low toxic and side effects, as well as excellent clinical efficacy, thereby providing more medication choices for patients with asthma.

### Summary of the Invention

In the present disclosure, the inventors firstly developed chimeric antibodies with excellent properties that are capable of binding to human TSLP. Based on these, the inventors developed fully human antibodies of the chimeric antibodies by studying and modifying the chimeric antibodies. The fully human antibodies of the invention have substantially the same (or even better) biological functions as the chimeric antibodies. The fully human antibodies not only have high affinity for TSLP, but also can effectively block TSLP-induced proliferation of Ba/F3 cells and block the ability of TSLP on activation and cytokine secretion of PBMC. Thus, the invention further relates to a pharmaceutical composition comprising the antibody or antigen-binding fragment thereof, and the use thereof in the preparation of a medicament for the prevention and/or treatment of asthma, allergic inflammation, allergic reactions, or autoimmune diseases.

The antibody of the invention has an extremely high degree of humanization, even being a fully human antibody, so that it can be safely administered to a human subject without triggering an immunogenic response. Therefore, the antibody of the invention is of great clinical value.

### The antibody of the invention

In one aspect, the invention provides an antibody or antigen-binding fragment thereof binding to TSLP, wherein the antibody or antigen-binding fragment thereof comprises complementary determining regions (CDRs) as follows:
(a) a CDR-H1 or a sequence variant thereof, a CDR-H2 or a sequence variant thereof, and a CDR-H3 or a sequence variant thereof contained in the heavy chain variable region (VH) as set forth in SEQ ID NO: 1, 17, 30, 40, 53 or 68; and/or a CDR-L1 or a sequence variant thereof, a CDR-L2 or a sequence variant thereof, and a CDR-L3 or a sequence variant thereof contained in the light chain variable region (VL) as set forth in SEQ ID NO: 2, 18, 31, 41, 54 or 69; or
(b) a CDR-H1 or a sequence variant thereof, a CDR-H2 or a sequence variant thereof, and a CDR-H3 or a sequence variant thereof contained in the VH as set forth in SEQ ID NO: 1; and/or a CDR-L1 or a sequence variant thereof, a CDR-L2 or a sequence variant thereof, and a CDR-L3 or a sequence variant thereof contained in the VL as set forth in SEQ ID NO: 2.

In certain embodiments, the antibody or antigen-binding fragment thereof comprises a CDR-H1 or a sequence variant thereof, a CDR-H2 or a sequence variant thereof, and a CDR-H3 or a sequence variant thereof contained in the VH as set forth in SEQ ID NO: 17; and/or a CDR-L1 or a sequence variant thereof, a CDR-L2 or a sequence variant thereof, and a CDR-L3 or a sequence variant thereof contained in the VL as set forth in SEQ ID NO: 18.

In certain embodiments, the antibody or antigen-binding fragment thereof comprises a CDR-H1 or a sequence variant thereof, a CDR-H2 or a sequence variant thereof, and a CDR-H3 or a sequence variant thereof contained in the VH as set forth in SEQ ID NO: 30; and/or a CDR-L1 or a sequence variant thereof, a CDR-L2 or a sequence variant thereof, and a CDR-L3 or a sequence variant thereof contained in the VL as set forth in SEQ ID NO: 31.

In certain embodiments, the antibody or antigen-binding fragment thereof comprises a CDR-H1 or a sequence variant thereof, a CDR-H2 or a sequence variant thereof, and a CDR-H3 or a sequence variant thereof contained in the VH as set forth in SEQ ID NO: 40; and/or a CDR-L1 or a sequence variant thereof, a CDR-L2 or a sequence variant thereof, and a CDR-L3 or a sequence variant thereof contained in the VL as set forth in SEQ ID NO: 41.

In certain embodiments, the antibody or antigen-binding fragment thereof comprises a CDR-H1 or a sequence variant thereof, a CDR-H2 or a sequence variant thereof, and a CDR-H3 or a sequence variant thereof contained in the VH as set forth in SEQ ID NO: 53; and/or a CDR-L1 or a sequence variant thereof, a CDR-L2 or a sequence variant thereof, and a CDR-L3 or a sequence variant thereof contained in the VL as set forth in SEQ ID NO: 54.

In certain embodiments, the antibody or antigen-binding fragment thereof comprises a CDR-H1 or a sequence variant thereof, a CDR-H2 or a sequence variant thereof, and a CDR-H3 or a sequence variant thereof contained in the VH as set forth in SEQ ID NO: 68; and/or a CDR-L1 or a sequence variant thereof, a CDR-L2 or a sequence variant thereof, and a CDR-L3 or a sequence variant thereof contained in the VL as set forth in SEQ ID NO: 69.

In certain preferred embodiments, the sequence variant is a CDR having a substitution, deletion or addition of one or several amino acids (for example, a substitution, deletion or addition of 1, 2 or 3 amino acids) as compared to the CDR from which it is derived.

In certain preferred embodiments, the substitution is a conservative substitution. Preferably, the CDRs are defined by the AbM, Chothia, Kabat or IMGT numbering system.

In certain embodiments, the VH and/or VL of the antibody or antigen-binding fragment thereof comprises framework regions (FRs) of an immunoglobulin derived from a human.

In certain embodiments, the antibody or antigen-binding fragment thereof binds to human TSLP and/or monkey TSLP.

In one aspect, the invention provides an antibody or antigen-binding fragment thereof capable of binding to TSLP, comprising: a heavy chain variable region (VH) and/or a light chain variable region (VL).

In certain embodiments, the antibody or antigen-binding fragment thereof according to the invention comprises a heavy chain variable region (VH) and/or a light chain variable region (VL) as follows, wherein CDRs are defined by the IMGT numbering system:
(a) a heavy chain variable region (VH) comprising 3 CDRs as follows: a CDR-H1 with a sequence as set forth in SEQ ID NO: 3 or a sequence of a substitution, deletion or addition of one or several amino acids (for example, a substitution, deletion or addition of 1, 2 or 3 amino acids) as compared to SEQ ID NO: 3, a CDR-H2 with a sequence as set forth in SEQ ID NO: 4 or a sequence of a substitution, deletion or addition of one or several amino acids (for example, a substitution, deletion or addition of 1, 2 or 3 amino acids) as compared to SEQ ID NO: 4, a CDR-H3 with a sequence as set forth in SEQ ID NO: 5 or a sequence of a substitution, deletion or addition of one or several amino acids (for example, a substitution, deletion or addition of 1, 2 or 3 amino acids) as compared to SEQ ID NO: 5; and/or,
   a light chain variable region (VL) comprising 3 CDRs as follows: a CDR-L1 with a sequence as set forth in SEQ ID NO: 6 or a sequence of a substitution, deletion or addition of one or several amino acids (for example, a substitution, deletion or addition of 1, 2 or 3 amino acids) as compared to SEQ ID NO: 6, a CDR-L2 with a sequence as set forth in SEQ ID NO: 7 or a sequence of a substitution, deletion or addition of one or several amino acids (for example, a substitution, deletion or addition of 1, 2 or 3 amino acids) as compared to SEQ ID NO: 7, a CDR-L3 with a sequence as set forth in SEQ ID NO: 8 or a sequence of a substitution, deletion or addition of one or several amino acids (for example, a substitution, deletion or addition of 1, 2 or 3 amino acids) as compared to SEQ ID NO: 8;
(b) a heavy chain variable region (VH) comprising 3 CDRs as follows: a CDR-H1 with a sequence as set forth in SEQ ID NO: 19 or a sequence of a substitution, deletion or addition of one or several amino acids (for example, a substitution, deletion or addition of 1, 2 or 3 amino acids) as compared to SEQ ID NO: 19, a CDR-H2 with a sequence as set forth in SEQ ID NO: 20 or a sequence of a substitution, deletion or addition of one or several amino acids (for example, a substitution, deletion or addition of 1, 2 or 3 amino acids) as compared to SEQ ID NO: 20, a CDR-H3 with a sequence as set forth in SEQ ID NO: 21 or a sequence of a substitution, deletion or addition of one or several amino acids (for example, a substitution, deletion or addition of 1, 2 or 3 amino acids) as compared to SEQ ID NO: 21; and/or,
   a light chain variable region (VL) comprising 3 CDRs as follows: a CDR-L1 with a sequence as set forth in SEQ ID NO: 22 or a sequence of a substitution, deletion or addition of one or several amino acids (for example, a substitution, deletion or addition of 1, 2 or 3 amino acids) as compared to SEQ ID NO: 22, a CDR-L2 with a sequence as set forth in SEQ ID NO: 23 or a sequence of a substitution, deletion or addition of one or several amino acids (for example, a substitution, deletion or addition of 1, 2 or 3 amino acids) as compared to SEQ ID NO: 23, a CDR-L3 with a sequence as set forth in SEQ ID NO: 24 or a sequence of a substitution, deletion or addition of one or several amino acids (for example, a substitution, deletion or addition of 1, 2 or 3 amino acids) as compared to SEQ ID NO: 24;
(c) a heavy chain variable region (VH) comprising 3 CDRs as follows: a CDR-H1 with a sequence as set forth in SEQ ID NO: 32 or a sequence of a substitution, deletion or addition of one or several amino acids (for example, a substitution, deletion or addition of 1, 2 or 3 amino acids) as compared to SEQ ID NO: 32, a CDR-H2 with a sequence as set forth in SEQ ID NO: 33 or a sequence of a substitution, deletion or addition of one or several amino acids (for example, a substitution, deletion or addition of 1, 2 or 3 amino acids) as compared to SEQ ID NO: 33, a CDR-H3 with a sequence as set forth in SEQ ID NO: 34 or a sequence of a substitution, deletion or addition of one or several amino acids (for example, a substitution, deletion or addition of 1, 2 or 3 amino acids) as compared to SEQ ID NO: 34; and/or,
   a light chain variable region (VL) comprising 3 CDRs as follows: a CDR-L1 with a sequence as set forth in SEQ ID NO: 35 or a sequence of a substitution, deletion or addition of one or several amino acids (for example, a substitution, deletion or addition of 1, 2 or 3 amino acids) as compared to SEQ ID NO: 35, a CDR-L2 with a sequence as set forth in SEQ ID NO: 23 or a sequence of a substitution, deletion or addition of one or several amino acids (for example, a substitution, deletion or addition of 1, 2 or 3 amino acids) as compared to SEQ ID NO: 23, a CDR-L3 with a sequence as set forth in SEQ ID NO: 24 or a sequence of a substitution, deletion or addition of one or several amino acids (for example, a substitution, deletion or addition of 1, 2 or 3 amino acids) as compared to SEQ ID NO: 24;
(d) a heavy chain variable region (VH) comprising 3 CDRs as follows: a CDR-H1 with a sequence as set forth in SEQ ID NO: 42 or a sequence of a substitution, deletion or addition of one or several amino acids (for example, a substitution, deletion or addition of 1, 2 or 3 amino acids) as compared to SEQ ID NO: 42, a CDR-H2 with a sequence as set forth in SEQ ID NO: 43 or a sequence of a substitution, deletion or addition of one or several amino acids (for example, a substitution, deletion or addition of 1, 2 or 3 amino acids) as compared to SEQ ID NO: 43, a CDR-H3 with a sequence as set forth in SEQ ID NO: 44 or a sequence of a substitution, deletion or addition of one or several amino acids (for example, a substitution, deletion or addition of 1, 2 or 3 amino acids) as compared to SEQ ID NO: 44; and/or,
   a light chain variable region (VL) comprising 3 CDRs as follows: a CDR-L1 with a sequence as set forth in SEQ ID NO: 45 or a sequence of a substitution, deletion or addition of one or several amino acids (for example, a substitution, deletion or addition of 1, 2 or 3 amino acids) as compared to SEQ ID NO: 45, a CDR-L2 with a sequence as set forth in SEQ ID NO: 46 or a sequence of a substitution, deletion or addition of one or several amino acids (for example, a substitution, deletion or addition of 1, 2 or 3 amino acids) as compared to SEQ ID NO: 46, a CDR-L3 with a sequence as set forth in SEQ ID NO: 47 or a sequence of a substitution, deletion or addition of one or several amino acids (for example, a substitution, deletion or addition of 1, 2 or 3 amino acids) as compared to SEQ ID NO: 47;
   or
(e) a heavy chain variable region (VH) comprising 3 CDRs as follows: a CDR-H1 with a sequence as set forth in SEQ ID NO: 55 or a sequence of a substitution, deletion or addition of one or several amino acids (for example, a substitution, deletion or addition of 1, 2 or 3 amino acids) as compared to SEQ ID NO: 55, a CDR-H2 with a sequence as set forth in SEQ ID NO: 56 or a sequence of a substitution, deletion or addition of one or several amino acids (for example, a substitution, deletion or addition of 1, 2 or 3 amino acids) as compared to SEQ ID NO: 56, a CDR-H3 with a sequence as set forth in SEQ ID NO: 57 or a sequence of a substitution, deletion or addition of one or several amino acids (for example, a substitution, deletion or addition of 1, 2 or 3 amino acids) as compared to SEQ ID NO: 57; and/or,
   a light chain variable region (VL) comprising 3 CDRs as follows: a CDR-L1 with a sequence as set forth in SEQ ID NO: 58 or a sequence of a substitution, deletion or addition of one or several amino acids (for example, a substitution, deletion or addition of 1, 2 or 3 amino acids) as compared to SEQ ID NO: 58, a CDR-L2 with a sequence as set forth in SEQ ID NO: 59 or a sequence of a substitution, deletion or addition of one or several amino acids (for example, a substitution, deletion or addition of 1, 2 or 3 amino acids) as compared to SEQ ID NO: 59, a CDR-L3 with a sequence as set forth in SEQ ID NO: 60 or a sequence of a substitution, deletion or addition of one or several amino acids (for example, a substitution, deletion or addition of 1, 2 or 3 amino acids) as compared to SEQ ID NO: 60.

In certain embodiments, the antibody or antigen-binding fragment thereof according to the invention comprises a heavy chain variable region (VH) and/or a light chain variable region (VL) as follows, wherein CDRs are defined by the IMGT numbering system:
(a) a heavy chain variable region (VH) comprising 3 CDRs as follows: a CDR-H1 with a sequence as set forth in SEQ ID NO: 3, a CDR-H2 with a sequence as set forth in SEQ ID NO: 4, a CDR-H3 with a sequence as set forth in SEQ ID NO: 5; and/or,
   a light chain variable region (VL) comprising 3 CDRs as follows: a CDR-L1 with a sequence as set forth in SEQ ID NO: 6, a CDR-L2 with a sequence as set forth in SEQ ID NO: 7, a CDR-L3 with a sequence as set forth in SEQ ID NO: 8;
(b) a heavy chain variable region (VH) comprising 3 CDRs as follows: a CDR-H1 with a sequence as set forth in SEQ ID NO: 19, a CDR-H2 with a sequence as set forth in SEQ ID NO: 20, a CDR-H3 with a sequence as set forth in SEQ ID NO: 21; and/or,
   a light chain variable region (VL) comprising 3 CDRs as follows: a CDR-L1 with a sequence as set forth in SEQ ID NO: 22, a CDR-L2 with a sequence as set forth in SEQ ID NO: 23, a CDR-L3 with a sequence as set forth in SEQ ID NO: 24;
(c) a heavy chain variable region (VH) comprising 3 CDRs as follows: a CDR-H1 with a sequence as set forth in SEQ ID NO: 32, a CDR-H2 with a sequence as set forth in SEQ ID NO: 33, a CDR-H3 with a sequence as set forth in SEQ ID NO: 34; and/or,
   a light chain variable region (VL) comprising 3 CDRs as follows: a CDR-L1 with a sequence as set forth in SEQ ID NO: 35, a CDR-L2 with a sequence as set forth in SEQ ID NO: 23, a CDR-L3 with a sequence as set forth in SEQ ID NO: 24;
(d) a heavy chain variable region (VH) comprising 3 CDRs as follows: a CDR-H1 with a sequence as set forth in SEQ ID NO: 42, a CDR-H2 with a sequence as set forth in SEQ ID NO: 43, a CDR-H3 with a sequence as set forth in SEQ ID NO: 44; and/or,
   a light chain variable region (VL) comprising 3 CDRs as follows: a CDR-L1 with a sequence as set forth in SEQ ID NO: 45, a CDR-L2 with a sequence as set forth in SEQ ID NO: 46, a CDR-L3 with a sequence as set forth in SEQ ID NO: 47;
   or
(e) a heavy chain variable region (VH) comprising 3 CDRs as follows: a CDR-H1 with a sequence as set forth in SEQ ID NO: 55, a CDR-H2 with a sequence as set forth in SEQ ID NO: 56, a CDR-H3 with a sequence as set forth in SEQ ID NO: 57; and/or,
   a light chain variable region (VL) comprising 3 CDRs as follows: a CDR-L1 with a sequence as set forth in SEQ ID NO: 58, a CDR-L2 with a sequence as set forth in SEQ ID NO: 59, a CDR-L3 with a sequence as set forth in SEQ ID NO: 60.

In certain embodiments, the antibody or antigen-binding fragment thereof according to the invention comprises a heavy chain variable region (VH) and/or a light chain variable region (VL) as follows, wherein CDRs are defined by the AbM numbering system:
(a) a heavy chain variable region (VH) comprising 3 CDRs as follows: a CDR-H1 with a sequence as set forth in SEQ ID NO: 9 or a sequence of a substitution, deletion or addition of one or several amino acids (for example, a substitution, deletion or addition of 1, 2 or 3 amino acids) as compared to SEQ ID NO: 9, a CDR-H2 with a sequence as set forth in SEQ ID NO: 10 or a sequence of a substitution, deletion or addition of one or several amino acids (for example, a substitution, deletion or addition of 1, 2 or 3 amino acids) as compared to SEQ ID NO: 10, a CDR-H3 with a sequence as set forth in SEQ ID NO: 11 or a sequence of a substitution, deletion or addition of one or several amino acids (for example, a substitution, deletion or addition of 1, 2 or 3 amino acids) as compared to SEQ ID NO: 11; and/or,
   a light chain variable region (VL) comprising 3 CDRs as follows: a CDR-L1 with a sequence as set forth in SEQ ID NO: 12 or a sequence of a substitution, deletion or addition of one or several amino acids (for example, a substitution, deletion or addition of 1, 2 or 3 amino acids) as compared to SEQ ID NO: 12, a CDR-L2 with a sequence as set forth in SEQ ID NO: 13 or a sequence of a substitution, deletion or addition of one or several amino acids (for example, a substitution, deletion or addition of 1, 2 or 3 amino acids) as compared to SEQ ID NO: 13, a CDR-L3 with a sequence as set forth in SEQ ID NO: 8 or a sequence of a substitution, deletion or addition of one or several amino acids (for example, a substitution, deletion or addition of 1, 2 or 3 amino acids) as compared to SEQ ID NO: 8;
(b) a heavy chain variable region (VH) comprising 3 CDRs as follows: a CDR-H1 with a sequence as set forth in SEQ ID NO: 25 or a sequence of a substitution, deletion or addition of one or several amino acids (for example, a substitution, deletion or addition of 1, 2 or 3 amino acids) as compared to SEQ ID NO: 25, a CDR-H2 with a sequence as set forth in SEQ ID NO: 26 or a sequence of a substitution, deletion or addition of one or several amino acids (for example, a substitution, deletion or addition of 1, 2 or 3 amino acids) as compared to SEQ ID NO: 26, a CDR-H3 with a sequence as set forth in SEQ ID NO: 27 or a sequence of a substitution, deletion or addition of one or several amino acids (for example, a substitution, deletion or addition of 1, 2 or 3 amino acids) as compared to SEQ ID NO: 27; and/or,
   a light chain variable region (VL) comprising 3 CDRs as follows: a CDR-L1 with a sequence as set forth in SEQ ID NO: 28 or a sequence of a substitution, deletion or addition of one or several amino acids (for example, a substitution, deletion or addition of 1, 2 or 3 amino acids) as compared to SEQ ID NO: 28, a CDR-L2 with a sequence as set forth in SEQ ID NO: 29 or a sequence of a substitution, deletion or addition of one or several amino acids (for example, a substitution, deletion or addition of 1, 2 or 3 amino acids) as compared to SEQ ID NO: 29, a CDR-L3 with a sequence as set forth in SEQ ID NO:24 or a sequence of a substitution, deletion or addition of one or several amino acids (for example, a substitution, deletion or addition of 1, 2 or 3 amino acids) as compared to SEQ ID NO: 24;
(c) a heavy chain variable region (VH) comprising 3 CDRs as follows: a CDR-H1 with a sequence as set forth in SEQ ID NO: 36 or a sequence of a substitution, deletion or addition of one or several amino acids (for example, a substitution, deletion or addition of 1, 2 or 3 amino acids) as compared to SEQ ID NO: 36, a CDR-H2 with a sequence as set forth in SEQ ID NO: 37 or a sequence of a substitution, deletion or addition of one or several amino acids (for example, a substitution, deletion or addition of 1, 2 or 3 amino acids) as compared to SEQ ID NO: 37, a CDR-H3 with a sequence as set forth in SEQ ID NO: 38 or a sequence of a substitution, deletion or addition of one or several amino acids (for example, a substitution, deletion or addition of 1, 2 or 3 amino acids) as compared to SEQ ID NO: 38; and/or,
   a light chain variable region (VL) comprising 3 CDRs as follows: a CDR-L1 with a sequence as set forth in SEQ ID NO: 39 or a sequence of a substitution, deletion or addition of one or several amino acids (for example, a substitution, deletion or addition of 1, 2 or 3 amino acids) as compared to SEQ ID NO: 39, a CDR-L2 with a sequence as set forth in SEQ ID NO: 29 or a sequence of a substitution, deletion or addition of one or several amino acids (for example, a substitution, deletion or addition of 1, 2 or 3 amino acids) as compared to SEQ ID NO: 29, a CDR-L3 with a sequence as set forth in SEQ ID NO: 24 or a sequence of a substitution, deletion or addition of one or several amino acids (for example, a substitution, deletion or addition of 1, 2 or 3 amino acids) as compared to SEQ ID NO: 24;
(d) a heavy chain variable region (VH) comprising 3 CDRs as follows: a CDR-H1 with a sequence as set forth in SEQ ID NO: 48 or a sequence of a substitution, deletion or addition of one or several amino acids (for example, a substitution, deletion or addition of 1, 2 or 3 amino acids) as compared to SEQ ID NO: 48, a CDR-H2 with a sequence as set forth in SEQ ID NO: 49 or a sequence of a substitution, deletion or addition of one or several amino acids (for example, a substitution, deletion or addition of 1, 2 or 3 amino acids) as compared to SEQ ID NO: 49, a CDR-H3 with a sequence as set forth in SEQ ID NO: 50 or a sequence of a substitution, deletion or addition of one or several amino acids (for example, a substitution, deletion or addition of 1, 2 or 3 amino acids) as compared to SEQ ID NO: 50; and/or,
   a light chain variable region (VL) comprising 3 CDRs as follows: a CDR-L1 with a sequence as set forth in SEQ ID NO: 51 or a sequence of a substitution, deletion or addition of one or several amino acids (for example, a substitution, deletion or addition of 1, 2 or 3 amino acids) as compared to SEQ ID NO: 51, a CDR-L2 with a sequence as set forth in SEQ ID NO: 52 or a sequence of a substitution, deletion or addition of one or several amino acids (for example, a substitution, deletion or addition of 1, 2 or 3 amino acids) as compared to SEQ ID NO: 52, a CDR-L3 with a sequence as set forth in SEQ ID NO:47 or a sequence of a substitution, deletion or addition of one or several amino acids (for example, a substitution, deletion or addition of 1, 2 or 3 amino acids) as compared to SEQ ID NO: 47;
   or
(e) a heavy chain variable region (VH) comprising 3 CDRs as follows: a CDR-H1 with a sequence as set forth in SEQ ID NO: 61 or a sequence of a substitution, deletion or addition of one or several amino acids (for example, a substitution, deletion or addition of 1, 2 or 3 amino acids) as compared to SEQ ID NO: 61, a CDR-H2 with a sequence as set forth in SEQ ID NO: 62 or a sequence of a substitution, deletion or addition of one or several amino acids (for example, a substitution, deletion or addition of 1, 2 or 3 amino acids) as compared to SEQ ID NO: 62, a CDR-H3 with a sequence as set forth in SEQ ID NO: 63 or a sequence of a substitution, deletion or addition of one or several amino acids (for example, a substitution, deletion or addition of 1, 2 or 3 amino acids) as compared to SEQ ID NO: 63; and/or,
   a light chain variable region (VL) comprising 3 CDRs as follows: a CDR-L1 with a sequence as set forth in SEQ ID NO: 64 or a sequence of a substitution, deletion or addition of one or several amino acids (for example, a substitution, deletion or addition of 1, 2 or 3 amino acids) as compared to SEQ ID NO: 64, a CDR-L2 with a sequence as set forth in SEQ ID NO: 65 or a sequence of a substitution, deletion or addition of one or several amino acids (for example, a substitution, deletion or addition of 1, 2 or 3 amino acids) as compared to SEQ ID NO: 65, a CDR-L3 with a sequence as set forth in SEQ ID NO: 60 or a sequence of a substitution, deletion or addition of one or several amino acids (for example, a substitution, deletion or addition of 1, 2 or 3 amino acids) as compared to SEQ ID NO: 60.

In certain embodiments, the antibody or antigen-binding fragment thereof according to the invention comprises a heavy chain variable region (VH) and/or a light chain variable region (VL) as follows, wherein CDR are defined by the AbM numbering system:
(a) a heavy chain variable region (VH) comprising 3 CDRs as follows: a CDR-H1 with a sequence as set forth in SEQ ID NO: 9, a CDR-H2 with a sequence as set forth in SEQ ID NO: 10, a CDR-H3 with a sequence as set forth in SEQ ID NO: 11; and/or,
   a light chain variable region (VL) comprising 3 CDRs as follows: a CDR-L1 with a sequence as set forth in SEQ ID NO: 12, a CDR-L2 with a sequence as set forth in SEQ ID NO: 13, a CDR-L3 with a sequence as set forth in SEQ ID NO: 8;
(b) a heavy chain variable region (VH) comprising 3 CDRs as follows: a CDR-H1 with a sequence as set forth in SEQ ID NO: 25, a CDR-H2 with a sequence as set forth in SEQ ID NO: 26, a CDR-H3 with a sequence as set forth in SEQ ID NO: 27; and/or,
   a light chain variable region (VL) comprising 3 CDRs as follows: a CDR-L1 with a sequence as set forth in SEQ ID NO: 28, a CDR-L2 with a sequence as set forth in SEQ ID NO: 29, a CDR-L3 with a sequence as set forth in SEQ ID NO: 24;
(c) a heavy chain variable region (VH) comprising 3 CDRs as follows: a CDR-H1 with a sequence as set forth in SEQ ID NO: 36, a CDR-H2 with a sequence as set forth in SEQ ID NO: 37, a CDR-H3 with a sequence as set forth in SEQ ID NO: 38; and/or,
   a light chain variable region (VL) comprising 3 CDRs as follows: a CDR-L1 with a sequence as set forth in SEQ ID NO: 39, a CDR-L2 with a sequence as set forth in SEQ ID NO: 29, a CDR-L3 with a sequence as set forth in SEQ ID NO: 24;
(d) a heavy chain variable region (VH) comprising 3 CDRs as follows: a CDR-H1 with a sequence as set forth in SEQ ID NO: 48, a CDR-H2 with a sequence as set forth in SEQ ID NO: 49, a CDR-H3 with a sequence as set forth in SEQ ID NO: 50; and/or,
   a light chain variable region (VL) comprising 3 CDRs as follows: a CDR-L1 with a sequence as set forth in SEQ ID NO: 51, a CDR-L2 with a sequence as set forth in SEQ ID NO: 52, a CDR-L3 with a sequence as set forth in SEQ ID NO: 47;
   or
(e) a heavy chain variable region (VH) comprising 3 CDRs as follows: a CDR-H1 with a sequence as set forth in SEQ ID NO: 61, a CDR-H2 with a sequence as set forth in SEQ ID NO: 62, a CDR-H3 with a sequence as set forth in SEQ ID NO: 63; and/or,
   a light chain variable region (VL) comprising 3 CDRs as follows: a CDR-L1 with a sequence as set forth in SEQ ID NO: 64, a CDR-L2 with a sequence as set forth in SEQ ID NO: 65, a CDR-L3 with a sequence as set forth in SEQ ID NO: 60.

In certain embodiments, the antibody or antigen-binding fragment thereof according to the invention comprises a heavy chain variable region (VH) and/or a light chain variable region (VL), wherein, as compared to the CDRs defined by IMGT or AbM as described above, at least one CDR in the heavy chain variable region (VH) and/or light chain variable region (VL) contains a mutation, wherein the mutation is a substitution, deletion or addition of one or several amino acids or any combination thereof (for example, a substitution, deletion or addition of 1, 2 or 3 amino acids, or any combination thereof).

Preferably, the substitution of the invention is a conservative substitution.

In certain embodiments, the VH of the antibody or antigen-binding fragment thereof according to the invention comprises framework regions (FRs) derived from a heavy chain variable region (VH) of a human immunoglobulin, and/or the VL of the antibody or antigen-binding fragment thereof comprises framework regions (FRs) derived from a light chain variable region (VL) of a human immunoglobulin. Therefore, in certain embodiments, the antibody or antigen-binding fragment thereof according to the invention is humanized. In certain embodiments, the antibody or antigen-binding fragment thereof according to the invention is of fully human.

In certain embodiments, the antibody or antigen-binding fragment thereof according to the invention comprises:
(a) a heavy chain framework region of a human immunoglobulin or a variant thereof, wherein the variant has conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids), as compared to the amino acid sequence encoded by the germline antibody gene from which it is derived; and/or
(b) a light chain framework region of a human immunoglobulin or a variant thereof, wherein the variant has conservative substitutions of up to 20 amino acids (e.g. conservative substitutions of up to 20, up to 15, up to 10, or up to 5 amino acids; e.g., conservative substitutions of 1, 2, 3, 4, 5, 6, 7, 8, 9, or 10 amino acids), as compared to the amino acid sequence encoded by the germline antibody gene sequence from which it is derived.

In certain embodiments, the humanization degree of the antibody or antigen-binding fragment thereof of the invention is at least 75%, at least 80%, at least 85%, at least 90%, at least 91%, at least 92%, at least 93%, at least 94%, at least 95%, at least 96%, at least 97%, at least 98% or at least 99%.

In certain embodiments, the antibody or antigen-binding fragment thereof according to the invention comprises:
(a) a heavy chain variable region (VH) comprising an amino acid sequence selected from the group consisting of:
   (i) the sequence as set forth in SEQ ID NO: 1, 17, 30, 40, 53 or 68;
   (ii) a sequence comprising a substitution, deletion or addition of one or several amino acids or any combination thereof (for example, a substitution, deletion or addition of 1, 2, 3, 4, 5, 6, 7, 8, 9 or 10 amino acids, or any combination thereof), as compared to the sequence as set forth in SEQ ID NO: 1, 17, 30, 40, 53 or 68; or
   (iii) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity to the sequence as set forth in SEQ ID NO: 1, 17, 30, 40, 53 or 68;
      and/or
(b) a light chain variable region (VL) comprising an amino acid sequence selected from the group consisting of:
   (iv) the sequence as set forth in SEQ ID NO: 2, 18, 31, 41, 54 or 69;
   (v) a sequence comprising a substitution, deletion or addition of one or several amino acids or any combination thereof (for example, a substitution, deletion or addition of 1, 2, 3, 4, 5, 6, 7, 8, 9 or 10 amino acids, or any combination thereof), as compared to the sequence as set forth in SEQ ID NO: 2, 18, 31, 41, 54 or 69; or
   (vi) a sequence having at least 70%, at least 75%, at least 80%, at least 85%, at least 90%, at least 91%, at least 92%, at least 93%, at least 94%, at least 95%, at least 96%, at least 97%, at least 98% or at least 99% sequence identity to the sequence as set forth in SEQ ID NO: 2, 18, 31, 41, 54 or 69.

In certain embodiments, the antibody or antigen-binding fragment thereof according to the invention comprises a VH as set forth in SEQ ID NO: 1, and/or, a VL as set forth in SEQ ID NO: 2.

In certain embodiments, the antibody or antigen-binding fragment thereof according to the invention comprises a VH as set forth in SEQ ID NO: 17, and/or, a VL as set forth in SEQ ID NO: 18.

In certain embodiments, the antibody or antigen-binding fragment thereof according to the invention comprises a VH as set forth in SEQ ID NO: 30, and/or, a VL as set forth in SEQ ID NO: 31.

In certain embodiments, the antibody or antigen-binding fragment thereof according to the invention comprises a VH as set forth in SEQ ID NO: 40, and/or, a VL as set forth in SEQ ID NO: 41.

In certain embodiments, the antibody or antigen-binding fragment thereof according to the invention comprises a VH as set forth in SEQ ID NO: 53, and/or, a VL as set forth in SEQ ID NO: 54.

In certain embodiments, the antibody or antigen-binding fragment thereof according to the invention comprises a VH as set forth in SEQ ID NO: 68, and/or, a VL as set forth in SEQ ID NO: 69.

In certain embodiments, the antibody or antigen-binding fragment thereof according to the invention comprises:
(a) a VH as set forth in SEQ ID NO: 1 and a VL as set forth in SEQ ID NO: 2;
(b) a VH as set forth in SEQ ID NO: 17 and a VL as set forth in SEQ ID NO: 18;
(c) a VH as set forth in SEQ ID NO: 30 and a VL as set forth in SEQ ID NO: 31;
(d) a VH as set forth in SEQ ID NO: 40 and a VL as set forth in SEQ ID NO: 41;
(e) a VH as set forth in SEQ ID NO: 53 and a VL as set forth in SEQ ID NO: 54;
(f) a VH as set forth in SEQ ID NO: 68 and a VL as set forth in SEQ ID NO: 69;
(g) a heavy chain variable region (VH) and a light chain variable region (VL), wherein the heavy chain variable region (VH) and the light chain variable region (VL) independently have at least 70%, at least 80%, at least 85%, at least 90%, at least 91%, at least 92%, at least 93%, at least 94%, at least 95%, at least 96%, at least 97%, at least 98% or at least 99% sequence identity to the VH and the VL in any one of (a) to (f), respectively; or
(h) a heavy chain variable region (VH) and a light chain variable region (VL), wherein the heavy chain variable region (VH) and the light chain variable region (VL) independently have a substitution, deletion or addition of one or several amino acids or any combination thereof (for example, a substitution, deletion or addition of 1, 2, 3, 4, 5, 6, 7, 8, 9 or 10 amino acids, or any combination thereof), as compared to the VH and the VL in any one of (a) to (f), respectively. Preferably, the substitution is a conservative substitution.

In certain embodiments, the heavy chain of the antibody or antigen-binding fragment thereof of the invention comprises a heavy chain constant region (CH) of a human immunoglobulin or a variant thereof, wherein the variant has conservative substitutions of up to 50 amino acids (for example, conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9, or 10 amino acids), as compared to the wild-type sequence from which it is derived. In certain embodiments, the light chain of the antibody or antigen-binding fragment thereof of the invention comprises a light chain constant region (CL) of a human immunoglobulin or a variant thereof, wherein the variant has conservative substitutions of up to 50 amino acids (for example, conservative substitutions of up to 45, up to 40, up to 35, up to 30, up to 25, up to 20, up to 15, up to 10, or up to 5 amino acids; e.g., conservative substitutions of 1, 2, 3, 4, 5, 6, 7, 8, 9, or 10 amino acids), as compared to the wild-type sequence from which it is derived.

In certain embodiments, the constant region is altered, e.g., mutated, to modify the properties of the anti-TSLP antibody molecule (e.g., altering one or more of the following characteristics: Fc receptor binding, antibody glycosylation, the number of cysteine residues, effector cell functions, or complement functions). The functions can be altered by replacing at least one amino acid residue in the antibody constant region with a different residue, for example, to alter the affinity of the antibody for an effector ligand (e.g., FcR or complement C1q), thereby changing (e.g., decreasing) effector functions. The Fc region of an antibody mediates several important effector functions, such as ADCC, phagocytosis (ADCP), CDC and so on.

In certain embodiments, the antibody or antigen-binding fragment thereof according to the invention comprises a heavy chain constant region (Fc), which is selected from the heavy chain constant regions, for example, of IgG1, IgG2, IgG3, IgG4, IgM, IgA1, IgA2, IgD and IgE; particularly of IgG1, IgG2, IgG3 and IgG4, and more particularly of IgG1 (e.g. human IgG1). In some embodiments, the heavy chain constant region of human IgG1 is as set forth in SEQ ID NO: 14. In some embodiments, the antibody or antigen-binding fragment thereof according to the invention comprises a light chain constant region, which is selected from, for example, kappa or lambda light chain constant region, preferably kappa light chain constant region (e.g. a human kappa light chain constant region).

In some embodiments, the antibody or antigen-binding fragment thereof comprises a heavy chain constant region of human IgG1. In some preferred embodiments, the antibody or antigen-binding fragment thereof comprises a constant region of human IgG1 as set forth in uniprot ID P01857 (SEQ ID NO: 74).

In some embodiments, the antibody or antigen-binding fragment thereof comprises a heavy chain constant region of human IgG1 (e.g., SEQ ID NO: 74) or a variant thereof, wherein the variant is mutated at least one of sites 234, 235, 237, 265, 297, 331, 329 and 434 according to the EU numbering system. In some embodiments, the variant comprises at least one of the mutations L234A, L235A, D265A, N297A, L234F, L235E, P331S, P329G, N434A, N434Y, N434F, N434W, N434S, N434G, N434H and N434Q. In some embodiments, the variant comprises at least one of the mutations L234A, L235A, G237A and N434A. In some embodiments, the variant of the heavy chain constant region of IgG1 comprises L234A, L235A and G237A. In some embodiments, the variant of the heavy chain constant region of IgG1 comprises L234A, L235A, G237A and N434A.

In some embodiments, the antibody or antigen-binding fragment thereof comprises the CH as set forth in SEQ ID NO: 14 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 14, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 14. The variant comprises N297A and/or N434A according to the EU numbering system. In some embodiments, the variant comprises N434A.

In some embodiments, the antibody or antigen-binding fragment thereof comprises the CH as set forth in SEQ ID NO: 15 or a variant thereof, wherein the variant has conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 15, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 15.

In certain embodiments, the antibody or antigen-binding fragment thereof comprises a heavy chain constant region of human IgG4 (e.g., SEQ ID NO: 75) or a variant thereof, wherein the variant is mutated at least one of sites 228 and/or 434 according to the EU numbering system.

In some embodiments, the mutant comprises S228P and/or N434A. In some embodiments, the variant of the heavy chain constant region of human IgG4 comprises S228P and N434A.

In some embodiments, the antibody or antigen-binding fragment thereof comprises the CH as set forth in SEQ ID NO: 70 or a variant thereof, wherein the variant has conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 70, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 70.

In some embodiments, the antibody or antigen-binding fragment thereof comprises a light chain constant region or a variant thereof. In some embodiments, the light chain constant region comprises a kappa light chain constant region. In some embodiments, the light chain constant region comprises a light chain constant region (CL) as set forth in SEQ ID NO: 16 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 16, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 16;

In some embodiments, the antibody or antigen-binding fragment thereof comprises a heavy chain constant region (CH) as set forth in SEQ ID NO: 14 and a light chain constant region (CL) as set forth in SEQ ID NO: 16.

In some embodiments, the antibody or antigen-binding fragment thereof comprises a heavy chain constant region (CH) as set forth in SEQ ID NO: 15 and a light chain constant region (CL) as set forth in SEQ ID NO: 16.

In some embodiments, the antibody or antigen-binding fragment thereof comprises a heavy chain constant region (CH) as set forth in SEQ ID NO: 70 and a light chain constant region (CL) as set forth in SEQ ID NO: 16.

In some embodiments, the above mutation(s) renders the antibody or antigen-binding fragment thereof to have no or reduced ADCP, ADCC and/or CDC activity, compared to the corresponding antibody or antigen-binding fragment thereof comprising human IgG4 or the heavy chain constant region of IgG4.

In some embodiments, the above mutation(s) renders the antibody or antigen-binding fragment thereof to have no or reduced ADCP, ADCC and/or CDC activity, compared to the corresponding antibody or antigen-binding fragment thereof having not the mutation or substitution described above.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 1 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof), compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 2 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof), compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 17 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof), compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 18 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof), compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 30 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 31 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 40 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 41 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 53 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 54 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody or antigen-binding fragment thereof according to the invention comprises:
(a) a heavy chain comprising an amino acid sequence selected from the group consisting of:
   (i) a sequence comprising a VH sequence as set forth in SEQ ID NO: 68 and a CH sequence as set forth in SEQ ID NO: 14, 15 or 70;
   (ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
(b) a light chain comprising an amino acid sequence selected from the group consisting of:
   (iv) a sequence comprising a VL sequence as set forth in SEQ ID NO: 69 and a CL sequence as set forth in SEQ ID NO: 16;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv).

In certain embodiments, the substitution in (ii) or (v) is a conservative substitution.

In certain embodiments, the antibody of the invention comprises: a heavy chain comprising a VH as set forth in SEQ ID NO: 1 and a heavy chain constant region (CH) as set forth in SEQ ID NO: 14, 15 or 70, and a light chain comprising a VL as set forth in SEQ ID NO: 2 and a light chain constant region (CL) as set forth in SEQ ID NO: 16.

In certain embodiments, the antibody of the invention comprises: a heavy chain comprising a VH as set forth in SEQ ID NO: 17 and a heavy chain constant region (CH) as set forth in SEQ ID NO: 14, 15 or 70, and a light chain comprising a VL as set forth in SEQ ID NO: 18 and a light chain constant region (CL) as set forth in SEQ ID NO: 16.

In certain embodiments, the antibody of the invention comprises: a heavy chain comprising a VH as set forth in SEQ ID NO: 30 and a heavy chain constant region (CH) as set forth in SEQ ID NO: 14, 15 or 70, and a light chain comprising a VL as set forth in SEQ ID NO: 31 and a light chain constant region (CL) as set forth in SEQ ID NO: 16.

In certain embodiments, the antibody of the invention comprises: a heavy chain comprising a VH as set forth in SEQ ID NO: 40 and a heavy chain constant region (CH) as set forth in SEQ ID NO: 14, 15 or 70, and a light chain comprising a VL as set forth in SEQ ID NO: 41 and a light chain constant region (CL) as set forth in SEQ ID NO: 16.

In certain embodiments, the antibody of the invention comprises: a heavy chain comprising a VH as set forth in SEQ ID NO: 53 and a heavy chain constant region (CH) as set forth in SEQ ID NO: 14, 15 or 70, and a light chain comprising a VL as set forth in SEQ ID NO: 54 and a light chain constant region (CL) as set forth in SEQ ID NO: 16.

In certain embodiments, the antibody of the invention comprises: a heavy chain comprising a VH as set forth in SEQ ID NO: 68 and a heavy chain constant region (CH) as set forth in SEQ ID NO: 14, 15 or 70, and a light chain comprising a VL as set forth in SEQ ID NO: 69 and a light chain constant region (CL) as set forth in SEQ ID NO: 16.

In certain embodiments, the antibody or antigen-binding fragment thereof according to the invention comprises a heavy chain and a light chain,
wherein the heavy chain comprises:
(i) a sequence as set forth in SEQ ID NO: 66 or 73;
(ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
(iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
   the light chain comprises:
   (iv) a sequence as set forth in SEQ ID NO: 67;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv);
   preferably, the substitution in (ii) or (v) is a conservative substitution.

In certain embodiments, the antibody or antigen-binding fragment thereof according to the invention comprises a heavy chain and a light chain,
wherein the heavy chain comprises:
(i) a sequence as set forth in SEQ ID NO: 71;
(ii) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (i); or
(iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
   the light chain comprises:
   (iv) a sequence as set forth in SEQ ID NO: 72;
   (v) a sequence having a substitution, deletion or addition of one or several amino acids or any combination thereof (for example, a substitution, deletion or addition of up to 50, up to 45, up to 40, up to 35, up to 30, up to 25, up to 20, up to 15, up to 10 or up to 5 amino acids or any combination thereof; for example, a substitution, deletion or addition of 1, 2, 3, 4, 5, 6, 7, 8, 9 or 10 amino acids, or any combination thereof) compared to the sequence in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv);
   preferably, the substitution in (ii) or (v) is a conservative substitution.

In certain embodiments, the antibody of the invention is a chimeric, humanized, or fully human antibody. In certain embodiments, the antibody or antigen-binding fragment thereof according to the invention is selected from the group consisting of scFv, Fab, Fab', (Fab')₂, Fv fragments, disulfide linked Fv (dsFv) and diabody.

In certain embodiments, the antibody or antigen-binding fragment thereof according to the invention may exhibit at least one of the following properties:
(a) binding to TSLP (e.g., human TSLP) with a KD of less than about 50 nM, e.g., less than about 40 nM, 30 nM, 20 nM, 10 nM, 1 nM, 0.1 nM, 1pM, 0.1 pM or less, wherein the KD is measured by techniques well known in the art, such as Fortibio or ELISA;
(b) binding to TSLP (e.g., human TSLP) with an EC50 of less than about 50 nM, e.g., less than about 40 nM, 30 nM, 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1pM, 0.1 pM or less, wherein the EC50 is measured by techniques well known in the art, such as flow cytometry or ELISA, for example, affinity ELISA or cell competitive ELISA;
(c) inhibiting the binding of TSLP to IL7Rα/TSLPR with an IC50 of less than about 50 nM, e.g., about 50 nM, 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1pM, 0.1 pM or less, wherein the IC50 is measured by ELISA;
(d) inhibiting or blocking TSLP-induced OX40L expression; |
(e) inhibiting or blocking TSLP-induced activation and/or proliferation of mast cells, DC, NKT cells;
(f) inhibiting or blocking TSLP-induced osteoprotegerin (OPG) secretion;
(g) inhibiting or blocking secretion of Th2 Cytokines, such as TARC, CCL22, IL-4, IL-13, or IL-5;
(h) good affinity for binding to FcRn;
(i) an isoelectric point (PI) of about 6.5 to about 8.5, such as about 6.5, about 7.0, about 7.1, about 7.2, about 7.3, about 7.4, about 7.5, about 7.7, about 7.9, about 8.0, about 8.2 or about 8.5.

In addition, the antibody of the invention has a good affinity for FcRn. In certain embodiments, the KD (M) value of affinity for FcRn is at the level of 10⁻⁹. The antibody of the invention has a longer half-life in vivo. The antibody of the invention also has a good hydrophilicity. In certain embodiments, the hydrophobic time of the antibody of the invention is assayed to be between 8 minutes and 14 minutes by a chromatographic column.

### Antibody Derivatives

The antibody or antigen-binding fragment thereof according to the invention may be derivatized, for example, by being linked to another molecule (e.g., another polypeptide or protein). Typically, derivatization (e.g., labeling) of an antibody or antigen-binding fragment thereof does not adversely affect its binding to TSLP (particularly human TSLP). Thus, the antibody or antigen-binding fragment thereof according to the invention also intends to cover such derivative forms. For example, the antibody or antigen-binding fragment thereof according to the invention may be linked to (by chemical coupling, gene fusion, non-covalently or other means) one or more other molecular moieties, e.g., another antibody (e.g., forming a bispecific antibody), a detection reagent, a pharmaceutical reagent, and/or a protein or polypeptide capable of mediating the binding of the antibody or the antigen-binding fragment to another molecule (e.g., avidin or a polyhistidine label).

One type of derivatized antibody (e.g., a bispecific antibody) is generated by cross-linking two or more antibodies (belonging to the same or different types). Methods for obtaining a bispecific antibody are well known in the art, and exemplary methods include, but not limited to, chemical cross-linking, cell engineering (hybridoma) or genetic engineering.

Another type of derivatized antibody is a labeled antibody. For example, the antibody or antigen-binding fragment thereof according to the invention may be linked to a detectable marker. The detectable marker of the invention may be any substance detectable by fluorescence, spectroscopy, photochemistry, biochemistry, immunology, electricity, optics, or chemistry. Such markers are well known in the art and the examples include, but not limited to, enzymes (e.g., horseradish peroxidase, alkaline phosphatase, beta-galactosidase, urease, glucose oxidase, etc.), radionuclides (e.g.,3H, 125I, 35S, 14C or 32P), fluorescent dyes (e.g., fluorescein isothiocyanate (FITC), fluorescein, tetramethyl rhodamine isothiocyanate (TRITC), phycoerythrin (PE), Texas red, rhodamine, quantum dots or cyanine dye derivatives (e.g., Cy7, Alexa 750)), acridine ester compounds, magnetic beads (e.g., Dynabeads ^{®}), calorimetric markers such as colloidal gold or colored glass or plastic (e.g. polystyrene, polypropylene, latex, etc.) beads, and biotin used to bind to avidin (e.g. streptavidin) modified by the above markers. The use of such markers was taught in those including, but limited to, U.S. Pat. 3,817,837; 3,850,752; 3,939,350; 3,996,345; 4,277,437; 4,275,149; and 4,366,241, all of which are incorporated herein by reference. The detectable marker described above can be detected by methods known in the art. For example, a radioactive marker may be detected using a photographic film or a scintillation calculator, and a fluorescent marker may be detected using a photodetector to detect the emitted light. An enzyme marker may be generally detected by providing a substrate for the enzyme and detecting the reaction product produced by the action of the enzyme on the substrate; a calorimetric marker may be detected by a simple visual coloring marker. In certain embodiments, such markers may be useful in immunoassays (e.g., enzyme-linked immunoassay, radioimmunoassay, fluorescence immunoassay, chemiluminescence immunoassay, etc.). In certain embodiments, the detectable markers described above can be linked to the antibody or antigen-binding fragment thereof according to the invention through linkers of different lengths to reduce potential steric hindrance.

In addition, the antibody or antigen-binding fragment thereof according to the invention may also be derivatized by using chemical groups, such as polyethylene glycol (PEG), methyl or ethyl, or glycosyl. These groups can be used to improve the biological characteristics of the antibody, such as increase the serum half-life.

Therefore, in one aspect of the invention, a conjugate comprising the monoclonal antibody or antigen-binding fragment thereof according to the invention and a coupling moiety is provided, wherein the coupling moiety is a detectable marker as described above, such as a radioisotope, a fluorescent substance, a luminescent substance, a colored substance, or an enzyme. The coupling moiety may also be a therapeutic agent.

As one of the derivatives of the antibody, the invention provides a multispecific antibody comprising a first antibody or fragment thereof, and an additional antibody or fragment thereof, or an antibody mimetic, wherein the first antibody or fragment thereof, the additional antibody or fragment thereof, or the antibody mimetic retains its original binding specificity. The first antibody or fragment thereof is any one of (monoclonal) antibodies or antigen-binding fragments thereof according to the invention binding to TSLP. As used herein, "antibody mimetic" refers to a substance that specifically binds to an antigen like an antibody, but without an antibody structure. They are usually artificial peptides or proteins having a molar mass of about 3 to 20 kDa, for example, a designed ankyrin repeat protein (DARPin) and fynomer. The designed ankyrin repeat protein (DARPin) can be linked to an IgG antibody, a scFv-Fc antibody fragment or the combination thereof, as described in CN104341529A. A bispecific fusion polypeptide is generated by fusing a fynomer against IL-17a to an anti-IL-6R antibody, as described in WO2015141862A1.

In certain embodiments, the multispecific antibody is formed by coupling the first antibody or antigen-binding fragment thereof with other antibody or antigen-binding fragment thereof or antibody mimetic, wherein each antibody or its antigen-binding fragment or antibody mimetic retains its original binding specificity, and the first antibody or antigen-binding fragment thereof is the antibody or antigen-binding fragment thereof according to the invention. In certain embodiments, the multispecific antibody is a bispecific antibody or trispecific antibody or tetraspecific antibody.

### Antibody Preparation

The antibody of the invention can be prepared by various methods known in the art, e.g., genetic engineering recombinant technology. For example, a DNA molecule encoding heavy and light chain genes of the antibody of the invention is obtained by chemical synthesis or PCR amplification. The obtained DNA molecule is inserted into an expression vector followed by transfecting host cells. Then, the transfected host cells are cultured under specific conditions to express the antibody of the invention.

The antigen-binding fragments of the invention can be obtained by hydrolyzing an intact antibody molecule (See Morimoto et al., J. Biochem. Biophys. Methods 24: 107-117 (1992) and Brennan et al., Science 229: 81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be isolated directly from the culture of recombinant host cells. Other technologies for preparing these antigen-binding fragments are well known to those of ordinary skill in the art.

Thus, in another aspect, the invention provides an isolated nucleic acid molecule comprising a nucleotide sequence encoding the antibody or antigen-binding fragment thereof according to the invention, or a heavy chain variable region and/or a light chain variable region thereof, or one or more CDRs thereof. In view of the degeneracy of codons known in the art, in some embodiments, the nucleotide sequence may be varied in accordance with the degeneracy of codons. In certain embodiments, the nucleotide sequence is codon optimized.

In certain embodiments, the isolated nucleic acid molecule according to the invention comprises: (i) a first nucleic acid and a second nucleic acid encoding a heavy chain variable region and a light chain variable region of the antibody or antigen-binding fragment thereof according to the invention, respectively, or (ii) a first nucleic acid encoding a heavy chain variable region and a heavy chain constant region, and a second nucleic acid encoding a light chain variable region and a light chain constant region, of the antibody or antigen-binding fragment thereof according to the invention, or (iii) a first nucleic acid and a second nucleic acid encoding a heavy chain and a light chain of the antibody or antigen-binding fragment thereof according to the invention, respectively. In certain embodiments, the first and second nucleic acids comprise nucleic acids having a degenerate sequence of, or a substantially identical sequence to, any one of the first and second nucleic acids in the above (i)-(iii). In certain embodiments, the degenerate sequence or substantially identical sequence refers to a sequence having at least about 85%, 90%, 95%, 99% or greater sequence identity or having one or more nucleotide substitutions or having a difference of not more than 3, 6, 15, 30 or 45 nucleotides, as compared to the nucleic acid molecules in (i)-(iii).

In another aspect, is provided a vector (e.g., a cloning vector or an expression vector), which comprises the isolated nucleic acid molecule according to the invention. In certain embodiments, the vector according to the invention is, for example, a plasmid, cosmid, phage, lentivirus or the like. In certain embodiments, the vector is capable of expressing the antibody or antigen-binding fragment thereof according to the invention in vivo in a subject (e.g., a mammal, e.g., a human).

In another aspect, is provided a host cell comprising the isolated nucleic acid molecule according to the invention or the vector according to the invention. The host cell may be a eukaryotic cell (e.g., a mammalian cell, an insect cell, a yeast cell) or a prokaryotic cell (e.g., Escherichia coli). Suitable eukaryotic cells include, but not limited to, NS0 cells, Vero cells, Hela cells, COS cells, CHO cells, HEK293 cells, BHK cells, and MDCKII cells. Suitable insect cells include, but not limited to, Sf9 cells. In certain embodiments, the host cell of the invention is a mammalian cell, such as CHO (e.g., CHO-K1, CHO-S, CHO DXB11, CHO DG44).

In another aspect, is provided a method of preparing the antibody or antigen-binding fragment thereof according to the invention, comprising culturing the host cell according to the invention under conditions allowing expression of the antibody or antigen-binding fragment thereof, and recovering the antibody or antigen-binding fragment thereof from the cultured host cell culture.

### Uses, therapeutic methods and pharmaceutical compositions

In another aspect of the invention, is provided a pharmaceutical composition comprising the antibody or antigen-binding fragment thereof, the nucleic acid, the vector, the host cell, the multispecific antibody, and/or the conjugate according to the invention, and a pharmaceutically acceptable carrier and/or excipient.

In certain embodiments, the pharmaceutical composition according to the invention comprises the antibody or antigen-binding fragment thereof according to the invention, and a pharmaceutically acceptable carrier and/or excipient.

In certain embodiments, the pharmaceutical composition according to the invention comprises the host cell according to the invention and a pharmaceutically acceptable carrier and/or excipient, wherein the host cell comprises the isolated nucleic acid molecule or the vector described above.

In certain embodiments, the pharmaceutical composition according to the invention comprises the multispecific antibody according to the invention, and a pharmaceutically acceptable carrier and/or excipient.

In certain embodiments, the pharmaceutical composition according to the invention comprises the conjugate according to the invention, and a pharmaceutically acceptable carrier and/or excipient.

In another aspect, the antibody or antigen-binding fragment thereof, the nucleic acid, the vector, the host cell, the multispecific antibody or the conjugate in the pharmaceutical composition according to the invention is to produce at least one of the following biological activities in a subject:
(1) inhibiting or blocking the binding of TSLP to TSLPR/IL7Rα;
(2) down-regulating or eliminating the activity of TSLP;
(3) down-regulating or blocking OX40L expression;
(4) inhibiting or blocking TSLP-induced osteoprotegerin (OPG) secretion;
(5) inhibiting or blocking the secretion of Th2-like cytokines such as TARC, CCL22, IL-4, IL-13, or IL-5;
(6) inhibiting or blocking TSLP-induced activation and/or proliferation of mast cells, DC, NKT cells.

The antibody or antigen-binding fragment thereof, the nucleic acid, the vector, the host cell, the multispecific antibody or the conjugate in the pharmaceutical composition of the invention is capable of inhibiting or blocking the binding of TSLP to TSLPR/IL7Rα. The binding of TSLP to TSLPR/IL7Rα may cause a variety of allergic inflammatory diseases, including allergic diseases and non-allergic diseases. These diseases include, but not limited to, asthma (including severe asthma), idiopathic pulmonary fibrosis, atopic dermatitis (AD), allergic conjunctivitis, allergic rhinitis (AR), Netherton syndrome (NS), eosinophilic esophagitis (EoE), food allergy, allergic diarrhea, eosinophilic gastroenteritis, allergic bronchopulmonary aspergillosis (ABPA), allergic fungal sinusitis, rheumatoid arthritis, COPD, systemic sclerosis, keloid, ulcerative colitis, chronic sinusitis (CRS) and nasal polyps, chronic eosinophilic pneumonia, eosinophilic bronchitis; abdominal diseases such as eosinophilic gastroenteritis, Churg-Strauss syndrome; eosinophil-related gastrointestinal diseases, such as eosinophilia/eosinophilic granuloma with polyangiitis, eosinophilic esophagitis and inflammatory bowel disease; urticaria, systemic mastocytosis, cutaneous mastocytosis, and recurrent idiopathic angioedema. Therefore, the antibody or antigen-binding fragment thereof, the nucleic acid, the vector, the host cell, the multispecific antibody or the conjugate in the pharmaceutical composition of the invention is capable of preventing or treating the above diseases.

The binding of TSLP to TSLPR/IL7Rα is also associated with autoimmune diseases. Therefore, the antibody or antigen-binding fragment thereof, the nucleic acid, the vector, the host cell, the multispecific antibody or the conjugate in the pharmaceutical composition of the invention is capable of preventing or treating autoimmune diseases, such as diabetes, myasthenia gravis, gastritis, pemphigus, primary biliary cirrhosis, multiple sclerosis, lupus, colitis, rheumatoid diseases, psoriasis and thyroid diseases.

In certain embodiments, in the pharmaceutical composition, the antibody or antigen-binding fragment thereof according to the invention and the additional pharmaceutically active agent are provided as separate components or as components in a single composition. Thus, the antibody or antigen-binding fragment thereof according to the invention can be administered in combination with or separately from, simultaneously with or sequentially with the additional pharmaceutically active agent.

In certain embodiments, the pharmaceutical composition may also comprise an additional pharmaceutical active agent.

The anti-TSLP antibody or antigen-binding fragment thereof according to the invention can be administered alone or in combination with other active agents. The anti-TSLP antibody or antigen-binding fragment thereof according to the invention can be administered separately from, simultaneously with or sequentially with one or more other active agents.

The anti-TSLP antibody or antigen-binding fragment thereof according to the invention can be administered in combination with any suitable immunosuppressant, including, but not limited to, anti-inflammatory agents, in particular by inhaling, intranasal, or parenteral administration of corticosteroids, such as budesonide, beclomethasone dipropionate, fluticasone propionate, ciclesonide, mometasone furoate, fluticasone furoate, fluticasone propionate, budesonide, ciclesonide, beclomethasone dipropionate, mometasone furoate, triamcinolone acetonide and prednisolone. In one embodiment, the anti-TSLP antibody or antigen-binding fragment thereof according to the invention can be administered in combination with a fixed dose of inhaled corticosteroids, such as a fixed dose of fluticasone furoate or fluticasone propionate. In one embodiment, the anti-TSLP antibody or antigen-binding fragment thereof according to the invention can be administered in combination with a non-steroidal glucocorticoid receptor agonist: LTD4 antagonists or LTB4 antagonists, including montelukast, pranlukast, zafirlukast, Accolate, etc.; A2A agonists; A2B antagonists; dopamine receptor agonists; or PDE4 inhibitors. In one embodiment, the anti-TSLP antibody or antigen-binding fragment thereof according to the invention can be administered in combination with pirfenidone or Nintedanib or avB6 antagonists.

The anti-TSLP antibody or antigen-binding fragment thereof according to the invention can be administered in combination with bronchodilators, such as beta-2 adrenergic receptor agonists and/or muscarinic antagonists. Suitable beta-2 adrenergic receptor agonists include vilanterol, salmeterol, salbutamol, formoterol, salmefamol, fenoterol, carmoterol, etanterol, naminterol, clenbuterol, pirbuterol, flerobuterol, reproterol, bambuterol, indacaterol, terbutaline and salts thereof. Suitable muscarinic antagonists include umeclidinium bromide, tiotropium bromide, glycopyrronium bromide, ipratropium, and their salts such as hydrobromide of umeclidinium bromide. In one embodiment, the anti-TSLP antibody or antigen-binding fragment thereof according to the invention can be administered in combination with a fixed dose of beta-2 adrenergic receptor agonists and/or muscarinic antagonists, such as a fixed dose of vilanterol triphenyl acetate or umeclidinium bromide, or both vilanterol triphenyl acetate and umeclidinium bromide.

The anti-TSLP antibody or antigen-binding fragment thereof according to the invention can be administered in combination with one or more bronchodilators and inhaled steroids. Such combination may include a double combination, such as fluticasone furoate and vilanterol triphenyl acetate, fluticasone furoate and umeclidinium bromide, fluticasone propionate and salmeterol, budesonide and formoterol, or mometasone and formoterol, and a triple treatment, such as fluticasone furoate, vilanterol triphenyl acetate and umeclidinium bromide. In one embodiment, the anti-TSLP antibody or antigen-binding fragment thereof according to the invention can be administered in combination with a fixed dose of inhaled corticosteroids and one or more bronchodilators, such as a fixed dose of fluticasone furoate and vilanterol triphenyl acetate, or fluticasone propionate and salmeterol, or fluticasone furoate and umeclidinium bromide, or fluticasone furoate, vilanterol triphenyl acetate and umeclidinium bromide.

In one embodiment, the anti-TSLP antibody or antigen-binding fragment thereof according to the invention can be administered in combination with an antagonist of a cytokine receptor, such as an antagonist of CCR-1, CCR-3, CCR-4, CCR-5, CCR-6, CCR-7, CCR-8, CCR-9 and CCR10, CXCR1, CXCR2, CXCR3, CXCR4, CXCR5. In one embodiment, the anti-TSLP antibody or antigen-binding fragment thereof according to the invention can be administered in combination with an antibody of other cytokine or cytokine receptor, such as anti-IgE antibody, anti-IL31 antibody, anti-IL31R antibody, anti-IL13 antibody, anti-endoglin antibody, anti-ILlb antibody, another anti-TSLP antibody or anti-hTSLPR antibody, or a combination thereof.

The anti-TSLP antibody or antigen-binding fragment thereof according to the invention can be administered in combination with the following: leukotriene antagonists such as montelukast, zafirlukast and pranlukast; PDE4 inhibitors such as roflumilast; xanthene; anti-IgE antibodies; IL-13 antagonists; IL-6 antagonists and antagonists of IL-1, IL-33, IL-25 or TNF-α.

The anti-TSLP antibody or antigen-binding fragment thereof according to the invention can be administered in combination with antihistamines or antitussive drugs, such as cetirizine hydrochloride, acetaminophen, clemastine fumarate, promethazine, loratadine, desloratadine, diphenhydramine and fexofenadine hydrochloride, activastine, astazole, azelastine, ebastine, epinastine, mizolastine and tefenadine.

The TSLP-binding antibody or antigen-binding fragment thereof according to the invention can be administered in combination with one or more other active agents selected from but not limited to: immunosuppressants (for example, corticosteroids, non-steroidal glucocorticoid receptor agonists, leukotriene D4 antagonists, leukotriene B4 antagonists, A2A agonists, A2B antagonists, dopamine receptor agonists, pirfenidone, nintedanib, or avB6 antagonists), bronchodilators (for example, beta-2 adrenergic receptor agonists, muscarinic antagonists, short-acting β2 receptor agonists, long-acting β 2 receptor agonists, short-acting anticholinergic drugs, methyl xanthine drugs, long-acting anticholinergic drugs), other cytokine or cytokine receptor antagonists or antibodies (for example, IL-13 antagonists, IL-6 antagonists, antagonists of IL-1, IL-33, IL-25 or TNF-alpha, anti-IgE antibodies, anti-IL31 antibodies, anti-IL31R antibodies, anti-IL13 antibodies, anti-endoglin antibodies, anti-ILlb antibodies, another anti-TSLP antibody or anti-hTSLPR antibody), antibiotics, radiotherapy, leukotriene antagonists (for example, montelukast, zafirlukast or pranlukast), PDE4 inhibitors (for example, roflumilast, xanthene), antihistamines or antitussive drugs.

In certain embodiments, the pharmaceutical composition is administered simultaneously with, separately from, or sequentially with other treatments, such as before, simultaneously with, or after an additional pharmaceutically active agent.

In another aspect of the invention, the antibody or antigen-binding fragment thereof, the nucleic acid, the vector, the host cell, the conjugate, or the multispecific antibody of the invention is provided for use in (1) inhibiting or blocking the binding of TSLP to TSLPR/IL7Rα; (2) down-regulating or eliminating the activity of TSLP; (3) down-regulating or blocking OX40L expression; (4) inhibiting or blocking TSLP-induced activation and/or proliferation of mast cells, DC, NKT cells, (5) inhibiting or blocking TSLP-induced osteoprotegerin (OPG) secretion; (6) inhibiting or blocking TSLP-induced secretion of Th2 cytokines, such as TARC, CCL22, IL-4, IL-13 or IL-5; and/or (7) preventing or treating allergic diseases, allergic responsiveness, or autoimmune diseases.

In another aspect of the invention, provided is the use of the antibody or antigen-binding fragment thereof, the nucleic acid, the vector, the host cell, the conjugate, or the multispecific antibody of the invention in the preparation of a medicament for:
(1) inhibiting or blocking the binding of TSLP to TSLPR/IL7Rα;
(2) down-regulating or eliminating the activity of TSLP;
(3) down-regulating or blocking OX40L expression;
(4) inhibiting or blocking TSLP-induced activation and/or proliferation of mast cells, DC, NKT cells;
(5) inhibiting or blocking TSLP-induced osteoprotegerin (OPG) secretion;
(6) inhibiting or blocking TSLP-induced secretion of Th2 cytokines, such as TARC, CCL22, IL-4, IL-13, or IL-5; and/or
(7) preventing or treating allergic diseases, allergic responsiveness, or autoimmune diseases.

In certain embodiments, when used for preparing a medicament, the host cell of the invention comprises the isolated nucleic acid molecule or the vector described above.

In certain embodiments, when the antibody or antigen-binding fragment thereof, the nucleic acid, the vector, the host cell, the multispecific antibody or the conjugate is used for preparing a medicament, the medicament is used for preventing and/or treating asthma, allergic inflammation, allergic reactions, or autoimmune diseases in a subject (e.g., human).

In certain embodiments, the subject is a mammal, including a non-human mammal and human. In certain embodiments, the subject is human.

In certain embodiments, the antibody or antigen-binding fragment thereof, the nucleic acid, the vector, the host cell, the multispecific antibody, the conjugate or the medicament of the invention is used for preventing and/or treating allergic inflammatory diseases, including allergic diseases and non-allergic diseases. These diseases include, but not limited to, asthma (including severe asthma), idiopathic pulmonary fibrosis, atopic dermatitis (AD), allergic conjunctivitis, allergic rhinitis (AR), Netherton syndrome (NS), eosinophilic esophagitis (EOE), food allergy, allergic diarrhea, eosinophilic gastroenteritis, allergic bronchopulmonary aspergillosis (ABPA), allergic fungal sinusitis, rheumatoid arthritis, chronic obstructive pulmonary disease(COPD), systemic sclerosis, keloid, ulcerative colitis, chronic sinusitis (CRS) and nasal polyps, chronic eosinophilic pneumonia, eosinophilic bronchitis; abdominal diseases such as eosinophilic gastroenteritis, Churg-Strauss syndrome; eosinophil-related gastrointestinal diseases, such as eosinophilia/eosinophilic granuloma with polyangiitis, eosinophilic esophagitis and inflammatory bowel disease; urticaria, systemic mastocytosis, cutaneous mastocytosis, and recurrent idiopathic angioedema.

In certain embodiments, the antibody or antigen-binding fragment thereof, the nucleic acid, the vector, the host cell, the multispecific antibody or the conjugate of the invention is used for preventing and/or treating autoimmune related diseases. In certain embodiments, the diseases include, but not limited to, hyperthyroidism, diabetes, myasthenia gravis, ulcerative colitis, gastritis, pemphigus, primary biliary cirrhosis, multiple sclerosis, lupus erythematosus, rheumatoid arthritis, and the like.

In another aspect, the invention provides a method for in vivo or in vitro at least one of (1) inhibiting or blocking the binding of TSLP to TSLPR/IL7Rα; (2) down-regulating or eliminating the activity of TSLP; (3) down-regulating or blocking OX40L expression; (4) inhibiting or blocking TSLP-induced osteoprotegerin (OPG) secretion; (5) inhibiting or blocking TSLP-induced secretion of Th2-like cytokines; (6) inhibiting or blocking TSLP-induced activation and/or proliferation of mast cells, DC, NKT cells, comprising: administering any one of the antibody or antigen-binding fragment thereof, the nucleic acid, the vector, the host cell, the multispecific antibody, the conjugate, or the pharmaceutical composition of the invention to cells or a subject.

Optionally, an additional pharmaceutically activate agent is administered simultaneously with, before or after administration of the antibody or antigen-binding fragment thereof, the nucleic acid, the vector, the host cell, the multispecific antibody, the conjugate or the pharmaceutical composition.

In certain embodiments, the subject is a mammal, including a non-human mammal and human; preferably, the subject is a human.

Thus, in another aspect, the invention provides a method of preventing and/or treating asthma, allergic reactions, allergic inflammation, or autoimmune diseases in a subject, comprising administering an effective amount of the antibody or antigen-binding fragment thereof, the nucleic acid, the vector, the host cell, the multispecific antibody, the conjugate or the pharmaceutical composition of the invention to the subject in need thereof.

The antibody or antigen-binding fragment thereof, the nucleic acid, the vector, the host cell, the multispecific antibody, the conjugate or the pharmaceutical composition of the invention can be formulated into any dosage form known in the medicine field, such as tablets, pills, suspensions, emulsions, solutions, gels, capsules, powders, granules, elixirs, lozenges, suppositories, injections (including injection solutions, sterile powders for injection, and concentrated injection solutions), inhalants, sprays, etc. The preferred dosage form depends on the intended administration route and the treatment use. The pharmaceutical composition of the invention should be sterile and stable under producing and storage conditions, and can be prepared into an injection.

In addition, the antibody or antigen-binding fragment thereof according to the invention may be present in a unit dose form in the pharmaceutical composition to facilitate administration.

The pharmaceutical composition according to the invention may include a "therapeutically effective amount" or "prophylactically effective amount" of the antibody or antigen-binding fragment thereof, the nucleic acid, the vector, the host cell, the multispecific antibody or the conjugate according to the invention. The "prophylactically effective amount" means an amount sufficient to prevent, stop, or delay the occurrence of a disease. The "therapeutically effective amount" means an amount sufficient to cure or at least partially prevent a disease and its complications in a patient suffering from the disease, for example 0.1 mg/mL to 5000 mg/mL.

In the invention, the subject may be a mammal (including a non-human mammal and human), such as a human.

### Detection methods and kits

The antibody or antigen-binding fragment thereof according to the invention is capable of binding to TSLP, thereby being useful in detecting the presence or level of TSLP in a sample.

Thus, in another aspect, the invention provides a kit comprising the antibody or antigen-binding fragment thereof according to the invention. In certain embodiments, the antibody or antigen-binding fragment thereof according to the invention carries a detectable marker. In a preferred embodiment, the kit further comprises a secondary antibody that specifically recognizes the antibody or antigen-binding fragment thereof according to the invention. Preferably, the secondary antibody further comprises a detectable marker.

In the invention, the detectable marker may be any substance detected by fluorescence, spectroscopy, photochemistry, biochemistry, immunology, electricity, optics or chemistry. Especially preferred is that such markers may be suitable for immunoassays (e.g., enzyme-linked immunoassay, radioimmunoassay, fluorescence immunoassay, chemiluminescence immunoassay, etc.). Such markers are well known in the art and include, but not limited to, enzymes (e.g. horseradish peroxidase, alkaline phosphatase, beta-galactosidase, urease, glucose oxidase, etc.), radionuclides (e.g., 3H, '12SI, 35S, 14C or 32P), fluorescent dyes (e.g., Fluorescein isothiocyanate (FITC), fluorescein, tetramethyl rhodamine isothiocyanate (TRITC), phycoerythrin (PE), Texas red, rhodamine, quantum dots or cyanine dye derivatives (e.g., Cy7, Alexa 750)), acridine ester compounds, magnetic beads (e.g., Dynabeads^{®}), calorimetric markers such as colloidal gold or colored glass or plastic (e.g. polystyrene, polypropylene, latex, etc.) beads, and biotin used to bind to avidin (e.g. streptavidin) modified by the above markers. The use of such markers was taught in the patents including, but not limited to, U.S. Pat. 3,817,837; 3,850,752; 3,939,350; 3,996,345; 4,277,437; 4,275,149; and 4,366,241, all of which are incorporated herein by reference. Markers encompassed by the invention can be detected by methods known in the art. For example, a radioactive marker may be detected using a photographic film or a scintillation calculator, and a fluorescent marker may be detected using a photodetector to detect the emitted light. An enzyme marker may be generally detected by providing a substrate for the enzyme and detecting the reaction product produced by the action of the enzyme on the substrate, and a calorimetric marker may be detected by a simple visual coloring marker. In some embodiments, the detectable markers described above may be linked to the recombinant protein of the invention via linkers of different lengths to reduce the potential steric hindrance.

In another aspect, the invention provides a method of detecting the presence or level of TSLP in a sample, comprising the step of employing the antibody or antigen-binding fragment thereof according to the invention.

In a preferred embodiment, the antibody or antigen-binding fragment thereof according to the invention carries a detectable marker.

In another preferred embodiment, the method further comprises detecting the antibody or antigen-binding fragment thereof according to the invention using a reagent with a detectable marker.

The method may be used for diagnostic or non-diagnostic purpose (e.g., TSLP-TSLP/IL-7Ra pathway studies, drug screening, histochemical analysis, etc.). In certain embodiments, the sample for non-diagnostic purpose is a cell sample, such as a cell line or an ex vivo cell culture.

In one embodiment, the invention provides a method of detecting the presence or level of TSLP in a sample, comprising contacting the sample with the antibody or antigen-binding fragment thereof according to the invention under conditions allowing to form a complex between the antibody or antigen-binding fragment thereof and TSLP, and detecting the formation of the complex.

In another aspect, the invention provides a method of diagnosing asthma, allergic inflammation, allergic reactions, or autoimmune diseases in a subject, comprising:
- contacting a sample from the subject with the antibody or antigen-binding fragment thereof, the multispecific antibody or the conjugate according to the invention under conditions allowing to form a complex between the antibody or antigen-binding fragment thereof and TSLP,
- detecting the formation of the complex,
wherein, an increased level of TSLP is indicative of asthma, allergic inflammation, allergic reactions, or autoimmune diseases, as compared with a healthy control.

Optionally, the subject is a mammal, including a non-human mammal and human. Preferably, the subject is a human.

Preferably, the allergic inflammation, allergic reactions, or autoimmune diseases are as described above.

In another aspect, the invention provides the use of the antibody or antigen-binding fragment thereof, the nucleic acid, the vector, the host cell, the multispecific antibody, the conjugate or the pharmaceutical composition according to the invention in the preparation of a medicament or kit for diagnosing asthma, allergic inflammation, allergic reactions, or autoimmune diseases.

In another aspect, provided is the use of the antibody or antigen-binding fragment thereof according to the invention in the preparation of a kit for detecting the presence or level of TSLP in a sample. In another aspect, the invention provides a diagnostic or therapeutic kit comprising one or more of the antibody or antigen-binding fragment thereof, the nucleic acid, the vector, the host cell, the multispecific antibody, the conjugate or the pharmaceutical composition according to the invention. Optionally, the diagnostic or therapeutic kit also includes an instruction.

The antibody or antigen-binding fragment according to the invention has a high affinity for binding to TSLP and good specificity. Therefore, the antibody or antigen-binding fragment according to the invention is suitable for preventing and/or treating asthma, allergic inflammation, allergic reactions, or autoimmune diseases. The fully human antibody of the invention has an extremely high degree of human origin and can be safely administered to a human subject without triggering an immunogenic response. Therefore, the antibody or antigen-binding fragment according to the invention is of great clinical value.

### Definitions of terms

Herein, unless defined otherwise, all scientific and technical terms used herein have the same meaning as those commonly understood by one of ordinary skill in the art to which this invention belongs. In addition, the cell culture, biochemistry, nucleic acid chemistry, immunology laboratory and other operation steps used herein are conventional steps widely used in corresponding fields. Meanwhile, in order to better understand the invention, definitions and explanations of related terms are provided below.

As used in the invention and in the appended claims, the singular forms such as "a/an" and "the" include plural meanings, unless the context clearly indicates otherwise. Therefore, the singular word "a/an" includes the meaning of "one or more".

As used herein, the term "antibody" refers to an immunoglobulin molecule that typically consists of two pairs of polypeptide chains, each having a light chain (LC) and a heavy chain (HC). Light chains are classified as either kappa (κ) or lambda (λ) light chains. Heavy chains can be classified as µ, δ, γ, α, or ε, and the isotypes of antibody can be defined as IgM, IgD, IgG, IgA and IgE, respectively. Within light and heavy chains, variable and constant regions are joined by a "J" segment of about 12 or more amino acids, the heavy chain also comprising a "D" segment of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain CL. The constant domain is not directly involved in the binding of an antibody to an antigen, but exhibits various effector functions, such as mediating the binding of an immunoglobulin to a host tissue or factor, including various cells of the immune system (e.g., effector cells) and a first component (C1q) of the classical complement system. The VH and VL regions can also be subdivided into hypervariable regions (called complementary determining region (CDR)) interspersed with relatively conservative regions called framework regions (FR). Each VH and VL consists of three CDRs and four FRs arranged from amino terminal to carboxyl terminal in the following order: FR1, CDR1, FR2, CDR2, FR3, CDR3, FR4. The variable regions (VH and VL) of each heavy chain/light chain pair form antigen-binding sites, respectively.

Herein, the CDRs contained in the antibody or antigen-binding fragment thereof according to the invention may be determined according to various numbering systems known in the art. In certain embodiments, the CDRs contained in the antibody or antigen-binding fragment thereof according to the invention are preferably determined by a Kabat, Chothia, AbM or IMGT numbering system.

As used herein, the term "framework region" or "FR" residues refer to those amino acid residues in the variable region of an antibody other than the CDR residues as defined above.

As used herein, the term "germline antibody gene" is a gene encoding an immunoglobulin expressed by a non-lymphocyte that does not undergo maturation processes of genetic rearrangement and maturation leading to the expression of a specific immunoglobulin. An advantage provided by various embodiments of the invention is derived from a consensus that amino acid sequences encoded by germline antibody genes retain more important amino acid sequence structures showing characteristics of individual animal species than those encoded by mature antibody genes. Therefore, when applied therapeutically to this species, it is less recognized as an exogenous substance by this species.

The term "antibody" is not limited by any particular method for producing antibodies. For example, it includes recombinant antibodies, monoclonal antibodies, and polyclonal antibodies. The antibody may be an antibody of different isotypes, such as an IgG (e.g., an IgG1, IgG2, IgG3, or IgG4 subtype), IgA1, IgA2, IgD, IgE, or IgM antibody.

As used herein, the term "antigen-binding fragment" of an antibody refers to a polypeptide fragment of an antibody, such as a polypeptide fragment of a full-length antibody, which retains the ability to specifically bind to the same antigen as bound by the full-length antibody and/or competes with the full-length antibody for specific binding to the antigen, which is also known as an "antigen-binding part". In general, see Fundamental Immunology, Ch. 7 (Paul, W., ed., 2nd Edition, Raven Press, N.Y. (1989), which is incorporated herein by reference in its entirety for all purposes. Antigen-binding fragments of an antibody can be produced by recombinant DNA technologies or by enzymatic or chemical cleavage of an intact antibody. Non-limiting examples of antigen-binding fragments include Fab, Fab', F (ab')₂, Fd, Fv, dAb and complementary determining region (CDR) fragments, single chain antibody (e.g. scFv), chimeric antibody, diabody, linear antibody, nanobody (e.g. by technology from Ablynx), domain antibody (e.g. by technology from Domantis), and a polypeptide comprising at least a portion of an antibody sufficient to confer the specific antigen-binding ability to the polypeptide. Engineering modified antibody variants are reviewed in Holliger et al., 2005; Nat Biotechnol, 23: 1126-1136.

As used herein, the term "full-length antibody" means an antibody consisting of two "full-length heavy chains" and two "full-length light chains". Wherein, "full-length heavy chain" refers to a polypeptide chain which, from N-terminal to C-terminal, consists of a heavy chain variable region (VH), a heavy chain constant region CH1 domain, a hinge region (HR), a heavy chain constant region CH2 domain and a heavy chain constant region CH3 domain; and, when the full-length antibody is an IgE isotype, it optionally further includes a heavy chain constant region CH4 domain. Preferably, a "full-length heavy chain" is a polypeptide chain consisting of VH, CH1, HR, CH2, and CH3 from N-terminal to C-terminal. A "full-length light chain" is a polypeptide chain consisting of a light chain variable region (VL) and a light chain constant region (CL) from N-terminal to C-terminal. Two pairs of full-length antibody chains are linked by a disulfide bridge between CL and CH1 and a disulfide bridge between HRs of two full-length heavy chains. The full-length antibody of the invention may be derived from a single species, such as a human; and can also be a chimeric antibody or a humanized antibody. The full-length antibody of the invention comprises two antigen-binding sites formed by VH and VL pairs, respectively, which sites specifically recognize/bind the same antigen.

As used herein, the term "Fd fragment" means an antibody fragment consisting of VH and CH1 domains; the term "dAb fragment" means an antibody fragment consisting of a VH domain (Ward et al., Nature 341: 544 546 (1989)); the term "Fab fragment" means an antibody fragment consisting of VL, VH, CL and CH1 domains; the term "F(ab')₂ fragment" means an antibody fragment comprising two Fab fragments linked by a disulfide bridge on the hinge region; the term "Fab' fragment" means a fragment obtained by reducing the disulfide bridge linking two heavy chain fragments in the F(ab')₂ fragment, which consists of a complete light chain and a Fd fragment (composed of VH and CH1 domains) of heavy chain.

As used herein, the term "Fv fragment" means an antibody fragment consisting of VL and VH domains of a single-arm of antibody. The Fv fragment is generally considered to be the smallest antibody fragment that can form a complete antigen-binding site. It is generally thought that six CDRs confer antigen-binding specificity to an antibody. However, even one variable region (such as a Fd fragment, which contains only three antigen-specific CDRs) can recognize and bind antigen, although possibly having a lower affinity than a complete binding site.

As used herein, the term "Fc fragment" means an antibody fragment formed by linking the second and third constant regions of the first heavy chain of an antibody to the second and third constant regions of the second heavy chain of the antibody via a disulfide bridge. Fc fragment of an antibody has many different functions, but does not involve in antigen binding.

As used herein, the term "scFv" refers to a single polypeptide chain comprising VL and VH domains, wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Ed. Roseburg and Moore, Springer-Verlag, New York, pp. 269-315 (1994)). Such scFv molecules may have a general structure: NH₂-VL-Linker-VH-COOH or NH₂-VH-Linker-VL-COOH. A suitable linker in the art consists of repeated GGGGS amino acid sequences or variants thereof. For example, a linker of amino acid sequence (GGGGS)₄ or a variant thereof can be used (Holliger et al. (1993), Proc. Natl. Acad. Sci. USA 90: 6444-6448). Other linkers that may be used in the invention are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56 and Roovers et al. (2001), Cancer Immunol. In some cases, a disulfide bridge may also be present between VH and VL of scFv. As used herein, the term "di-scFv" refers to an antibody fragment formed by linking two scFvs.

As used herein, the term "diabody" means that the VH and VL domains are expressed on a single polypeptide chain but the used linker is too short to allow for pairing between two domains of the same chain, thus forcing the domain to pair with a complementary domain of the other chain and producing two antigen-binding sites (see, for example, Holliger P. et al., Proc. Natl. Acad. Sci. USA 90: 6444-6448 (1993), and Poljak R.J. et al., Structure 2:1121-1123 (1994)).

As used herein, "antibody mimetic" refers to specifically binds to an antigen as an antibody, but without antibody structure. They are usually artificial peptides or proteins having a molar mass of about 3 to 20 kDa. Examples are designed ankyrin repeat protein (DARPin) and fynomer. The designed ankyrin repeat protein (DARPin) can be linked to an IgG antibody, a scFv-Fc antibody fragment or combination thereof, as described in CN104341529A. An anti-IL-17a fynomer binds to an anti-IL-6R antibody, as described in WO2015141862A1.

Each of the above antibody fragments retains the ability to specifically bind to the same antigen as bound by the full-length antibody, and/or competes with the full-length antibody for specific binding to an antigen.

An antigen-binding fragment (e.g., the antibody fragment described above) may be obtained from a given antibody (e.g., the antibody provided by the invention) using conventional technologies known to those skilled in the art (e.g., recombinant DNA technologies or enzymatic or chemical cleavage methods), and the antigen-binding fragment of the antibody is specifically screened in the same manner as for an intact antibody.

Herein, unless clearly indicated otherwise in the context, reference to the term "antibody" includes not only an intact antibody but also an antigen-binding fragment thereof.

As used herein, the terms "monoclonal antibody", "McAb" and "mAb" have the same meaning and are used interchangeably, which refers to an antibody from a population of highly homologous antibody molecules, that is, a population of identical antibody molecules except for natural mutations that may occur spontaneously. A monoclonal antibody has a high specificity for a single epitope on an antigen. Compared to a monoclonal antibody, polyclonal antibodies usually contain at least two or more different antibodies, which usually recognize different epitopes on an antigen. Furthermore, the modifier "monoclonal" simply indicates that the features of an antibody are obtained from a population of highly homologous antibodies and cannot be understood as requiring any particular method to prepare the antibody.

As used herein, the term "chimeric antibody" means such an antibody where one part of its light chain or/and heavy chain is derived from an antibody (which can be derived from a specific species or belong to a specific antibody class or subclass), and the other part of the light chain or/and heavy chain is derived from another antibody (which may be derived from the same or different species or belong to the same or different antibody class or subclass), which in any case retains binding activity to a target antigen (U.S. Patent 4,816, 567 to Cabilly et al.; Morrison et al., Proc. Natl. Acad. Sci. USA, 81: 6851 6855 (1984)).

In the invention, the expected properties of the inventive antibody include: (1) inhibiting or blocking the binding of TSLP to TSLPR/IL7Rα; (2) down-regulating or eliminating the activity of TSLP; (3) down-regulating or blocking OX40L expression; (4) inhibiting or blocking secretion of Th2-like cytokines; (5) preventing and/or treating allergic inflammatory diseases. The humanized antibody of the invention retains one or more of the above-mentioned expected properties of the parent antibody (a human antibody or mouse-human chimeric antibody).

For preparing a humanized antibody, a mouse CDR region can be inserted into a human framework sequence using methods known in the art (see U.S. Pat. No. 5,225,539 to Winter; U.S. Pat. Nos. 5,530,101; 5,585,089; 5,693,762 and 6,180, 370 to Queen et al.; and Lo, Benny, K.C., editor, in Antibody Engineering: Methods and Protocols, volume 248, Humana Press, New Jersey, 2004). Alternatively, transgenic animals can also be utilized, which are incapable of producing endogenous immunoglobulins after immunization but capable of producing an intact human antibody library (see, for example, Jakobovits et al., 1993, Proc. Natl. Acad. Sci. USA 90: 2551; Jakobovits et al., 1993, Nature 362: 255-258; Bruggermann et al., 1993, Year in Immunology 7:33; and Duchosal et al., 1992, Nature 355: 258; Lonberg et al. (1994) Nature 368 (6474): 856-859; WO02/43478). Other methods of antibody humanization include phage display technology (Hoogenboom et al., 1991, J.Mol. Biol. 227: 381; Marks et al., J.Mol. Biol. 1991, 222: 581-597; Vaughan et al., 1996, Nature Biotech 14: 309).

As used herein, the term "humanization degree" is an indicator for evaluating the number of non-human amino acid residues in a humanized antibody. The humanization degree of humanized antibodies can be predicted, for example, by DomainGapAlign from the IMGT website for the homology of variable region sequences with human V domains.

As used herein, the term "specifically binding" refers to non-random binding reaction between two molecules, such as reaction between an antibody and an antigen against which it is. The strength or affinity of the specific binding interaction can be indicated by the equilibrium dissociation constant (K_{D}) of the interaction. Herein, the term "K_{D}" refers to the dissociation equilibrium constant of specific antibody-antigen interaction, which is used to describe the binding affinity between an antibody and an antigen. The smaller the dissociation equilibrium constant, the tighter the antibody-antigen binding, and the higher the affinity between the antibody and the antigen. In certain embodiments, an antibody specifically binding to an antigen (or an antibody specific for an antigen) means that the antibody binds to the antigen with a K_{D} of less than about 10⁻⁸ M, for example less than about 10⁻⁸ M, 10⁻⁹ M, 10⁻¹⁰ M or 10⁻¹¹ M or less. In some embodiments, when K_{D}≤ 10 × 10⁻⁸ M, the antibody or antigen-binding fragment thereof of the invention is considered as specifically binding to TSLP.

The specific binding properties between two molecules can be measured using methods well known in the art. One of the methods involves measuring the rate of formation and dissociation of antigen-binding site/antigen complexes. Both "binding rate constant" (ka or kon) and "dissociation rate constant" (kdis or koff) can be calculated from the concentration and the actual rates of association and dissociation (see Malmqvist M, Nature, 1993, 361: 186-187). The ratio of kdis/kon is equal to the dissociation constant K_{D} (see Davies et al., Annual Rev Biochem, 1990; 59: 439-473). K_{D}, kon and kdis values can be measured by any effective method. In certain embodiments, the dissociation constant can be measured by bioluminescence interferometry (e.g., ForteBio Octet assay). In addition, the dissociation constant can also be measured by surface plasmon resonance technology (such as Biacore) or Kinexa.

As used herein, the term "vector" refers to a nucleic acid vehicle into which a polynucleotide can be inserted. When enabling the expression of a protein encoded by the inserted polynucleotide, the vector is called as expression vector. Vectors can be introduced into host cells through transformation, transduction, or transfection, so that the carried genetic material elements can be expressed in host cells. Vectors are well known to those skilled in the art, including but not limited to: plasmids; phagmids; cosmids; artificial chromosomes, such as yeast artificial chromosomes (YAC), bacterial artificial chromosomes (BAC), or P1-derived artificial chromosomes (PAC); phages, such as λ phage or M13 phage, and animal viruses. Animal viruses that can be used as vectors include, but not limited to, retroviruses (including lentivirus), adenoviruses, adeno-associated viruses, herpesviruses (e.g., herpes simplex virus), poxviruses, baculoviruses, papillomaviruses, papovaviruses (e.g., SV40). A vector may contain elements controlling expression, including but not limited to a promoter sequence, a transcription initiation sequence, an enhancer sequence, a selection element, and a reporter gene. In addition, the vector may also contain an origin of replication.

As used herein, the term "host cell" refers to a cell into which a vector can be introduced, including, but not limited to, prokaryotic cells such as Escherichia coli or Bacillus subtilis, fungal cells such as yeast cells or Aspergillus, insect cells such as 52 drosophila cells or Sf9, or animal cells such as fibroblasts, CHO cells, COS cells, NSO cells, HeLa cells, BHK cells, HEK 293 cells or human cells.

As used herein, the term "identity" is used to refer to the sequence matching degree between two polypeptides or two nucleic acids. When a position in two sequences for comparison is occupied by the same base or amino acid monomer subunit (for example, a position in each of two DNA molecules is occupied by adenine, or a position in each of two polypeptides is occupied by lysine), then the molecules are identical at that position. "Percentage identity" between two sequences is a function of the number of matching positions shared by the two sequences divided by the number of positions to be compared and ×100. For example, if six positions of the ten positions in two sequences match, then the two sequences are 60% identical. For example, the DNA sequences CTGACT and CAGGTT have 50% identity (3 positions of the total 6 positions match). Typically, the two sequences are compared and aligned for maximum identity. Such an alignment can be realized by using, for example, the method of Needleman et al. (1970) J. Mol. Biol. 48: 443-453, which can be conveniently performed by a computer program such as Align program (DNAstar, Inc). Additionally, the percentage identity between two amino acid sequences can be determined by the algorithm of Needleman and Wunsch (J Mol Biol. 48: 444-453 (1970)) in the GAP program incorporated in the GCG software package (available in www.gcg.com), using either a Blossum Matrix 62 or PAM250, and a gap weight of 16, 14, 12, 10, 8, 6 or 4 and a length weight of 1, 2, 3, 4, 5 or 6. The % identity between two amino acid sequences can also be determined by the algorithm of E. Meyers and W. Miller (Computer. App. Biosci., 4: 11-17 (1988)).

As used herein, a sequence having "% identity" retains important biological activities, such as antibody-binding specificity, of the sequence which it is aligned with or derived from. A sequence having a substitution, deletion or addition of one or several amino acids or any combination thereof retains important biological activities, such as antibody-binding specificity, of the sequence which it is aligned with or derived from. A nucleotide sequence having "% identity" or having a difference of not more than 3, 6, 15, 30 or 45 nucleotides can exhibit the functions similar to the nucleotide sequence which it is aligned with or derived from, for example, all the expressed proteins can specifically bind to the same antigen or molecule.

As used herein, the term "conservative substitution" means an amino acid substitution that does not adversely affect or alter the expected properties of a protein/polypeptide comprising an amino acid sequence. For example, a conservative substitution may be introduced by standard technologies known in the art, such as site-directed mutagenesis and PCR-mediated mutagenesis. A conservative amino acid substitution includes a substitution of an amino acid residue with an amino acid residue having a similar side chain, e.g., with a residue that is physically or functionally similar to the corresponding amino acid residue (e.g., having similar size, shape, charge, chemical property, including the ability to form covalent or hydrogen bonds, etc.). Families of amino acid residues having similar side chains have been defined in the art. These families include amino acids having alkaline side chains (e.g., lysine, arginine and histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta branched side chains (e.g., threonine, valine, isoleucine) and aromatic side chains (e.g. tyrosine, phenylalanine, tryptophan, histidine). Therefore, it is preferred to replace a corresponding amino acid residue with another amino acid residue from the same side chain family. Methods for identifying conservative substitution of amino acids are well known in the art (see, for example, Brummell et al., Biochem. 32: 1180-1187 (1993); Kobayashi et al., Protein Eng. 12 (10): 879-884 (1999); and Burks et al., Proc. Natl Acad. Set USA 94: 412-417 (1997), which are incorporated herein by reference).

The compilation of twenty conventional amino acids involved herein follows the conventional usage. See, for example, Immunology-A Synthesis (2nd Edition, E. S. Golub and D. R. Gren, Eds., Sinauer Associates, Sunderland, Mass. (1991)), which is incorporated herein by reference. Herein, the terms "polypeptide" and "protein" have the same meaning and are used interchangeably. Also, in the invention, amino acids are generally expressed by single-letter and three-letter abbreviations well known in the art. For example, alanine can be expressed as A or Ala; arginine as R or Arg; glycine as G or Gly; glutamine as Q or Gln.

As used herein, the term "pharmaceutically acceptable carrier and/or excipient" means a carrier and/or excipient that is pharmacologically and/or physiologically compatible with the subject and active ingredient, which are well known in the art (see, for example, Remington's Pharmaceutical Sciences. Edited by Gennaro AR, 19th ed. Pennsylvania: Mack Publishing Company, 1995) and include, but not limited to, pH modulators, surfactants, adjuvants, ionic strength enhancers, diluents, osmotic pressure maintaining reagents, delayed absorption reagents, preservatives. For example, the pH modulators include, but not limited to, phosphate buffers. The surfactants include, but not limited to cationic, anionic, or nonionic surfactants, such as Tween-80. The ionic strength enhancers include, but not limited to, sodium chloride. The preservatives include, but not limited to, various antibacterial and antifungal agents, such as p-hydroxybenzoate, trichloro-tert-butyl alcohol, phenol, sorbic acid, etc. The osmotic pressure maintaining reagents include, but not limited to, sugars, NaCl and the like. The delayed absorption reagents include, but not limited to, monostearates and gelatin. Diluents include, but not limited to, water, aqueous buffers (e.g., buffer saline), alcohols and polyols (e.g., glycerol), etc. The preservatives include, but not limited to, various antibacterial and antifungal agents, such as thiomersalate, 2-phenoxyethanol, p-hydroxybenzoate, trichloro-tert-butyl alcohol, phenol, sorbic acid, etc. The stabilizers have a meaning commonly understood by those skilled in the art, which can stabilize the desired activity of the active ingredient in drug, including but limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate).

As used herein, the term "prevention" refers to a process for preventing or delaying the onset of a disease or condition or symptom (e.g., asthma, allergic inflammation, allergic reactions, or autoimmune diseases) in vivo in a subject. As used herein, the term "treatment" refers to a process for achieving a beneficial or desired clinical result. For purposes of the invention, beneficial or desired clinical result includes, but not limited to, alleviation of a symptom, diminishment of extent of disease, stabilizing (i.e., not worsening) state of a disease, delay or slowing of disease progression, amelioration or palliation of disease state, and remission (whether partial or all) of symptoms, no matter detectable or undetectable. In addition, "treatment" can also mean prolonging survival as compared to the expected survival (if without treatment).

As used herein, the term "subject" refers to a mammal, such as a primate mammal, such as a non-human primate mammal or human. In some embodiments, the subject (e.g., human) has asthma, allergic inflammation, allergic reaction, or autoimmune disease, or is at a risk of suffering from such disease.

As used herein, the term "effective amount" refers to an amount sufficient to achieve or at least partially achieve a desired effect. For example, an effective amount for preventing a disease (e.g., asthma, allergic inflammation, allergic reactions, or autoimmune diseases) is an amount sufficient to prevent, stop, or delay the onset of the disease (e.g., asthma, allergic inflammation, allergic reactions, or autoimmune diseases); a therapeutic effective amount refers to an amount sufficient to cure or at least partially prevent a disease and its complications of a patient who have already suffered from the disease. To determine such effective amount is within the scope of capability of those skilled in the art. For example, the amount effective for a therapeutic use will depend on the severity of the disease to be treated, the general state of the patient's own immune system, the general condition of the patient such as age, weight and gender, route of administration, and other treatments administered concurrently, etc.

As used herein, the term "immune cell" includes cells that have hematopoietic origins and play a role in an immune response, for example, lymphocytes, such as B cells and T cells; natural killer cells; myeloid cells, such as monocytes, macrophages, eosinophils, mast cells, basophils, and granulocytes.

As used herein, the term "immune response" means the action of immune cells (such as lymphocytes, antigen presenting cells, phagocytes or granulocytes) and soluble macromolecules (including antibodies, cytokines and complements) produced by immune cells or liver, which leads to selective damage, destruction or removal from human body of invasive pathogens, pathogen-infected cells or tissues, cancer cells, or normal human cells or tissues in the case of an autoimmune or pathological inflammation. Herein, the term "antigen-specific T cell response" refers to an immune response produced by T cells when the T cells are stimulated by an antigen specific to the T cells. Non-limiting examples of responses produced by T cells in response to antigen-specific stimulation include proliferation of T cells and production of cytokines (e.g., IL-2).

As used herein, the term "effector function" refers to those biological activities which contribute to the Fc region of an antibody (Fc region of a natural sequence or Fc region of an amino acid sequence variant) and vary with antibody isotypes.

The term "pharmaceutically acceptable" means, when a molecule entity, a fragment of a molecule or a composition is properly administered to an animal or human, they will not produce unfavorable, allergic, or other adverse reactions. Specific examples of materials that may be used as pharmaceutically acceptable carrier or component thereof include sugars (e.g., lactose), starch, cellulose and derivatives thereof, vegetable oils, gelatin, polyols (e.g., propylene glycol), alginic acid, and the like.

### Beneficial effects of the invention

Compared with the prior art, the technical solutions of the invention have the following beneficial effects:
(1) The antibody of the invention can specifically recognize/bind TSLP with high affinity, inhibit or block the binding of TSLP to TSLPR/IL7RR, inhibit or block TSLP-induced proliferation of Ba/F3 cells in vitro/in vivo, and block TSLP-induces activation and cytokine secretion of PBMC. Thus, the antibody of the invention is capable of inhibiting or blocking TSLP-induced activation and/or proliferation of mast cells, DC, NKT cells, inhibiting or blocking TSLP-induced OX40L expression, osteoprotegerin (OPG) secretion, or TSLP-induced secretion of Th2 cytokines such as TARC, CCL22, IL-4, IL-13 or IL-5. Therefore, the antibody of the invention has potential in preventing and/or treating asthma, other allergic reactions, or autoimmune diseases.
(2) The antibody of the invention has good thermal stability, hydrophilicity, isoelectric point, and affinity for FcRn.
(3) Some of the antibodies of the invention are fully human antibodies, and thus can be safely administered to a subject without triggering an immunogenic response. Therefore, the antibody of the invention is of great clinical value.

### Abbreviations

- CDR: Complementary determining region in the variable region of an immunoglobulin
- FR: Antibody framework region: amino acid residues in the variable region of an antibody except for CDR residues
- VH: Heavy chain variable region of an antibody
- VL: Light chain variable region of an antibody
- IgG: Immunoglobulin G
- AbM: The definitions of AbM CDR comes from Martin's study (Martin ACR, Cheetham JC, Rees AR (1989) Modelling antibody hypervariable loops: A combined algorithm. Proc Natl Acad Sci USA 86: 9268-9272), which have integrated parts of definitions of Kabat and Chothia.
- Kabat: Immunoglobulin alignment and numbering system proposed by Elvin A. Kabat (see, for example, Kabat et al., Sequences of Proteins of Immunological Interest, 5th Ed. Public Health Service, National Institutions of Health, Bethesda, Md., 1991).
- Chothia: Immunoglobulin numbering system proposed by Chothia et al., which is a classical rule for identifying CDR region boundaries based on the location of structural ring regions (see, for example, Chothia & Lesk (1987) J. Mol. Biol. 196: 901-917; Chothia et al. (1989) Nature 342: 878-883).
- IMGT: A numbering system based on the international ImmunoGenetics information system^{®} (IMGT) initiated by Lefranc et al., see Lefranc et al., Dev. Comparat. Immunol. 27:55-77, 2003.
- mAb: Monoclonal antibody
- EC₅₀: A concentration that produces 50% efficacy or binding
- IC₅₀: A concentration that produces 50% inhibition
- ELISA: Enzyme-linked immunosorbent assay
- PCR: Polymerase chain reaction
- HRP: Horseradish peroxidase
- TSLP: Thymic stromal lymphopoietin
- TSLPR: Thymic stromal lymphopoietin receptor
- IL7Rα: Interleukin 7 receptor alpha subunit
- TARC: Thymus and activation-regulated chemokine
- hFc: Fc segment of human IgG antibody
- KD: Dissociation equilibrium constant
- CDR-H1: Complementary determining region 1 in heavy variable region of immunoglobulin
- CDR-H2: Complementary determining region 2 in heavy variable region of immunoglobulin
- CDR-H3: Complementary determining region 3 in heavy variable region of immunoglobulin
- CDR-L1: Complementary determining region 1 in light variable region of immunoglobulin
- CDR-L2: Complementary determining region 2 in light variable region of immunoglobulin
- CDR-L3: Complementary determining region 3 in light variable region of immunoglobulin

### Brief Description of the Drawings

Figure 1: Detection of Ba/F3 cell line overexpressing both human TSLPR/IL7Rα genes.
Figure2A: Detection of inhibitory activity of chimeric antibody 25A5C5 on the proliferation of Ba/F3-hTSLPR-hIL7Rα cells.
Figure 2B: Detection of inhibitory activity of chimeric antibodies 27C2B6 and 37C2D10 on the proliferation of Ba/F3-hTSLPR-hIL7Rα cells.
Figure 2C: Detection of inhibitory activity of chimeric antibodies 43B1A8 and 90H3H11 on the proliferation of Ba/F3-hTSLPR-hIL7Rα cells.
Figure 3A: Detection of binding affinity of recombinant fully human antibodies 25A5C5-hIgG, 27C2B6-hlgG and 37C2D10-hIgG to human TSLP protein.
Figure 3B: Detection of binding affinity of recombinant fully human antibodies 43B1A8-hIgG and 90H3H11-hIgG to human TSLP protein.
Figure 4A: Detection of inhibitory activity of recombinant fully human antibodies 25A5C5-hIgG, 27C2B6-hIgG and 37C2D10-hIgG on the proliferation of Ba/F3-hTSLPR-hIL7Rα cells.
Figure 4B: Detection of inhibitory activity of recombinant fully human antibody 43B1A8-hlgG on the proliferation of Ba/F3-hTSLPR-hIL7Rα cells.
Figure 4C: Detection of inhibitory activity of recombinant fully human antibody 90H3H11-hIgG on the proliferation of Ba/F3-hTSLPR-hIL7Rα cells.
Figure 5: Detection of inhibitory activity of recombinant fully human antibodies 43B1A8-hlgG and 90H3H11-hIgG on secretion of TARC by PBMC.
Figure 6: Detection of thermal stability (Tm) of recombinant fully human antibodies 43B1A8-hIgG and 90H3H11-hIgG.
Figure 7A: Detection of inhibitory activity of recombinant fully human antibody 43B1-H2L2 on the proliferation of Ba/F3-hTSLPR-hIL7Rα cells.
Figure 7B: Detection of inhibitory activity of recombinant fully human antibody 43B1-H6L1 on the proliferation of Ba/F3-hTSLPR-hIL7Rα cells.
Figure 8: Detection of inhibitory activity of recombinant fully human antibody 43B1-H2L2 on secretion of MDC cytokines by PBMC cells.
Figure 9: Pharmacokinetic analysis of recombinant fully human antibody 43B1-H2L2 molecule in cynomolgus monkeys.

### Sequence information

The sequence information related to the invention is described in the following table.

| **SEQ ID NO.** | **Description** | **SEQ ID NO.** | **Description** |
|---|---|---|---|
| **1** | **Heavy chain variable region of antibody 25A5C5** | **39** | **AbM 37C2D10 CDR-L1** |
| **2** | **Light chain variable region of antibody 25A5C5** | **40** | **Heavy chain variable region of antibody 43B1A8** |
| **3** | **IMGT 25A5C5 CDR-H1** | **41** | **Light chain variable region of antibody 43B1A8** |
| **4** | **IMGT 25A5C5 CDR-H2** | **42** | **IMGT 43B1A8 CDR-H1** |
| **5** | **IMGT 25A5C5 CDR-H3** | **43** | **IMGT 43B1A8 CDR-H2** |
| **6** | **IMGT 25A5C5 CDR-L1** | **44** | **IMGT 43B1A8 CDR-H3** |
| **7** | **IMGT 25A5C5 CDR-L2** | **45** | **IMGT 43B1A8 CDR-L1** |
| **8** | **IMGT/ AbM 25A5C5 CDR-L3** | **46** | **IMGT 43B1A8 CDR-L2** |
| **9** | **AbM 25A5C5 CDR-H1** | **47** | **IMGT/ AbM 43B1A8 CDR-L3** |
| **10** | **AbM 25A5C5 CDR-H2** | **48** | **AbM 43B1A8 CDR-H1** |
| **11** | **AbM 25A5C5 CDR-H3** | **49** | **AbM 43B1A8 CDR-H2** |
| **12** | **AbM 25A5C5 CDR-L1** | **50** | **AbM 43B1A8 CDR-H3** |
| **13** | **AbM 25A5C5 CDR-L2** | **51** | **AbM 43B1A8 CDR-L1** |
| **14** | **Modified heavy chain constant region of human IgG1** | **52** | **AbM 43B1A8 CDR-L2** |
| **15** | **Modified heavy chain constant region of human IgG1** | **53** | **Heavy chain variable region of antibody 90H3H11** |
| **16** | **Human κ light chain constant region** | **54** | **Light chain variable region of antibody 90H3H11** |
| **17** | **Heavy chain variable region of antibody 27C2B6** | **55** | **IMGT 90H3H11 CDR-H1** |
| **18** | **Light chain variable region of antibody 27C2B6** | **56** | **IMGT 90H3H11 CDR-H2** |
| **19** | **IMGT 27C2B6 CDR-H1** | **57** | **IMGT 90H3H11 CDR-H3** |
| **20** | **IMGT 27C2B6 CDR-H2** | **58** | **IMGT 90H3H11 CDR-L1** |
| **21** | **IMGT 27C2B6 CDR-H3** | **59** | **IMGT 90H3H11 CDR-L2** |
| **22** | **IMGT 27C2B6 CDR-L1** | **60** | **IMGT/ AbM 90H3H11 CDR-L3** |
| **23** | **IMGT 27C2B6 CDR-L2/ 37C2D10 CDR-L2** | **61** | **AbM 90H3H11 CDR-H1** |
| **24** | **IMGT/AbM 27C2B6 CDR-L3/ IMGT/AbM 37C2D10 CDR-L3** | **62** | **AbM 90H3H11 CDR-H2** |
| **25** | **AbM 27C2B6 CDR-H1** | **63** | **AbM 90H3H11 CDR-H3** |
| **26** | **AbM 27C2B6 CDR-H2** | **64** | **AbM 90H3H11 CDR-L1** |
| **27** | **AbM 27C2B6 CDR-H3** | **65** | **AbM 90H3H11 CDR-L2** |
| **28** | **AbM 27C2B6 CDR-L1** | **66** | **43B1A8-hlgG heavy chain sequence** |
| **29** | **AbM 27C2B6 CDR-L2/ 37C2D10 CDR-L2** | **67** | **43B1A8-hIgG/43B1-H6L1 light chain sequence** |
| **30** | **Heavy chain variable region of antibody 37C2D10** | **68** | **Heavy chain variable region of antibody 43B1-H2L2** |
| **31** | **Light chain variable region of antibody 37C2D10** | **69** | **Light chain variable region of antibody 43B1-H2L2** |
| **32** | **IMGT 37C2D10 CDR-H1** | **70** | **Heavy chain constant region of modified human IgG4** |
| **33** | **IMGT 37C2D10 CDR-H2** | **71** | **Heavy chain amino acid sequence of antibody 43B1-H2L2** |
| **34** | **IMGT 37C2D10 CDR-H3** | **72** | **Light chain amino acid sequence of antibody 43B1-H2L2** |
| **35** | **IMGT 37C2D10 CDR-L1** | **73** | **Heavy chain amino acid sequence of antibody 43B1-H6L1** |
| **36** | **AbM 37C2D10 CDR-H1** | **74** | **Human IgG1 constant region** |
| **37** | **AbM 37C2D10 CDR-H2** | **75** | **Human IgG4 heavy chain constant region** |
| **38** | **AbM 37C2D10 CDR-H3** | | |

### Detailed Description of the Invention

The invention is now illustrated with reference to the following examples which are intended to exemplify but not limit the invention.

Unless indicated otherwise, the processes of molecular biology experiments and immunoassays used in the invention are essentially carried out with reference to the processes described in J. Sambrook et al., Molecular Cloning: A Laboratory Manual, 2nd ed., Cold Spring Harbor Press, 1989; and F. M. Ausubel et al., Short Protocols in Molecular Biology, 3rd ed., John Wiley & Sons, Inc., 1995. Skilled persons in the art will appreciate that the examples are intended to exemplify the invention and are not intended to limit the scope of the invention.

### Example 1: preparation of antigens

Human TSLP or monkey TSLP was expressed in Escherichia coli or mammalian cells. The amino acid sequence of human TSLP refers to NP_149024.1 in the protein database of NCBI. The amino acid sequence of cynomolgus monkey (Macaca fascicularis) TSLP refers to XP_005557555.1 in the protein database of NCBI. Antigens used in the present application include TSLP expressed in a modified form, such as a fusion of 6 consecutive histidines as a label fused to the C-terminal of the TSLP sequence (TSLP-His). The above-mentioned human and monkey TSLP sequences were codon optimized by GenScript(Nanjing) Co., Ltd, synthesized in an expression vector (i.e., plasmids containing the complete coding sequence of human TSLP), expressed in Escherichia coli or mammalian cells HEK293F and purified.

The natural receptor of TSLP is a heterodimer, which is composed of human TSLPR and human IL7R alpha (IL7Rα) subunit. The sequence of human TSLPR refers to Uniprot: Q9HC73.1; the sequence of human IL7Rα subunit refers to GenBank: AAR08908.1. Human hIL7Rα-hTSLPR-hFc fusion protein was composed of the extracellular domain of human IL7Rα (E21-D239), the extracellular domain of human TSLPR receptor (Q23-K231) and part of the coding sequence of human IgG1 Fc region (Hinge-CH2-CH3) in tandem. It was constructed into the expression vector pLVX after codon optimization. The stable expressing cell line of HEK293F was established by using the pLVX vector and finally the fusion protein of hIL7Rα-hTSLPR-hFc was obtained by purification.

### Example 2: Construction and identification of the cell line overexpressing both human TSLPR/IL7Rα genes

### 2.1: Construction of a cell line overexpressing both human TSLPR/IL7Rα genes

In order to verify the efficacy of human TSLP antibodies in blocking the binding of human TSLP to human TSLPR/IL7Rα receptor, the complete amino acid coding sequence of human TSLPR (Gene ID: UniProtKB/Swiss-Prot: Q9HC73.1, synthesized by GenScript(Nanjing) Co., Ltd) and the complete amino acid coding sequence of human IL7R alpha subunit (Gene ID: GenBank: AAR08908.1, synthesized by GenScript(Nanjing) Co., Ltd) were codon optimized by GenScript(Nanjing) Co., Ltd and cloned into lentiviral vectors pLVX-IRES-puro and pLVX-IRES-zeocin, respectively, and mouse pro-B cell line Ba/F3 cells (purchased from COBIOER BIOSCIENCES CO.,LTD) were infected by the virus obtained by the preparation method using a lentivirus packaging system described in Mohammadi Z et al., Mol Biotechnol. 2015 Sep; 57(9): 793-800, followed by selecting via puromycin + bleomycin and screening single clones, to obtain monoclonal stable cell line Ba/F3-hTSLPR-hIL7Rα.

### 2.2 Detection of Ba/F3 cell line overexpressing both human TSLPR/IL7Rα genes

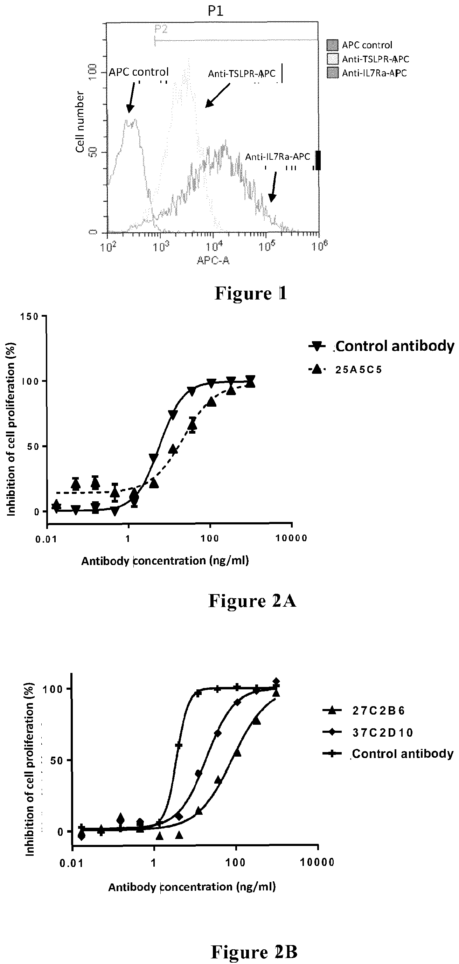

The cell line was detected by flow cytometry (flow cytometry: Beckman, CytoFlex; detection antibodies: APC-anti-human TSLPR (Biolegend), APC-anti-human IL7Rα (Biolegend)), to determine if the monoclonal cells correctly expressed human TSLPR and human IL7Rα. As shown in Fig.1, the flow cytometry results showed that Ba/F3-hTSLPR-hIL7Rα was a monoclonal cell line expressing both genes (nearly 100%) with good homogeneity, which can be used for subsequent experiments.

### Example 3: mouse immunization and hybridoma fusion

### 3.1 Mouse immunization

Fully human transgenic mice H2L2 (Harbour BioMed) were immunized for multiple times using human TSLP (NP_149024.1) expressed by Escherichia coli, human TSLP (NP_149024.1) expressed by mammalian cells, monkey TSLP (XP_005557555.1) expressed by mammalian cells and plasmids containing the complete coding sequence of human TSLP, respectively. Booster immunization was carried out every two weeks, for 5-6 times in total. During immunization, the serum titer of anti-human TSLP antibody was detected by ELISA every two weeks (see Example 4.1), and after multiple rounds of immunization, depending on the titer, mice having the best titer were selected for fusion to produce hybridoma.

### 3.2 Fusion method

After single-cell suspensions of spleen and lymph node were mixed, SP2/0 myeloma cells in an equivalent amount were added and mixed evenly. The mixed solution of cells was washed and resuspended with electrofusion buffer, and after electrofusion using a BTX-ECM2001 electrofusion device, the cell suspension was immediately transferred from the fusion chamber to a complete fusion culture medium and incubated at 37°C for 1 hour. The cells were plated to a 96-well plate at a density of 2×10⁴ cells per well. After culturing for 5 days, the medium was exchanged with the complete fusion medium, and in 7-10 days, the supernatant was collected for screening hybridoma.

### Example 4: Hybridoma screening

### 4.1 ELISA screening of human TSLP-binding

The soluble human TSLP-His protein was diluted to 1 µg/mL in 1×CBS coating buffer, then added to 96-well plates, and incubated overnight at 4°C. The 96-well plates were washed with PBST and blocked with a blocking solution (PBS+2% BSA) at 37°C for 2 hours. The hybridomas supernatant, or the same volume of blocking solution were added to plates, and incubated in an incubator at 37°C for 2 hours. The 96-well plates were added with goat anti-rat IgG-HRP, and incubated in an incubator at 37°C for 1 hour, then washed and read OD at 450 nm.

### 4.2 ELISA screening of monkey TSLP-binding

The monkey TSLP-His protein was diluted to 1 µg/mL in CBS coating buffer, then added to 96-well plates, and incubated overnight at 4°C. The 96-well plates were washed with PBST and blocked with a blocking solution (PBS+2% BSA) at 37°C for 2 hours. The hybridomas supernatant, or the same volume of blocking solution were added to plates, and incubated in an incubator at 37°C for 2 hours. The 96-well plates were added with goat anti-rat IgG-HRP, and incubated in an incubator at 37°C for 1 hour, then washed and read OD at 450 nm.

### 4.3 ELISA screening of blocking the binding of human TSLP-His to chimeric receptor IL7Rα-TSLPR-hFc

According to the following scheme, the hybridoma supernatant or purified antibody was assayed for blocking the binding of human TSLP-His to chimeric receptor IL7Rα-TSLPR-hFc.

The soluble hIL7Rα-hTSLPR-hFc protein was diluted in 1×CBS coating buffer, then added to 96-well plates, and incubated overnight at 4°C. The 96-well plate was washed with PBST and blocked with a blocking solution (PBS+2% BSA) at 37°C for 2 hours. The hTSLP-His protein (+/-) recombinantly expressed by mammalian cells was added to the plates, together with the hybridoma supernatant or the same volume of blocking solution, and incubated in an incubator at 37°C for 2 hours. The 96-well plates were added with mouse anti-His-HRP, and incubated in an incubator at 37°C for 1 hour, then washed and read OD at 450 nm. Hybridomas with strong inhibition rate were selected as candidate clones.

According to the activities assayed by affinity ELISA and competitive ELISA above, positive clones were selected and subcloned by the limited dilution method to obtain subclones. Finally, the hybridoma subclones which can bind to both human TSLP-His and monkey TSLP-His, as well as block the binding of human TSLP-His to chimeric receptor hIL7Rα-hTSLPR-hFc were obtained. As shown in Table 1, five single clones had strong binding ability for both human TSLP (hTSLP) and monkey TSLP (cTSLP), and also blocked the binding ability of human TSLP to its receptor hIL7Rα-hTSLPR-hFc, with an inhibition rate exceeding 60%.

**Table 1: Screening of anti-TSLP hybridoma subclone**

| **Clone** | **hTSLP binding (OD450)** | **cTSLP binding (OD450)** | **hTSLP/hIL7Rα-hTSLPR-hFc inhibition rate (%)** |
|---|---|---|---|
| **25A5C5** | **2.4** | **0.12** | **95.9** |
| **27C2B6** | **1.4** | **1.11** | **69.8** |
| **37C2D10** | **1.1** | **1.12** | **81.0** |
| **43B1A8** | **1.92** | **1.11** | **80.7** |
| **90H3H11** | **0.7** | **1.02** | **87.0** |

### Example 5: Preparation of anti-TSLP chimeric antibody

Control antibody expression: the sequence of control antibody refers to chEMBL database (ID: CHEMBL3707229). The base sequences of heavy and light chains of control antibody were synthesized in pTT5 expression vector, and transiently transfected and expressed by CHO-S Cells (Purchased from Thermo), followed by affinity purification using protein A (MabSelect SuRe, GE), to obtain the control antibody.

Single hybridoma clones were cultured in serum-free medium to obtain 50 mL of supernatant, followed by purification using Protein A (MabSelect SuRe, GE), to obtain chimeric antibodies of hybridomas. Since H2L2 mouse antibody constant region was genetically modified into a rat constant region, the obtained purified antibody was a chimeric antibody with fully human variable regions carrying rat Fc. The purified antibodies were quantified by spectrophotometry to obtain chimeric antibodies 25A5C5, 27C2B6, 37C2D10, 43B1A8 and 90H3H11.

### Example 6: ELISA detection of the affinity of anti-TSLP chimeric antibodies for TSLP

The affinity of chimeric antibodies 25A5C5, 27C2B6, 37C2D10, 43B1A8 and 90H3H11 for human or monkey TSLP was assayed by ELISA. The specific method was briefed as follows: coating human or monkey TSLP-His antigen into 96-well plates, after overnight at 4°C, adding antibodies diluted with different concentration gradients, respectively, after incubating for 2 hours, adding goat anti-rat Fc-HRP secondary antibody, and after incubating for 1 hour, reading the absorption value at 450 nm wavelength by microplate reader.

The results were shown in Table 2, in which chimeric antibodies 25A5C5 and 27C2B6 each had an affinity for human TSLP substantially comparable to the control antibody, while antibodies **43B1A8** and 90H3H11 each had higher affinity for human TSLP than that of the control antibody. Antibodies 25A5C5 and 90H3H11 had no binding to monkey TSLP; while 37C2D10, 27C2B6 and 43B1A8 each strongly bind to monkey TSLP.

**Table 2: Affinity of anti-TSLP chimeric antibody to TSLP**

| **Antibody** | **hTSLP binding EC50 (nM)** | **cTSLP binding EC50 (nM)** |
|---|---|---|
| **25A5C5** | **0.208** | **No binding** |
| **27C2B6** | **0.221** | **0.216** |
| **37C2D10** | **0.300** | **0.495** |
| **43B1A8** | **0.115** | **0.568** |
| **90H3H11** | **0.139** | **>100** |
| **Control antibody** | **0.233** | **0.336** |

### Example 7: Detection of activity of anti-TSLP chimeric antibody of blocking TSLP/hIL7Rα-hTSLPR-hFc binding by competitive ELISA

Human TSLP binds to human hIL7Rα-hTSLPR-hFc heterodimer receptor to activate downstream signaling pathways. Competitive ELISA was applied to assay the activity of chimeric antibodies of blocking the binding of chimeric receptor hIL7Rα-hTSLPR-hFc to the antigen. The specific steps refers to Example 4.3, the gradient diluted chimeric antibodies were added to a plate. The results were shown in the Table 3, in which all 5 chimeric antibodies effectively blocked the binding of human TSLP to human hIL7Rα-hTSLPR-hFc heterodimer receptor.

**Table 3: Activity of anti-TSLP chimeric antibody by competitive ELISA**

| **Antibody** | **25A5C5** | **27C2B6** | **37C2D10** | **43B1A8** | **90H3H11** | **Control antibody** |
|---|---|---|---|---|---|---|
| **IC50 (nM)** | **0.89** | **0.77** | **0.51** | **1.33** | **1.65** | **0.93** |

Example 8: Inhibition of anti-TSLP chimeric antibody on the proliferation of Ba/F3-hTSLPR-hIL7Rα cells

The activity of anti-TSLP chimeric antibody was detected by inhibiting Ba/F3-hTSLPR-hIL7Rα cells proliferation. Receptor proteins hTSLPR and hIL7Rα were expressed on the cell surface of Ba/F3-hTSLPR-hIL7Rα, wherein, the dimer of the extracellular domains of these two receptor proteins can bind to human TSLP, and the intracellular domains thereof can further transduce signal to activate the intracellular STAT5 phosphorylation, and promote the proliferation of Ba/F3-hTSLPR-hIL7Rα cells.

The specific steps were as follows: taking an appropriate amount of Ba/F3-hTSLPR-hIL7Rα stable cell line for centrifuging and washing with 1640 + 10% FBS culture medium for 2 times, to remove the recombinant mouse IL3 in the culture medium; incubating hTSLP-His (+/-) together with purified anti-TSLP chimeric antibody/or control anti-TSLP antibody (Control antibody) in each well at room temperature for 30 minutes; adding 1.5×10⁴ Ba/F3-hTSLPR-hIL7Rα cells per well and incubating for 3 days; adding CCK8 (RHINO BIO, QDY-003-D) to each well, and reading OD at 450 nm, exporting the data and analyzing the inhibition of chimeric antibody on cell proliferation by using Prism Graphpad software.

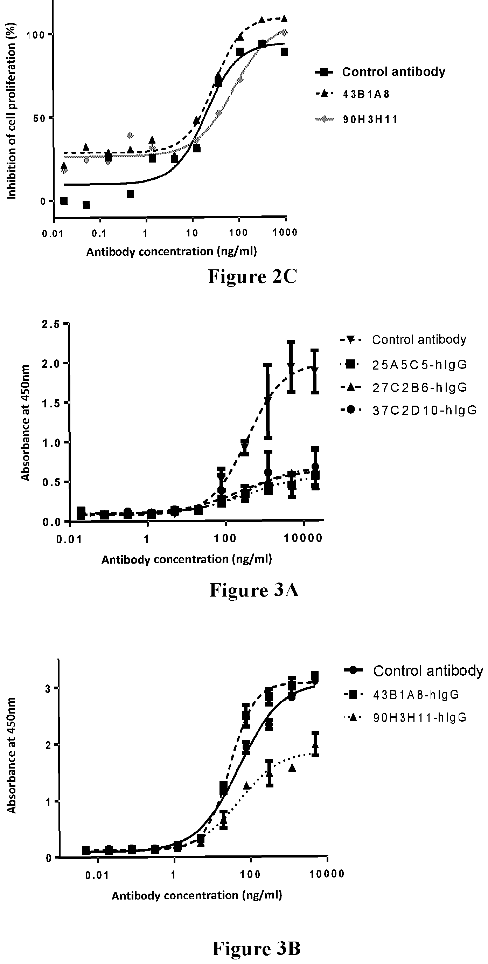

As shown in Fig. 2A-2C and Table 4, all 5 chimeric antibodies obviously inhibited the proliferation of Ba/F3-hTSLPR-hIL7Rα cells.

**Table 4: Assay of activity of chimeric antibody of blocking human TSLP-induced proliferation of Ba/F3-hTSLPR-hIL7Rα cells**

| **Antibody** | **25A5C5** | **27C2B6** | **37C2D10** | **43B1A8** | **90H3H11** | **Control antibody** |
|---|---|---|---|---|---|---|
| **EC50 (nM)** | **0.11** | **1.22** | **0.28** | **0.10** | **0.25** | **0.05** |

### Example 9: Amplification of variable regions of anti-TSLP chimeric antibody

Hybridoma cells were cultured to about 2 × 10⁶, lysed by using TRIzol reagent (Thermo Fisher Sci. Cat # 15596026) to extract RNA, then a cDNA reverse transcription kit (Thermo Fisher Sci. Cat # 18080-200) was applied for the synthesis of the first strand cDNA. With reference to the methods of IMGT and AbM, as well as the sequence analysis of all murine antibodies, multiple pairs of upstream primers for variable regions were designed by selecting the regions with high homology, downstream primers were designed by using CH1 homologous sequence, and the light and heavy chain variable regions of the antibodies were obtained by PCR amplification with primer pool, and the PCR product was purified using a DNA purification kit (Qiagen, Cat#28104) and cloned into pTT-5 vector, about 10 clones were selected for sequencing in each ligation reaction to obtain the sequences of variable regions, which were further analyzed by IMGT and AbM databases.

**Table 5: Amino acid sequences and NOs of variable regions and CDRs of anti-human TSLP chimeric antibodies**

| **Clone** | **25A5C5** | | **27C2B6** | | **37C2D10** | | **43B1A8** | | **90H3H11** | |
|---|---|---|---|---|---|---|---|---|---|---|
| **Heavy Chain variable region** | **1** | | **17** | | **30** | | **40** | | **53** | |
| **Light Chain variable region** | **2** | | **18** | | **31** | | **41** | | **54** | |
| **Numbering system** | **IMGT** | **AbM** | **IMGT** | **AbM** | **IMGT** | **AbM** | **IMGT** | **AbM** | **IMGT** | **AbM** |
| **Heavy chain CDR1** | **3** | **9** | **19** | **25** | **32** | **36** | **42** | **48** | **55** | **61** |
| **Heavy chain CDR2** | **4** | **10** | **20** | **26** | **33** | **37** | **43** | **49** | **56** | **62** |
| **Heavy chain CDR3** | **5** | **11** | **21** | **27** | **34** | **38** | **44** | **50** | **57** | **63** |
| **Light Chain CDR1** | **6** | **12** | **22** | **28** | **35** | **39** | **45** | **51** | **58** | **64** |
| **Light Chain CDR2** | **7** | **13** | **23** | **29** | **23** | **29** | **46** | **52** | **59** | **65** |
| **Light Chain CDR3** | **8** | **8** | **24** | **24** | **24** | **24** | **47** | **47** | **60** | **60** |

### Example 10: Expression, purification and binding affinity detection of recombinant antibody

The amino acid sequences of the light chain variable regions of 25A5C5, 27C2B6, 37C2D10, **43B1A8** and 90H3H11 were fused to the amino acid sequence of the light chain κ constant region (SEQ ID NO: 16), respectively, and the amino acid sequences of the heavy chain variable regions thereof were fused to the amino acid sequence of the heavy chain constant region of IgG1 (SEQ ID NO: 14), respectively, and the nucleotide sequences corresponding to these sequences were constructed into pTT5 vectors. The pTT5 vectors corresponding to the heavy and light chains of each recombinant fully human antibody were simultaneously transfected into CHO-S (purchased from Thermo), the supernatant was purified using Protein A (MabSelect SuRe, GE), and the purified recombinant fully human antibodies were named as 25A5C5-hlgG, 27C2B6-hIgG, 37C2D10-hlgG, 43B1A8-hIgG and 90H3H11-hIgG, respectively, each being quantified for protein concentration by spectrophotometry.

Detection of the affinity of anti-TSLP recombinant fully human antibodies for mammal-expressed recombinant human or monkey TSLP-His. The specific experimental steps were briefed as follows: coating human TSLP-His or monkey TSLP-His antigen into a 96-well plate, after overnight at 4°C, adding serially diluted antibodies, respectively, after incubating for 2 hours, adding HRP-labeled goat anti-human Fc secondary antibody, and after incubating for 1 hour, reading at wavelength of 450 nm.

The results were shown in Fig. 3A-3B, in which all 5 candidate antibodies can bind to human TSLP. Also, as shown in Table 6, 37C2D10-hlgG, 43B1A8-hIgG and 90H3H11-hlgG each had higher affinity for hTSLP than the control antibody. 25A5C5-hlgG and 27C2B6-hlgG each had comparable affinity for hTSLP with the control antibody. 27C2B6-hlgG, 37C2D10-hlgG and 43B1A8-hIgG each had comparable affinity for monkey TSLP with the control antibody.

**Table 6: The binding of recombinant fully human antibodies for human/monkey TSLP**

| **Antibody** | **25A5C5-hlgG** | **27C2B6-hlgG** | **37C2D10-hlgG** | **43B1A8-hIgG** | **90H3H11-hlgG** | **Control antibody** |
|---|---|---|---|---|---|---|
| **hTSLP binding EC50 (nM)** | **1.45** | **1.62** | **0.90** | **0.80** | **1.36** | **1.40** |
| **cTSLP binding EC50 (nM)** | **No binding** | **0.18** | **0.17** | **0.23** | **>100** | **0.19** |

### Example 11: Detection of the dynamic affinity of anti-TSLP fully human antibody for TSLP

The dynamic affinity of anti-TSLP fully human chimeric antibody for TSLP was detected by Fortibio, a commonly used dynamic affinity detection device. The method was described briefly as follows: carrying out series dilution of human or monkey TSLP with PBST to obtain 100 nM, 50 nM, 25 nM, 12.5 µM, 6.25 nM, 3.125 nM, 1.5625 nM and 0 nM; pre-wetting a ProA biosensor (Pall Life Sciences) with PBST buffer before use; diluting the recombinant fully human antibody to 5µg/mL with PBST and immobilizing on the ProA sensor; then placing the sensor with immobilized antibody in PBST buffer for equilibrating 60s to obtain the baseline and transferring to the antigen diluent for binding for 60s, and then dissociating in PBST for 180s; regenerating the sensor with 10 mM Gly (pH 1.5) after an analysis cycle. Date analysis 11.0 version (Pall) with 1:1 model was used to determine the association rate constant (Ka) and the dissociation rate constant (Kd), which were then used to calculate the dissociation equilibrium constant (KD).

As shown in Table 7, the KD values of 25A5C5-hlgG, 43B1A8-hIgG and 90H3H11-hlgG binding to human TSLP were less than that of the control antibody, showing stronger affinity, which were consistent with the affinity ELISA results. For binding to monkey TSLP, 25A5C5-hlgG had no affinity; while 37C2D10-hlgG, 90H3H11-hlgG and 43B1A8-hlgGlt each showed good binding with an affinity in the order of 10⁻⁸ M to 10⁻⁹ M.

**Table7: Dynamic affinity analysis of fully human antibodies**

| **Antibody** | **Affinity for hTSLP KD (M)** | **Affinity for cTSLP KD(M)** |
|---|---|---|
| **25A5C5-hlgG** | **5.58E-10** | **No binding** |
| **37C2D10-hlgG** | **1.76E-09** | **2.01E-08** |
| **43B1A8-hIgG** | **5.81E-10** | **1.02E-09** |
| **90H3H11-hlgG** | **6.04E-10** | **1.08E-08** |
| **Control antibody** | **8.00E-10** | **1.15E-09** |

Example 12: Inhibition of recombinant fully human antibodies on the proliferation of Ba/F3-hTSLPR-hIL7α cells

The activity of recombinant fully human antibody was assayed by the proliferation inhibition method of Ba/F3-hTSLPR-hIL7α cells. The method refers to the preceding Example 8.

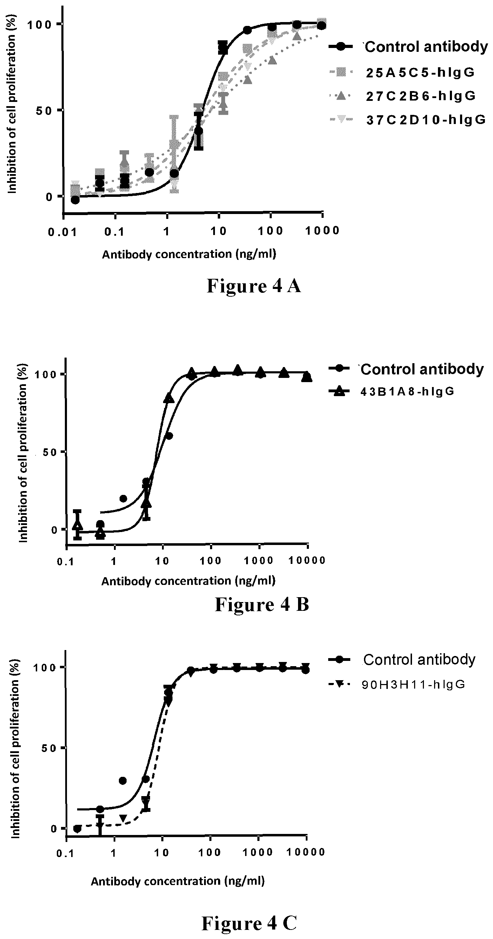

The results were shown in Table 8, in which 27C2B6-hlgG and 37C2D10-hlgG can inhibit human TSLP-induced proliferation of Ba/F3-hTSLPR-hIL7Rα cells; 25A5C5-hlgG and 90H3H11-hlgG were substantially comparable to the control antibody in terms of inhibiting human TSLP-induced proliferation of Ba/F3-hTSLPR-hIL7Rα cells; while 43B1A8-hIgG had a significantly stronger inhibitory activity than (about 0.25 times) that of the control antibody, as shown in the Fig. 4A-C.

**Table 8: Inhibition activity of recombinant fully human antibodies on the proliferation of Ba/F3-hTSLPR-hIL7Rα cells**

| **Antibody** | **hTSLP EC50(nM)** |
|---|---|
| **25A5C5-hlgG** | **0.044** |
| **27C2B6-hlgG** | **0.064** |
| **37C2D10-hlgG** | **0.060** |
| **43B1A8-hIgG** | **0.031** |
| **90H3H11-hlgG** | **0.050** |
| **Control antibody** | **0.041** |

### Example 13: Detection of inhibition activity of recombinant fully human antibody on human TSLP-induced PBMC secretion of thymus and activation-regulated chemokine (TARC).

The functional activity of fully human anti-TSLP antibody in primary cells was assessed by inhibiting human TSLP-induced secretion of TARC (a Th2-like cytokine) by human PBMC cells. CD11⁺ DC cells were contained in the PBMCs, and studies have shown that TSLP can bind to receptor TSLPR/IL7Rα and activate CD11⁺ DC cells, up-regulate OX40L and promote CD11⁺ DC cells to secrete Th2-like cytokines (e.g., TARC and CCL22). Anti-TSLP antibodies can block the binding of TSLP to TSLPR/IL7Rα on the surface of DC cells, thereby blocking the activation of DC cells and secretion of Th2-like cytokines by DC cell.

The method was briefly described as follows: isolating human peripheral blood PBMC using Ficoll separation solution (GE), incubating hTSLP-His (+/-) together with recombinant fully human antibodies or the control antibody for 30 minutes at room temperature, and adding 2×10⁵ PBMC cells per cell, incubating for 48 hours. The supernatant was collected and analyzed by ELISA for the human TARC, and the inhibition of the hybridoma supernatant or purified antibody on TARC secretion was assayed. TARC ELISA kit (Sino Biological) was used to detect the TARC level in the supernatant.

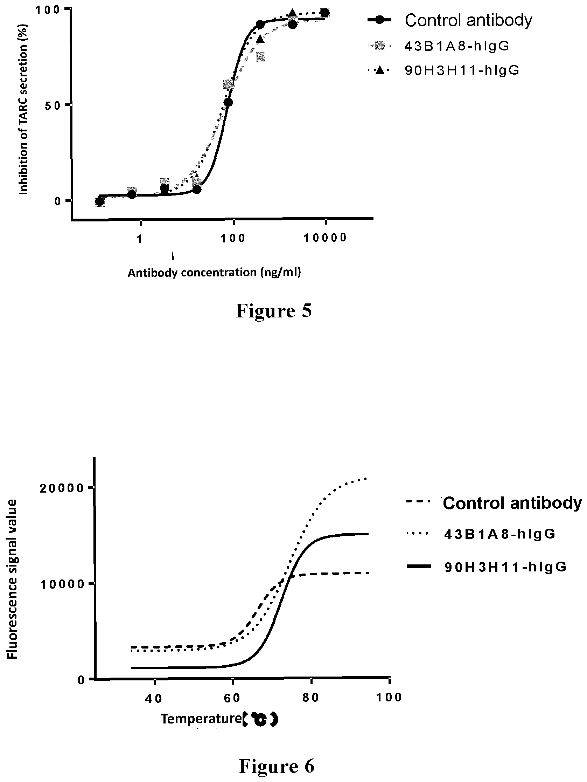

The results were shown in Table 9 and Fig. 5, in which the EC50 values of fully human antibodies 43B1A8-hIgG and 90H3H11-hlgG were lower than that of the control antibody, indicating that the activities thereof of inhibiting TSLP-induced secretion of TARC by PBMC were higher than that of control antibody.

**Table 9: Inhibition of recombinant fully human antibody on human TSLP-induced PBMC secretion of TARC**

| **Antibody** | **43B1A8-hlgG** | **90H3H11-hlgG** | **Control antibody** |
|---|---|---|---|
| **EC50(nM)** | **0.44** | **0.42** | **0.51** |

### Example 14: Assay of the Tm value of recombinant fully human antibody

The Tm value of anti-TSLP antibody was measured by DSF (differential fluorescence scanning technology). The specific experimental steps were as follows: mixing 12.5µL of 40x SYPRO Orange dye (Life Technologies Co., Ltd., Cat No. 56651), 5µL of 1mg/mL fully human anti-TSLP antibody (diluted in PBS) and 7.5µL of sterile water in an EP tube, adding the sample mixture to a Q-PCR system (AB Applied Biosystems ABI, 7500) for reaction, Q-PCR parameter settings: Target (ROX), program (25°C, 3 min; a rate of 1%, 95°C; 95°C, 2 min). The results were inputted into Graph Prism software to calculate the V50 value. As shown in Fig. 6 and Table 10, the Tm values of 43B1A8-hIgG (74.72°C) and 90H3H11-hlgG (72.58°C) were higher than that of the control antibody (66.47°C), clearly indicating that the fully human antibodies prepared by the invention had better thermal stability.

**Table 10: Tm value of recombinant fully human anti-TSLP antibody**

| **Antibody** | **Control antibody** | **43B1A8-hlgG** | **90H3H11-hlgG** |
|---|---|---|---|
| **Tm(°C)** | **66.47** | **74.72** | **72.58** |

### Example 15: Assay of hydrophobicity of recombinant fully human antibody

The hydrophobicity comparison was analyzed by Agilent 1260 HPLC combined with TOSOH Tskgel Buty-NPR (2.5) chromatographic column. In order to compare the hydrophobicity difference of three antibodies, these three antibodies were directly loaded for analysis. Mobile phase A: 1.5M (NH₄)₂SO₄; mobile phase B: 25mM Na₂HPO₄(pH7.0) +25% IPA. The retention times of the three antibodies were shown in Table 11, wherein, the longer the retention time, the stronger the hydrophobicity of the antibody, 90H3H11-hlgG had a hydrophilicity comparable to that of the control antibody; 43B1A8-hIgG had a better hydrophilicity than that of the control antibody, indicating that the process development and antibody aggregation will be superior to the control antibody.

**Table 11: Detection of hydrophobicity of recombinant fully human antibody**

| Antibody | Control antibody | 43B1A8-hIgG | 90H3H11-hlgG |
|---|---|---|---|
| Retention time (minutes) | 13.93 | 12.2 | 13.61 |

### Example 16: Preparation of recombinant modified fully human antibodies 43B1-H6L1 and 43B1-H2L2

The in vivo half-life of an antibody was closely related to the isoelectric point, affinity for FcRn, glycosylation modification and immunogenicity thereof. In order to prolong the half-life of 43B1A8-hIgG antibody, the affinity for FcRn was enhanced by modifying the amino acid sequences of the heavy chain and light chain of the antibody.

Modification scheme 1: hIgG1 Fc (SEQ ID NO: 14) was replaced with IgG4 (SEQ ID NO: 70 ) containing 2 amino acid mutations, one being from N to A at amino acid position 434 (EU numbering system), and the other from S to P at amino acid position 228 (EU numbering system) of IgG4.

Modification scheme 2: N was replaced with A at amino acid position 434 (EU numbering system) of hIgG1 Fc (SEQ ID NO: 14) to obtain SEQ ID NO: 15; and R was mutated into G at position 16 (Chothia numbering system) of FR1 of the heavy chain of 43B1A8-hIgG; and R was mutated into Q at position 79 (Chothia numbering system) of FR3 of the light chain of 43B1A8-hIgG.

The antibodies obtained by the above modification schemes 1 and 2 were named as 43B1-H6L1 and 43B1-H2L2, respectively.

The heavy chain variable region sequence of 43B1-H2L2 is SEQ ID NO: 68; the heavy chain constant region sequence thereof is SEQ ID NO: 15; the light chain variable region sequence thereof is SEQ ID NO: 69; and the light chain constant region sequence thereof is SEQ ID NO: 16.

The heavy chain variable region sequence of 43B1-H6L1 is SEQ ID NO: 40; the heavy chain constant region sequence thereof is SEQ ID NO: 70; the light chain variable region sequence thereof is SEQ ID NO: 41; and the light chain constant region sequence thereof is SEQ ID NO: 16.

**Table 12: Amino acid sequences of antibodies 43B1-H6L1 and 43B1-H2L2 and NOs (SEQ ID NO)**

| Antibody | Heavy chain | Heavy chain variable region | Heavy chain constant region | Light chain | Light chain variable region | Light chain constant region |
|---|---|---|---|---|---|---|
| **43B1A8-hlgG** | **66** | **40** | **14** | **67** | **41** | **16** |
| **43B1-H2L2** | **71** | **68** | **15** | **72** | **69** | **16** |
| **43B1-H6L1** | **73** | **40** | **70** | **67** | **41** | **16** |

The heavy and light chains of the above two modified molecules 43B1-H2L2 and 43B1-H6L1 were constructed into pTT5 expression vectors, respectively, and after extracting plasmids, they were transfected into CHO-S cells (purchased from Thermo), after culturing for about 10 days, the cell supernatant was purified using Protein A (MabSelect SuRe, GE), and the purified recombinant fully human antibodies were quantified for protein content by spectrophotometry.

Example 17: Assay of dynamic affinity of recombinant fully human antibodies 43B1-H6L1 and 43B1-H2L2 for TSLP

The dynamic affinity of anti-TSLP fully human antibody for TSLP was detected by Fortibio. The method refers to the preceding Example 11. The results were shown in Table 13, in which 43B1-H6L1 and 43B1-H2L2 human antibody molecules each had a dynamic affinity for hTSLP not weaker than that of the control antibody.

**Table 13: Analysis of dynamic affinity of modified fully human antibody molecules**

| **Antibody** | **hTSLP affinity KD (M)** |
|---|---|
| **43B1-H2L2** | **2.33E-10** |
| **43B1-H6L1** | **4.19E-10** |
| **Control antibody** | **3.73E-10** |

### Example 18: Assay of isoelectric points (PI) of recombinant fully human antibodies 43B1-H6L1 and 43B1-H2L2

The isoelectric points of 43B1-H6L1, 43B1-H2L2 and 43B1A8-hIgG antibodies were detected by the isoelectric focusing method, which was briefly described as follows: Maurice isoelectric focusing system (ProteinSimple) combined with its capillary cartridge was used for analysis. In order to compare the isoelectric point differences of the three antibodies, these three antibodies were diluted with water, and a pH gradient was formed by using ampholyte 3-10 (GE) at a final concentration of 4% in the detection system. The results were shown in Table 14, in which the isoelectric points of 43B1-H6L1 and 43B1-H2L2 were lower than that of 43B1A8-hIgG by 1.3 and 0.4, respectively, indicating that the modification schemes were successful.

**Table 14: Comparison of isoelectric points (PI) of recombinant fully human antibodies**

| **Antibody** | **43B1-H2L2** | **43B1-H6L1** | **43B1A8-hlgG** |
|---|---|---|---|
| **Isoelectric point** | **8.2** | **7.3** | **8.6** |

### Example 19: Assay of dynamic affinity of recombinant fully human antibodies 43B1-H6L1 and 43B1-H2L2 for FcRn

The dynamic affinity of the modified anti-TSLP fully human antibodies for FcRn was detected by Fortibio. The method was described briefly as follows: carrying out series dilution of anti-TSLP antibody with PBST (pH 6.0) to obtain 200 nM, 100 nM, 50 nM, 25 nM, 12.5 µM, 6.25 nM, 3.125 nM and 1.5625 nM and 0 nM; pre-wetting a SA biosensor (Pall Life Sciences) with PBST (pH 6.0) buffer before use; diluting biotin-labeled FcRn to 2.3 µg/mL and immobilizing on the SA sensor; then placing the sensor with immobilized FcRn in PBST (pH 6.0) buffer for equilibrating for 60s to obtain the baseline and transferring to the antibody diluent for binding for 60s, and then dissociating in PBST (pH 6.0) for 60s; regenerating the sensor with PBST (pH 7.4) after an analysis cycle. Date analysis 11.0 version (Pall) using 1:1 model was used to determine the association rate constant (Ka) and the dissociation rate constant (Kd), which were then used to calculate the dissociation equilibrium constant (KD).

As shown in Table 15, 43B1-H6L1 and 43B1-H2L2 each had a dynamic affinity (KD) for FcRn less than that of the control antibody, showing an affinity for FcRn of about 2 times of that of the control antibody.

**Table15: Analysis of affinity of modified fully human antibodies for FcRn**

| **Antibody** | **Affinity for FcRn KD (M)** |
|---|---|
| **43B1-H2L2** | **5.25E-09** |
| **43B1-H6L1** | **4.23E-09** |
| **Control antibody** | **1.12E-08** |

### Example 20: Assay of inhibitory activities of recombinant fully human antibodies 43B1-H6L1 and 43B1-H2L2 on the proliferation of Ba/F3-hTSLPR-hIL7Rα cells

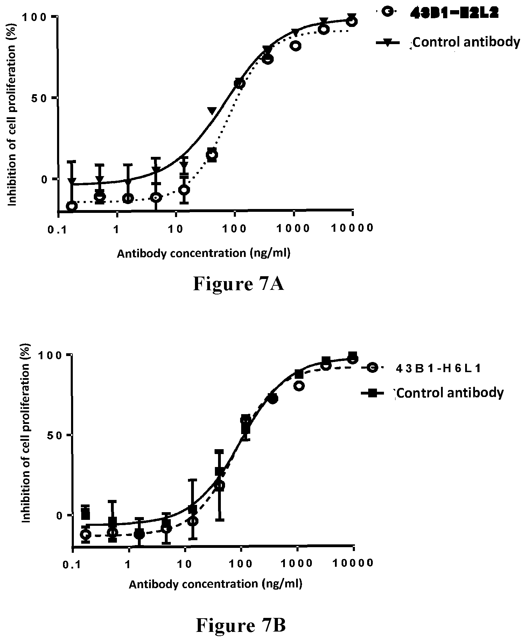

The activities of recombinant fully human antibodies 43B1-H6L1 and 43B1-H2L2 were detected by the proliferation inhibition method of Ba/F3 cells. The method refers to the preceding Example 8, and the activity EC50 of antibody was analyzed based on the data. The results were shown in Table 16 and Fig. 7A and 7B, in which 43B1-H2L2 and 43B1-H6L1 can inhibit human TSLP-induced proliferation of Ba/F3-hTSLPR-hIL7Rα cells, and the EC50 values thereof were comparable to that of the control antibody, indicating that fully human antibodies 43B1-H2L2 and 43B1-H6L1 each had a comparable ability of inhibiting the cell activity of TSLP.

**Table 16: Inhibitory activity of recombinant fully human antibody on the proliferation of Ba/F3-hTSLPR-hIL7Rα cells**

| | **43B1-H2L2** | **43B1-H6L1** | **Control antibody** |
|---|---|---|---|
| **hTSLP EC50 (nM)** | **0.64** | **0.46** | **0.57** |

### Example 21: Detection of inhibitory activity of recombinant fully human antibody on human TSLP-induced PBMC secretion of macrophage-derived chemokine (MDC).

The functional activity of the anti-TSLP fully human antibodies in primary cells was assessed by inhibiting human TSLP-induced secretion of MDC by human PBMC cells. PBMC comprises DC cells. Studies have shown that TSLP can bind to receptor TSLPR/IL7Rα on the cell surface and activate DC cells, up-regulate OX40L and promote DC cells to secrete Th2-like cytokines (e.g., TARC and MDC). Anti-TSLP antibodies can block the binding of TSLP to TSLPR/IL7Rα on the surface of DC cells, thereby blocking the activation of DC cells and the secretion of Th2-like cytokines by DC cells.

The method was briefly described as follows: isolating human peripheral blood PBMC using Ficoll separation solution (GE), incubating hTSLP-His (+/-) together with recombinant fully human antibodies or the control antibody for 30 minutes at room temperature, and adding 2×10⁵ PBMC cells per cell, and incubating for 120 hours. The supernatant was collected and analyzed by ELISA for the production of human MDC, and the inhibition of the antibody on the secretion of MDC was measured. MDC ELISA kit (Raybiotech) was used to detect the TARC level in the supernatant.

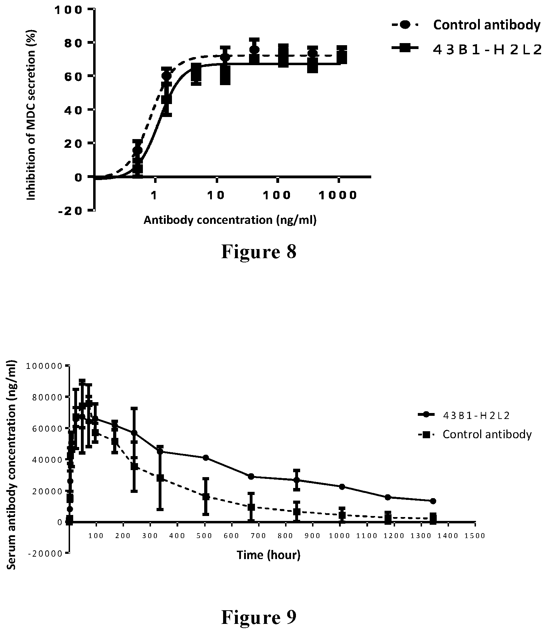

The results were shown in Table 17 and Fig. 8, in which 43B1-H2L2 and the control antibody can significantly inhibit TSLP-induced production of MDC, with EC50 values of 7.84 pM and 5.63 pM, respectively.

**Table 17: Inhibition of recombinant fully human antibody on human TSLP-induced PBMC secretion of MDC**

| **Antibody** | **43B1-H2L2** | **Control antibody** |
|---|---|---|
| **EC50 (pM)** | **7.84** | **5.63** |

### Example 22: In vivo pharmacokinetic analysis of recombinant fully human antibody in cynomolgus monkey

The sequence of candidate molecule 43B1-H2L2 of the present application contains N434A mutation in order to affect its affinity for FcRn, thereby prolonging its half-life of drug metabolism in vivo. Therefore, in this example, the pharmacokinetics of 43B1-H2L2 molecule in cynomolgus monkey in vivo was studied by subcutaneous injection, and was compared to that of the control antibody. The methods and results were as follows: four cynomolgus monkeys (Macaca fascicularis, available from Hainan Jingang Biotech Co., Ltd.) were selected and divided into two groups, one male and one female per group. The dose of subcutaneous injection was 5 mg/kg. Blood samples were collected at zero, 5 minutes, 30 minutes, 2 hours, 4 hours, 8 hours, 1 day, 2 days, 3 days, 4 days, 7 days, 10 days, 14 days, 21 days, 28 days, 35 days, 42 days, 49 days, and 56 days after administration, and left at room temperature for 1 hour until coagulation, and centrifuged to obtain the serum samples which were then frozen at -80°C to be tested. The antibody concentration in serum was determined by ELISA, and the results were analyzed as follows: the pharmacokinetic parameters and curves of single subcutaneous administration were shown in Table 18 and Fig. 9. These results showed that 43B1-H2L2 antibody had a longer half-life as well as a higher AUC of blood concentration, about one time of that of the control antibody.

**Table 18: Pharmacokinetic study of recombinant fully human antibody in cynomolgus monkey by subcutaneous administration**

| **PK parameters** | **t_{1/2}(h)** | **Tₘₐₓ(h)** | **Cₘₐₓ(ng/ml)** | **AUC₍₀₋ₜ₎(hr^{∗}ng/ml)** |
|---|---|---|---|---|
| **43B1-H2L2** | **549.3731** | **48** | **67373.4** | **47271741** |
| **Control antibody** | **267.3537** | **72** | **75870.56** | **25600470** |

Although specific embodiments of the invention have been described in detail, those skilled in the art will understand that various modifications and changes may be made to the details in accordance with all the published teachings, which are also included in the scope of the invention. The protection scope of the invention is defined by the appended claims and any equivalents thereof.

## Claims

1. An antibody or antigen-binding fragment thereof that binds to thymic stromal lymphopoietin (TSLP), wherein the antibody or antigen-binding fragment thereof comprises complementary determining regions (CDRs) as follows:
(a) a CDR-H1 or a sequence variant thereof, a CDR-H2 or a sequence variant thereof, and a CDR-H3 or a sequence variant thereof, contained in the heavy chain variable region (VH) as set forth in SEQ ID NO: 1, 17, 30, 40, 53 or 68; and/or
(b) a CDR-L1 or a sequence variant thereof, a CDR-L2 or a sequence variant thereof, and a CDR-L3 or a sequence variant thereof, contained in the light chain variable region (VL) as set forth in SEQ ID NO: 2, 18, 31, 41, 54 or 69;
preferably, the sequence variant is a CDR having a substitution, deletion or addition of one or more amino acids (for example, a substitution, deletion or addition of 1, 2 or 3 amino acids) as compared to the CDR from which it is derived; preferably, the substitution is a conservative substitution.

2. The antibody or antigen-binding fragment thereof according to claim 1, wherein the antibody or antigen-binding fragment thereof comprises:
(1) a VH and/or a VL, wherein, as defined by the IMGT numbering system:
(1a) the VH comprises a CDR-H1 with the sequence of SEQ ID NO: 3, a CDR-H2 with the sequence of SEQ ID NO: 4 and a CDR-H3 with the sequence of SEQ ID NO: 5; and/or, the VL comprises a CDR-L1 with the sequence of SEQ ID NO: 6, a CDR-L2 with the sequence of SEQ ID NO: 7 and a CDR-L3 with the sequence of SEQ ID NO: 8;
(1b) the VH comprises a CDR-H1 with the sequence of SEQ ID NO: 19, a CDR-H2 with the sequence of SEQ ID NO: 20 and a CDR-H3 with the sequence of SEQ ID NO: 21; and/or,
the VL comprises a CDR-L1 with the sequence of SEQ ID NO: 22, a CDR-L2 with the sequence of SEQ ID NO: 23 and a CDR-L3 with the sequence of SEQ ID NO: 24;
(1c) the VH comprises a CDR-H1 with the sequence of SEQ ID NO: 32, a CDR-H2 with the sequence of SEQ ID NO: 33 and a CDR-H3 with the sequence of SEQ ID NO: 34; and/or,
the VL comprises a CDR-L1 with the sequence of SEQ ID NO: 35, a CDR-L2 with the sequence of SEQ ID NO: 23 and a CDR-L3 with the sequence of SEQ ID NO: 24;
(1d) the VH comprises a CDR-H1 with the sequence of SEQ ID NO: 42, a CDR-H2 with the sequence of SEQ ID NO: 43 and a CDR-H3 with the sequence of SEQ ID NO: 44; and/or,
the VL comprises a CDR-L1 with the sequence of SEQ ID NO: 45, a CDR-L2 with the sequence of SEQ ID NO: 46 and a CDR-L3 with the sequence of SEQ ID NO: 47;
or
(1e) the VH comprises a CDR-H1 with the sequence of SEQ ID NO: 55, a CDR-H2 with the sequence of SEQ ID NO: 56 and a CDR-H3 with the sequence of SEQ ID NO: 57; and/or,
the VL comprises a CDR-L1 with the sequence of SEQ ID NO: 58, a CDR-L2 with the sequence of SEQ ID NO: 59 and a CDR-L3 with the sequence of SEQ ID NO: 60;
(2) a VH and/or a VL, wherein, as defined by the AbM numbering system:
(2a) the VH comprises a CDR-H1 with the sequence of SEQ ID NO: 9, a CDR-H2 with the sequence of SEQ ID NO: 10 and a CDR-H3 with the sequence of SEQ ID NO: 11; and/or, the VL comprises a CDR-L1 with the sequence of SEQ ID NO: 12, a CDR-L2 with the sequence of SEQ ID NO: 13 and a CDR-L3 with the sequence of SEQ ID NO: 8;
(2b) the VH comprises a CDR-H1 with the sequence of SEQ ID NO: 25, a CDR-H2 with the sequence of SEQ ID NO: 26 and a CDR-H3 with the sequence of SEQ ID NO: 27; and/or,
the VL comprises a CDR-L1 with the sequence of SEQ ID NO: 28, a CDR-L2 with the sequence of SEQ ID NO: 29 and a CDR-L3 with the sequence of SEQ ID NO: 24;
(2c) the VH comprises a CDR-H1 with the sequence of SEQ ID NO: 36, a CDR-H2 with the sequence of SEQ ID NO: 37 and a CDR-H3 with the sequence of SEQ ID NO: 38; and/or,
the VL comprises a CDR-L1 with the sequence of SEQ ID NO: 39, a CDR-L2 with the sequence of SEQ ID NO: 29 and a CDR-L3 with the sequence of SEQ ID NO: 24;
(2d) the VH comprises a CDR-H1 with the sequence of SEQ ID NO: 48, a CDR-H2 with the sequence of SEQ ID NO: 49 and a CDR-H3 with the sequence of SEQ ID NO: 50; and/or,
the VL comprises a CDR-L1 with the sequence of SEQ ID NO: 51, a CDR-L2 with the sequence of SEQ ID NO: 52 and a CDR-L3 with the sequence of SEQ ID NO: 47;
or
(2e) the VH comprises a CDR-H1 with the sequence of SEQ ID NO: 61, a CDR-H2 with the sequence of SEQ ID NO: 62 and a CDR-H3 with the sequence of SEQ ID NO: 63; and/or,
the VL comprises a CDR-L1 with the sequence of SEQ ID NO: 64, a CDR-L2 with the sequence of SEQ ID NO: 65 and a CDR-L3 with the sequence of SEQ ID NO: 60;
or
(3) a VH and/or a VL, wherein, as compared to the VH and/or the VL in any one of (1a), (1b), (1c), (1d), (1e) or (2a), (2b), (2c), (2d), (2e), at least one CDR contains a mutation, wherein the mutation is a substitution, deletion or addition of one or several amino acids or any combination thereof (for example, a substitution, deletion or addition of 1, 2 or 3 amino acids, or any combination thereof); preferably, the substitution is a conservative substitution;
preferably, the antibody or antigen-binding fragment thereof binds to human TSLP and/or monkey TSLP.

3. The antibody or antigen-binding fragment thereof according to claim 1 or 2, wherein the antibody or antigen-binding fragment thereof comprises:
(a) a VH as set forth in SEQ ID NO: 1, 17, 30, 40, 53 or 68, and/or a VL as set forth in any one of SEQ ID NO: 2, 18, 31,41, 54 or 69;
(b) a VH having at least 70%, at least 80%, at least 85%, at least 90%, at least 91%, at least 92%, at least 93%, at least 94%, at least 95%, at least 96%, at least 97%, at least 98% or at least 99% sequence identity to any VH in (a); and/or, a VL having at least 70%, at least 80%, at least 85%, at least 90%, at least 91%, at least 92%, at least 93%, at least 94%, at least 95%, at least 96%, at least 97%, at least 98% or at least 99% sequence identity to any VL in (a); or
(c) a VH having a substitution, deletion or addition of one or several amino acids or any combination thereof (for example, a substitution, deletion or addition of 1, 2, 3, 4, 5, 6, 7, 8, 9 or 10 amino acids, or any combination thereof) as compare to any VH in (a); and/or, a VL having a substitution, deletion or addition of one or several amino acids or any combination thereof (for example, a substitution, deletion or addition of 1, 2, 3, 4, 5, 6, 7, 8, 9 or 10 amino acids, or any combination thereof) as compare to any VL in (a); preferably, the substitution is a conservative substitution.

4. The antibody or antigen-binding fragment thereof according to any one of claims 1-3, wherein the antibody or antigen-binding fragment thereof comprises:
(a) a VH as set forth in SEQ ID NO: 1 and a VL as set forth in SEQ ID NO: 2;
(b) a VH as set forth in SEQ ID NO: 17 and a VL as set forth in SEQ ID NO: 18;
(c) a VH as set forth in SEQ ID NO: 30 and a VL as set forth in SEQ ID NO: 31;
(d) a VH as set forth in SEQ ID NO: 40 and a VL as set forth in SEQ ID NO: 41;
(e) a VH as set forth in SEQ ID NO: 53 and a VL as set forth in SEQ ID NO: 54;
(f) a VH as set forth in SEQ ID NO: 68 and a VL as set forth in SEQ ID NO: 69;
(g) a VH and a VL, wherein the VH has at least 70%, at least 80%, at least 85%, at least 90%, at least 91%, at least 92%, at least 93%, at least 94%, at least 95%, at least 96%, at least 97%, at least 98% or at least 99% sequence identity to; and/or, the VL has at least 70%, at least 80%, at least 85%, at least 90%, at least 91%, at least 92%, at least 93%, at least 94%, at least 95%, at least 96%, at least 97%, at least 98% or at least 99% sequence identity to the VH and the VL in any of (a) to (f); or
(h) a VH and a VL, wherein the VH has a substitution, deletion or addition of one or several amino acids or any combination thereof (for example, a substitution, deletion or addition of 1, 2, 3, 4, 5, 6, 7, 8, 9 or 10 amino acids, or any combination thereof); and/or the VL has a substitution, deletion or addition of one or several amino acids or any combination thereof (for example, a substitution, deletion or addition of 1, 2, 3, 4, 5, 6, 7, 8, 9 or 10 amino acids, or any combination thereof), as compared to the VH and VL in any of (a) to (f); preferably, the substitution is a conservative substitution.

5. The antibody or antigen-binding fragment thereof according to any one of claims 1-4, wherein the antibody or antigen-binding fragment thereof is a chimeric antibody, humanized antibody, or fully human antibody.

6. The antibody or antigen-binding fragment thereof according to any one of claims 1-5, wherein the antibody or antigen-binding fragment thereof further comprises:
(a) a heavy chain constant region (CH) of a human immunoglobulin or a variant thereof; and/or
(b) a light chain constant region (CL) of a human immunoglobulin or a variant thereof, wherein the variant has at least 70%, at least 80%, at least 85%, at least 90%, at least 91%, at least 92%, at least 93%, at least 94%, at least 95%, at least 96%, at least 97%, at least 98% or at least 99% sequence identity to the wild-type sequence from which it is derived; alternatively, the variant has a substitution, deletion or addition of one or more amino acids or any combination thereof (e.g. a substitution, deletion or addition of up to 50, up to 45, up to 40, up to 35, up to 30, up to 25, up to 20, up to 15, up to 10, or up to 5 amino acids or any combination thereof; e.g., a substitution, deletion or addition of 1, 2, 3, 4, 5, 6, 7, 8, 9, or 10 amino acids or any combination thereof) as compared to the wild-type sequence from which it is derived; preferably, the substitution is a conservative substitution;
preferably, the heavy chain constant region is an IgG heavy chain constant region, such as an IgG1, IgG2, IgG3 or IgG4 heavy chain constant region;
preferably, the antibody or antigen-binding fragment thereof comprises a heavy chain constant region of human IgG1;
preferably, the light chain constant region is kappa or lambda light chain constant region, and more preferably, the antibody or antigen-binding fragment thereof comprises a human kappa light chain constant region.

7. The antibody or antigen-binding fragment thereof according to claim 6, wherein the heavy chain constant region or the variant thereof comprises:
(1) a heavy chain constant region of human IgG1 or a variant thereof, wherein the variant is mutated at least one of positions 234, 235, 237, 265, 297, 331, 329 and 434 according to the EU numbering system, preferably, the variant comprises at least one of the following mutations: L234A, L235A, D265A, N297A, L234F, L235E, P331S, P329G, N434A, N434Y, N434F, N434W, N434S, N434G, N434H and N434Q; more preferably, the variant comprises at least one of the following mutations: L234A, L235A, G237A and N434A; more preferably, the IgG1 variant comprises the mutations of L234A, L235A and G237A; and more preferably, the IgG1 variant comprises the mutations of L234A, L235A, G237A and N434A; or
(2) a CH as set forth in SEQ ID NO: 14 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 14, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 14; the variant comprises N297A and/or N434A according to the EU numbering system, preferably, the variant comprises N434A;
(3) a CH as set forth in SEQ ID NO: 15 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 15, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 15; or
(4) a heavy chain constant region of human IgG4 or a variant thereof, wherein the variant is mutated at least one of positions 228 and 434 according to the EU numbering system; preferably, the variant comprises S228P and/or N434A; preferably, the variant comprises S228P and N434A; or
(5) a CH as set forth in SEQ ID NO: 70 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 70, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 70;
and/or
the light chain constant region or the variant thereof comprises:
(6) a kappa light chain constant region; or
(7) a light chain constant region (CL) as set forth in SEQ ID NO: 16 or a variant thereof, wherein the variant comprises conservative substitutions of up to 20 amino acids (e.g., conservative substitutions of up to 20, up to 15, up to 10, or up to 5 amino acids; e.g., conservative substitutions of 1, 2, 3, 4, 5, 6, 7, 8, 9, or 10 amino acids) as compared to SEQ ID NO: 16, or having at least 70%, at least 80%, at least 85%, at least 90%, at least 91%, at least 92%, at least 93%, at least 94%, at least 95%, at least 96%, at least 97%, at least 98% or at least 99% sequence identity to SEQ ID NO: 16;
preferably, the antibody or antigen-binding fragment thereof comprises a heavy chain constant region (CH) as set forth in SEQ ID NO: 14 and a light chain constant region (CL) as set forth in SEQ ID NO: 16;
preferably, the antibody or antigen-binding fragment thereof comprises a heavy chain constant region (CH) as set forth in SEQ ID NO: 15 and a light chain constant region (CL) as set forth in SEQ ID NO: 16;
preferably, the antibody or antigen-binding fragment thereof comprises a heavy chain constant region (CH) as set forth in SEQ ID NO: 70 and a light chain constant region (CL) as set forth in SEQ ID NO: 16;
preferably, the mutation or substitution renders the antibody or antigen-binding fragment thereof to have no or reduced ADCP, ADCC and/or CDC activity, as compared to the corresponding antibody or antigen-binding fragment thereof not comprising said mutation or substitution.

8. The antibody or antigen-binding fragment thereof according to any one of claims 1-7, wherein the antibody comprises:
(a) a heavy chain comprising a VH as set forth in SEQ ID NO: 1 and a CH as set forth in SEQ ID NO: 14, 15 or 70, and a light chain comprising a VL as set forth in SEQ ID NO: 2 and a CL as set forth in SEQ ID NO: 16;
(b) a heavy chain comprising a VH as set forth in SEQ ID NO: 17 and a CH as set forth in SEQ ID NO: 14, 15 or 70, and a light chain comprising a VL as set forth in SEQ ID NO: 18 and a CL as set forth in SEQ ID NO: 16;
(c) a heavy chain comprising a VH as set forth in SEQ ID NO: 30 and a CH as set forth in SEQ ID NO: 14, 15 or 70, and a light chain comprising a VL as set forth in SEQ ID NO: 31 and a CL as set forth in SEQ ID NO: 16;
(d) a heavy chain comprising a VH as set forth in SEQ ID NO: 40 and a CH as set forth in SEQ ID NO: 14, 15 or 70, and a light chain comprising a VL as set forth in SEQ ID NO: 41 and a CL as set forth in SEQ ID NO: 16;
(e) a heavy chain comprising a VH as set forth in SEQ ID NO: 53 and a CH as set forth in SEQ ID NO: 14, 15 or 70, and a light chain comprising a VL as set forth in SEQ ID NO: 54 and a CL as set forth in SEQ ID NO: 16;
(f) a heavy chain comprising a VH as set forth in SEQ ID NO: 68 and a CH as set forth in SEQ ID NO: 14, 15 or 70, and a light chain comprising a VL as set forth in SEQ ID NO: 69 and a CL as set forth in SEQ ID NO: 16;
or
(g) a heavy chain and a light chain, wherein, the heavy chain has at least 80%, at least 85%, at least 90%, at least 91%, at least 92%, at least 93%, at least 94%, at least 95%, at least 96%, at least 97%, at least 98% or at least 99% sequence identity to, and/or, the light chain has at least 70%, at least 80%, at least 85%, at least 90%, at least 91%, at least 92%, at least 93%, at least 94%, at least 95%, at least 96%, at least 97%, at least 98% or at least 99% sequence identity to the heavy chain and the light chain in any of (a) - (f).

9. The antibody or antigen-binding fragment thereof according to any one of claims 1-8, wherein the antibody or antigen-binding fragment thereof comprises:
(a) a heavy chain and a light chain,
the heavy chain comprising:
(i) a sequence as set forth in SEQ ID NO: 66 or 73;
(ii) a sequence having a substitution, deletion or addition of one or several amino acids or any combination thereof (e.g. a substitution, deletion or addition of up to 50, up to 45, up to 40, up to 35, up to 30, up to 25, up to 20, up to 15, up to 10, or up to 5 amino acids or any combination thereof; e.g., a substitution, deletion or addition of 1, 2, 3, 4, 5, 6, 7, 8, 9, or 10 amino acids or any combination thereof) as compared to the sequence in (i); or
(iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
the light chain comprising:
(iv) a sequence as set forth in SEQ ID NO: 67;
(v) a sequence having a substitution, deletion or addition of one or several amino acids or any combination thereof (e.g. a substitution, deletion or addition of up to 50, up to 45, up to 40, up to 35, up to 30, up to 25, up to 20, up to 15, up to 10, or up to 5 amino acids or any combination thereof; e.g., a substitution, deletion or addition of 1, 2, 3, 4, 5, 6, 7, 8, 9, or 10 amino acids or any combination thereof) as compared to the sequence in (iv); or
(vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv);
preferably, the substitution in (ii) or (v) is a conservative substitution;
or
(b) a heavy chain and a light chain,
the heavy chain comprising:
(i) a sequence as set forth in SEQ ID NO: 71;
(ii) a sequence having a substitution, deletion or addition of one or several amino acids or any combination thereof (e.g. a substitution, deletion or addition of up to 50, up to 45, up to 40, up to 35, up to 30, up to 25, up to 20, up to 15, up to 10, or up to 5 amino acids or any combination thereof; e.g., a substitution, deletion or addition of 1, 2, 3, 4, 5, 6, 7, 8, 9, or 10 amino acids or any combination thereof) as compared to the sequence in (i); or
(iii) a sequence having at least 80%, at least 85%, at least 90%, at least 91%, at least 92%, at least 93%, at least 94%, at least 95%, at least 96%, at least 97%, at least 98% or at least 99% sequence identity to the sequence in (i); and
the light chain comprising:
(iv) a sequence as set forth in SEQ ID NO: 72;
(v) a sequence having a substitution, deletion or addition of one or several amino acids or any combination thereof (e.g. a substitution, deletion or addition of up to 50, up to 45, up to 40, up to 35, up to 30, up to 25, up to 20, up to 15, up to 10, or up to 5 amino acids or any combination thereof; e.g., a substitution, deletion or addition of 1, 2, 3, 4, 5, 6, 7, 8, 9, or 10 amino acids or any combination thereof) as compared to the sequence in (iv); or
(vi) a sequence having at least 80%, at least 85%, at least 90%, at least 91%, at least 92%, at least 93%, at least 94%, at least 95%, at least 96%, at least 97%, at least 98% or at least 99% sequence identity to the sequence in (iv);
preferably, the substitution in (ii) or (v) is a conservative substitution.

10. The antibody or antigen-binding fragment thereof according to any one of claims 1-9, wherein the antibody or antigen-binding fragment thereof is selected from the group consisting of scFv, Fab, Fab', (Fab')₂, Fv fragments, disulfide linked Fv (dsFv) and a diabody.

11. The antibody or antigen-binding fragment thereof according to any one of claims 1-10, wherein the antibody or antigen-binding fragment thereof carries a marker;
preferably, the antibody or antigen-binding fragment thereof carries a detectable marker such as an enzyme (e.g., horseradish peroxidase), a radionuclide, a fluorescent dye, a luminescent substance (e.g., a chemiluminescent substance) or biotin.

12. The antibody or antigen-binding fragment thereof according to any one of claims 1-11, wherein the antibody or antigen-binding fragment thereof exhibits at least one of the following characteristics:
(a) binding to TSLP (e.g., human TSLP) with a K_{D} of less than about 50 nM, e.g., less than about 20 nM, 10 nM, 1 nM, 0.1 nM, 0.01 nM, 1 pM, 0.1 pM or less, wherein the K_{D} is measured by Fortibio or ELISA;
(b) binding to TSLP (e.g., human TSLP) with an EC50 of less than about 50 nM, e.g., less than about 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1 pM, 0.1 pM or less, wherein the EC50 is measured by flow cytometry or ELISA;
(c) inhibiting the binding of TSLP to IL7Rα/TSLPR with an IC50 of less than about 50 nM, e.g., about 50 nM, 20 nM, 10 nM, 1 nM, 0.9 nM, 0.8 nM, 0.7 nM, 0.6 nM, 0.5 nM, 0.4 nM, 0.3 nM, 0.2 nM, 0.1 nM, 0.01 nM, 1 pM, 0.1 pM or less, wherein the IC50 is measured by ELISA;
(d) inhibiting or blocking TSLP-induced activation and/or proliferation of mast cells, DC, NKT cells;
(e) inhibiting or blocking TSLP-induced OX40L expression;
(f) inhibiting or blocking TSLP-induced osteoprotegerin (OPG) secretion;
(g) inhibiting or blocking TSLP-induced secretion of Th2 Cytokines, such as TARC, CCL22, IL-4, IL-13 or IL-5;
(h) having a good affinity for binding to FcRn;
(i) having an isoelectric point (PI) of about 6.5 to about 8.5, such as about 6.5, about 7.0, about 7.1, about 7.2, about 7.3, about 7.4, about 7.5, about 7.7, about 7.9, about 8.0, about 8.2 or about 8.5.

13. An isolated nucleic acid molecule encoding the antibody or antigen-binding fragment thereof according to any one of claims 1-12.

14. A vector comprising the isolated nucleic acid molecule according to claim 13; preferably, the vector is a cloning vector or an expression vector.

15. A host cell comprising the isolated nucleic acid molecule according to claim 13, or the vector according to claim 14.

16. A method for preparing the antibody or antigen-binding fragment thereof according to any one of claims 1-12, comprising: culturing the host cell according to claim 15 under conditions allowing expression of the antibody or antigen-binding fragment thereof, and recovering the antibody or antigen-binding fragment thereof from the cultured host cell culture.

17. A multispecific antibody, comprising the antibody or antigen-binding fragment thereof binding to TSLP according to any one of claims 1-12, and an additional antibody or fragment thereof or an antibody mimetic;
preferably, the multispecific antibody is a bispecific antibody or trispecific antibody or tetraspecific antibody.

18. A conjugate comprising the antibody or antigen-binding fragment thereof according to any one of claims 1-12 and a coupling moiety, wherein the coupling moiety is a detectable marker, such as a radioisotope, a fluorescent substance, a luminescent substance, a colored substance or enzyme, or the coupling moiety is a therapeutic agent.

19. A pharmaceutical composition comprising the antibody or antigen-binding fragment thereof according to any one of claims 1-12, the nucleic acid according to claim 13, the vector according to claim 14, the host cell according to claim 15, the multispecific antibody according to claim 17, and/or the conjugate according to claim 18, and a pharmaceutically acceptable carrier and/or excipient.

20. The pharmaceutical composition according to claim 19, wherein the pharmaceutical composition is used in at least one of the following biological activities in a subject:
(a) inhibiting or blocking the binding of TSLP to IL7Rα-TSLPR,
(b) down-regulating or eliminating the activity of TSLP,
(c) down-regulating or blocking OX40L expression,
(d) inhibiting or blocking TSLP-induced activation and/or proliferation of mast cells, DC, NKT cells,
(e) inhibiting or blocking TSLP-induced osteoprotegerin (OPG) secretion,
(f) inhibiting or blocking TSLP-induced secretion of Th2 Cytokines, such as TARC, CCL22, IL-4, IL-13 or IL-5.

21. A kit comprising the antibody or antigen-binding fragment thereof according to any one of claims 1-12, and/or the nucleic acid according to claim 13, and/or the vector according to claim 14, and/or the host cell according to claim 15, and/or the multispecific antibody according to claim 17, and/or the conjugate according to claim 18, and/or the pharmaceutical composition of claim 19 or 20, and optionally an instruction of use.

22. Use of the antibody or antigen-binding fragment thereof according to any one of claims 1-12, and/or the nucleic acid according to claim 13, and/or the vector according to claim 14, and/or the host cell according to claim 15, and/or the multispecific antibody according to claim 17, and/or the conjugate according to claim 18, and/or the pharmaceutical composition of claim 19 or 20 in the preparation of a medicament for preventing and/or treating an allergic inflammation or an autoimmune disease;
optionally, the allergic inflammation is selected from at least one of the group consisting of:
asthma, idiopathic pulmonary fibrosis, atopic dermatitis (AD), allergic conjunctivitis, allergic rhinitis (AR), Netherton syndrome, eosinophilic esophagitis (EOE), food allergy, allergic diarrhea, eosinophilic gastroenteritis, allergic bronchopulmonary aspergillosis (ABPA), allergic fungal sinusitis, rheumatoid arthritis, chronic obstructive pulmonary disease(COPD), systemic sclerosis, keloid, ulcerative colitis, chronic sinusitis (CRS) and nasal polyps, chronic eosinophilic pneumonia, eosinophilic bronchitis; Churg-Strauss syndrome, eosinophilia, eosinophilic granuloma with polyangiitis, inflammatory bowel disease, urticaria, systemic mastocytosis, cutaneous mastocytosis, and recurrent idiopathic angioedema; optionally, the autoimmune disease is selected from the group consisting of: diabetes, myasthenia gravis, gastritis, pemphigus, primary biliary cirrhosis, multiple sclerosis, lupus, colitis, rheumatoid diseases, psoriasis and thyroid diseases; optionally, the use comprises administration of the medicament in combination with one or more additional therapeutics selected from, but not limited to: immunosuppressants (for example, corticosteroids, non-steroidal glucocorticoid receptor agonists, leukotriene D4 antagonists, leukotriene B4 antagonists, A2A agonists, A2B antagonists, dopamine receptor agonists, pirfenidone, nintedanib, or avB6 antagonists), bronchodilators (for example, β-2 adrenergic receptor agonists, muscarinic antagonists, short-acting β2 receptor agonists, long-acting β2 receptor agonists, short-acting anticholinergic drugs, methyl xanthine drugs, long-acting anticholinergic drugs), other cytokine or cytokine receptor antagonists or antibodies (for example, IL-13 antagonists, IL-6 antagonists, antagonists of IL-1, IL-33, IL-25 or TNF-alpha, anti-IgE antibodies, anti-IL31 antibodies, anti-IL31R antibodies, anti-IL13 antibodies, anti-endoglin antibodies, anti-ILlb antibodies, another anti-TSLP antibody or anti-hTSLPR antibodies), antibiotics, radiotherapy, leukotriene antagonists (for example, montelukast, zafirlukast or pranlukast), PDE4 inhibitors (for example, roflumilast, xanthene), antihistamines or antitussive drugs;
optionally, the antibody or antigen-binding fragment thereof is administered sequentially or simultaneously with an additional therapeutic.

23. A method of in vivo/in vitro at least one of: (a) inhibiting or blocking the binding of TSLP to IL7Rα-TSLPR; (b) down-regulating or eliminating the activity of TSLP; (c) down-regulating or blocking OX40L expression; (d) inhibiting or blocking TSLP-induced activation and/or proliferation of mast cells, DC, NKT cells, (e) inhibiting or blocking TSLP-induced osteoprotegerin (OPG ) secretion; (f) inhibiting or blocking TSLP-induced secretion of Th2 cytokines, such as TARC, CCL22, IL-4, IL-13 or IL-5, comprising:
administering an effective amount of the antibody or antigen-binding fragment thereof according to any one of claims 1-12, the nucleic acid according to claim 13, the vector according to claim 14, the host cell according to claim 15, the multispecific antibody according to claim 17, the conjugate according to claim 18, or the pharmaceutical composition according to claim 19 or 20,
optionally, an additional therapeutic is administered simultaneously with, before or after administration of the antibody or antigen-binding fragment thereof, the nucleic acid, the vector, the host cell, the multispecific antibody, the conjugate, or the pharmaceutical composition.

24. A method of preventing and/or treating an allergic inflammation or an autoimmune disease in a subject, comprising administering to a subject in need thereof an effective amount of the pharmaceutical composition according to claim 19 or 20, wherein the subject is a mammal; preferably, the subject is a human,
optionally, an additional therapeutic is administered simultaneously with, before or after administration of the pharmaceutical composition,
optionally, the allergic inflammation is selected from at least one of the group consisting of: asthma, idiopathic pulmonary fibrosis, atopic dermatitis (AD), allergic conjunctivitis, allergic rhinitis (AR), Netherton syndrome, eosinophilic esophagitis (EOE), food allergy, allergic diarrhea, eosinophilic gastroenteritis, allergic bronchopulmonary aspergillosis (ABPA), allergic fungal sinusitis, rheumatoid arthritis, COPD, systemic sclerosis, keloid, ulcerative colitis, chronic sinusitis (CRS) and nasal polyps, chronic eosinophilic pneumonia, eosinophilic bronchitis; Churg-Strauss syndrome, eosinophilia, eosinophilic granuloma with polyangiitis, inflammatory bowel disease, urticaria, systemic mastocytosis, cutaneous mastocytosis, and recurrent idiopathic angioedema;
optionally, the autoimmune disease is selected from the group consisting of: diabetes, myasthenia gravis, gastritis, pemphigus, primary biliary cirrhosis, multiple sclerosis, lupus, colitis, rheumatoid diseases, psoriasis and thyroid diseases;
optionally, the additional therapeutic is selected from but not limited to: immunosuppressants (for example, corticosteroids, non-steroidal glucocorticoid receptor agonists, leukotriene D4 antagonists, leukotriene B4 antagonists, A2A agonists, A2B antagonists, dopamine receptor agonists, pirfenidone, nintedanib, or avB6 antagonists), bronchodilators (for example, beta-2 adrenergic receptor agonists, muscarinic antagonists, short-acting β2 receptor agonists, long-acting β 2 receptor agonists, short-acting anticholinergic drugs, methyl xanthine drugs, long-acting anticholinergic drugs), other cytokine or cytokine receptor antagonists or antibodies (for example, IL-13 antagonists, IL-6 antagonists, antagonists of IL-1, IL-33, IL-25 or TNF-alpha, anti-IgE antibodies, anti-IL31 antibodies, anti-IL31R antibodies, anti-IL13 antibodies, anti-endoglin antibodies, anti-ILlb antibodies, another anti-TSLP antibody or anti-hTSLPR antibodies), antibiotics, radiotherapy, leukotriene antagonists (for example, montelukast, zafirlukast or pranlukast), PDE4 inhibitors (for example, roflumilast, xanthene), antihistamines or antitussive drugs.

25. A method of detecting the presence or level of TSLP in a sample, comprising contacting the sample with the antibody or antigen-binding fragment thereof according to any one of claims 1-12 under conditions allowing the formation of a complex between the antibody or antigen-binding fragment thereof and TSLP, and detecting the formation of the complex.

26. Use of the antibody or antigen-binding fragment thereof according to any one of claims 1-12, or the nucleic acid according to claim 13, or the vector according to claim 14, or the host cell according to claim 15, or the multispecific antibody according to claim 17, or the conjugate according to claim 18, or the pharmaceutical composition of claim 19 or 20 in the preparation of a medicament or kit for diagnosing an allergic inflammation or an autoimmune disease,
optionally, the allergic inflammation is selected from at least one of the group consisting of: asthma, idiopathic pulmonary fibrosis, atopic dermatitis (AD), allergic conjunctivitis, allergic rhinitis (AR), Netherton syndrome, eosinophilic esophagitis (EOE), food allergy, allergic diarrhea, eosinophilic gastroenteritis, allergic bronchopulmonary aspergillosis (ABPA), allergic fungal sinusitis, rheumatoid arthritis, COPD, systemic sclerosis, keloid, ulcerative colitis, chronic sinusitis (CRS) and nasal polyps, chronic eosinophilic pneumonia, eosinophilic bronchitis; Churg-Strauss syndrome, eosinophilia, eosinophilic granuloma with polyangiitis, inflammatory bowel disease, urticaria, systemic mastocytosis, cutaneous mastocytosis, and recurrent idiopathic angioedema;
optionally, the autoimmune disease is selected from the group consisting of: diabetes, myasthenia gravis, gastritis, pemphigus, primary biliary cirrhosis, multiple sclerosis, lupus, colitis, rheumatoid diseases, psoriasis and thyroid diseases.
